# EUROPEAN PATENT SPECIFICATION

(11) **EP 2 582 769 B1**
(45) Date of publication and mention of the grant of the patent: **19.11.2014**
(21) Application number: 11728604.7
(22) Date of filing: 16.06.2011
(51) Int. Cl.: C09K 11/06, H05B 33/10, H05B 33/14, C07D 307/91

(54) **ORGANIC ELECTRONIC DEVICES COMPRISING A LAYER OF A DIBENZOFURANE COMPOUND AND A 8-HYDROXYQUINOLINOLATO EARTH ALKALINE METAL, OR ALKALI METAL COMPLEX**
ORGANISCHE ELEKTRONISCHE VORRICHTUNGEN MIT EINER SCHICHT AUS EINER DIBENZOFURANVERBINDUNG UND EINEM 8-HYDROXYCHINOLINOLAT-ERDALKALIMETALL ODER ALKALIMETALLKOMPLEX
DISPOSITIFS ÉLECTRONIQUES ORGANIQUES COMPRENANT UNE COUCHE D'UN COMPOSÉ DE DIBENZOFURANE ET UN MÉTAL ALCALINO-TERREUX 8-HYDROXYQUINOLINOLATO, OU UN COMPLEXE MÉTALLIQUE ALCALIN

(30) Priority: 18.06.2010 EP 10166507
(43) Date of publication of application: 24.04.2013
(73) Proprietor: BASF SE, 67056 Ludwigshafen (DE)
(72) Inventor: LANGER, Nicolle, 64646 Heppenheim (DE); WAGENBLAST, Gerhard, 67157 Wachenheim (DE); MOLT, Oliver, 69469 Weinheim (DE); SCHILDKNECHT, Christian, San Diego, CA 92124 (US); FUCHS, Evelyn, 68199 Mannheim (DE); DORMANN, Korinna, 67098 Bad Dürkheim (DE); WATANABE, Soichi, 68165 Mannheim (DE); GEßNER, Thomas, 69120 Heidelberg (DE); LENNARTZ, Christian, 67105 Schifferstadt (DE); WOLLEB, Heinz, CH-4232 Fehren (CH); TANABE, Junichi, Hyogo 661-0044 (JP)
(74) Representative: Leybach, Holger Hugo
(86) International application number: PCT/EP2011/060025
(87) International publication number: WO 2011/157790

(56) References cited:
- WO-A1-03/046107
- WO-A1-2006/087521
- WO-A1-2006/128800

## Description

The present invention provides an organic electronic device including a first electrode, a second electrode, and an organic layer interposed between the first electrode and the second electrode, wherein the organic layer comprises an organic metal complex of formula I and a compound of formula II, or III and constitutes the electron transport layer.

WO10072300 relates to organic electroluminescent devices which comprise triazine derivatives optionally in combination with an organic alkali metal compound as the electron transport material. As an example of an organic alkali metal compound lithium quinolate is mentioned.

WO2006128800 discloses compounds of the formula 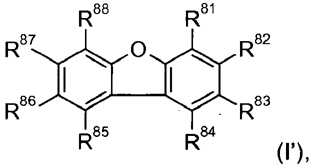 or 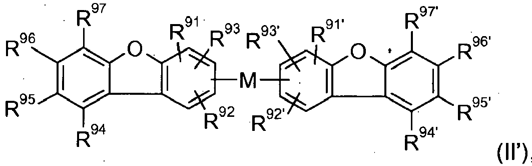 and their use in electroluminescent devices. According to WO2006128800 the compounds of formula I', or II' can be used as the light emitting material in the light emitting layer, optionally as a host or guest component (as fluorescence emitter, or as host for triplet emitter), or electron transport layer.

M. Thelakkat et al., Chem. Mater. 12 (2000) 3012-3019 describes the synthesis of lithium-quinolate complexes, 8-hydroxyquinolinolatolithium (Liq) and 2-methyl-8-hydroxyquinolinolatolithium (LiMeq) and their use as emitter and electron injection/transport materials in conventional two-layer organic light-emitting diodes in combination with N,N"-bis(*p*-methoxyphenyl)-N,N'-diphenylbenzidine (DMeOTPD) as hole transport material (HTL). The lithium complexes were also examined as interface materials in combination with 8-hydroxyquinolinolato-Al(III) (Alq₃) as emitter material. The lithium complexes increase the efficiency of an optimized indium-tin oxide (ITO)/DMeOTPD/Alq₃/Al device considerably when used as a thin interface layer between Alq₃ and aluminum. The improvement of device characteristics with lithium quinolates is similar to that obtained with LiF salt.

Z.L. Zhang et al., Synthetic Metals 158 (2008) 810-814 describes organic light-emitting diodes with 8-hydroxy-quinolinato lithium doped 4',7-diphenyl-1,10-phenanthroline as electron transport layer (ETL), and tetrafluro-tetracyano-quinodimethane doped 4,4',4"-tris(3-methylphenylphenylamono)triphenylamine as hole transport layer (HTL). Compared with the referenced device (without doping), the current efficiency and power efficiency of the pi-n device are enhanced by approximately 51% and 89%, respectively. This improvement is attributed to the improved conductivity of the transport layers and the efficient charge balance in the emission zone.

S. H. Choi et al., Japanese Journal of Applied Physics 48 (2009) 062101 reports improved efficiency and lifetime of OLEDs with lithium quinolate doped electron transport layers. The electron transport layer consists of LGC201 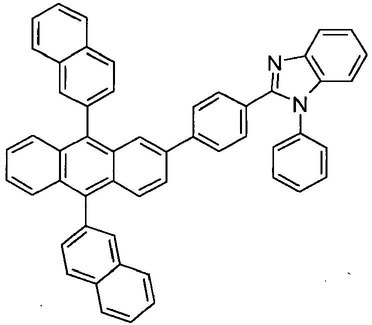 (LG Chem) and Liq.

WO2010040777A1 relates to the use of siloles substituted with condensed ring systems in organic-electronic applications and also special siloles substituted with condensed ring systems and use thereof in organic-electronic applications. 8-Hydroxyquinolinolatolithium (Liq) is mentioned as an example of a material of an electron transport material.

The organic electroluminescent compounds according to US2009230852A1 are represented by Chemical Formula 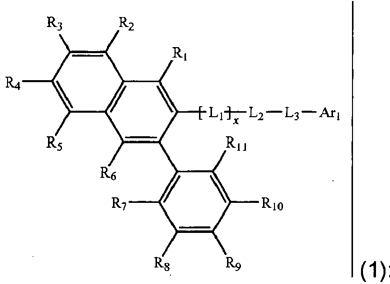 wherein, L₁ represents (C6-C60)arylene or (C3-C60)heteroarylene containing one or more heteroatom(s) selected from N, O and S, or a bivalent group selected from the following structures: L₂ and L₃ independently represent a chemical bond, or (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S; Ar₁ represents NR₄₁R₄₂, (C6-C60)aryl, (C3-C60)heteroaryl containing one or more heteroatom(s) selected from N, O and S, a 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: and x is an integer from 1 to 4. Lithium quinolate (Liq) is used in Example 1 as an electron injection material.

US2009189519A1 relates to organic compounds represented by formula 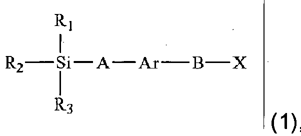 which are preferably used as host materials. Lithium quinolate (Liq) is used in Examples 1 to 5 as an electron injection material.

WO10001817A relates to an organic thin-film light-emitting element containing an organic compound represented by general formula Y-(A¹-Ar)ₙ₁ (1) and a donor compound as an electronic transport layer. In general formula (1), Y represents a substituted or unsubstituted pyrene or a substituted or unsubstituted anthracene; A¹ represents a member selected from the group consisting of a single bond, arylene groups and heteroarylene groups; Ar represents a member selected from the group consisting of carbazolyl groups, dibenzofuranyl groups and dibenzothiophenyl groups; the groups may be substituted or unsubstituted; and n¹ represents an integer of 1-3. Examples of compounds of formula 1 are , 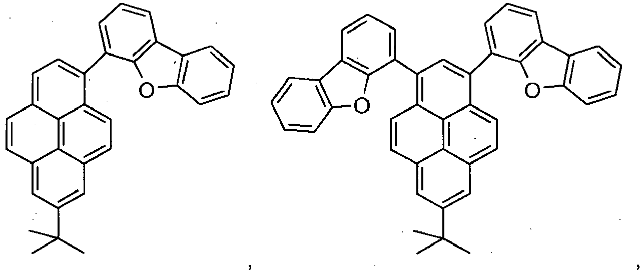 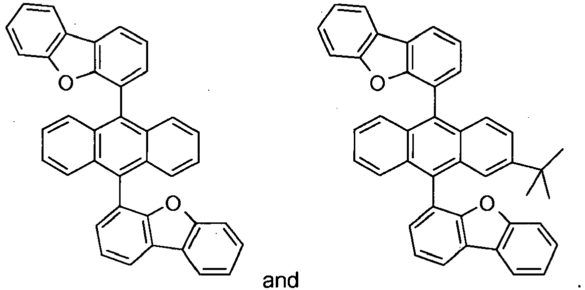 Liq is mentioned as an example of the donor compound. In the Working Example the electron transport layer consists of a 1:1 mixture of Liq and

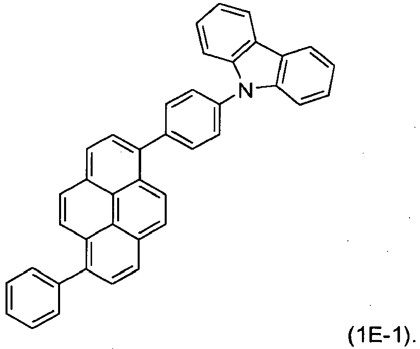

It was the object of the present invention to provide organic electronic devices, especially organic light emitting devices showing good efficiencies, good operative lifetimes and a high stability to thermal stress, and a low operating voltage.

Said object has been solved by an organic electronic device including a first electrode, a second electrode, and an organic layer interposed between the first electrode and the second electrode, wherein the organic layer comprises
an organic metal complex of formula 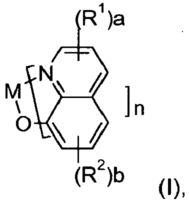 and
a compound of formula 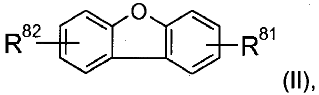 or 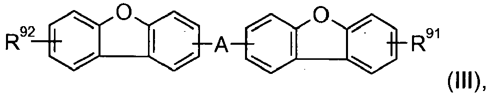 wherein
R¹ and R² are independenly of each other F, C₁-C₈alkyl, or C₆-C₁₄aryl, which may optionally be substituted by one, or more C₁-C₈alkyl groups, or
two substituents R¹ and/or R² combine to form a fused benzene ring group, which may optionally be substituted by one, or more C₁-C₈alkyl groups,
a and b are independently of each other 0, or an integer 1 to 3,
R⁸¹ is phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
R⁸² is phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
R⁹¹ is phenanthryl, pyrenyl, triphenylenyl, 1,10-phenanthrolinyl, triazinyl, dibenzothiophenyl, pyrimidinyl, or dibenzofuranyl, each of which may optionally be substituted by one, or more C₁-C₁₈alkyl, C₆-C₁₄aryl, or C₂-C₂₀heteroaryl groups,
R⁹² is H, phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
A is a single bond, an arylene, or heteroarylene group, each of which may optionally be substituted by one, or more C₁-C₁₈alkyl groups; or -SiR⁸³R^{B4}-, wherein R⁸³ and R⁸⁴ are independently of each other a C₁-C₁₈alkyl, or a C₆-C₁₄aryl group, which may optionally be substituted by one, or more C₁-C₁₈alkyl groups;
M is an alkali metal atom, or an earth alkaline metal atom,
n is 1, if M is an alkali metal atom, n is 2, if M is an earth alkaline metal atom.

If the organic layer comprising the compounds of formula I and II, or III constitutes the electron transport layer of an OLED OLEDs having superior life time, power efficiency, quantum efficiency and/or a low operating voltage are obtained.

Examples of M are Li, Na, K, Rb, Cs, Be, Mg, Ca, Sr, or Ba. Li, Na and K are preferred, Li is most preferred. Examples of the metal complex of formula I are 8-hydroxyquinolinolato lithium (Liq) and 2-methyl-8-hydroxyquinolinolato lithium (LiMeq). The most preferred metal complex is 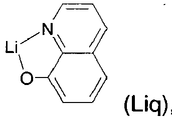 which can exist as the single species, or in other forms such as Li_{g}Q_{g}, where g is an integer, for example Li₆Q₆. Q represents 8-hydroxyquinolate ligand or a derivative of 8-hydroxyquinolate

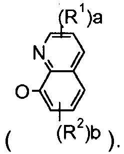

Preferably, the substituents R⁸¹ and R⁸² are in ortho-, or para-position with respect to the oxygen atom of the dibenzofurane moiety.

Preferably, the substituents A, R⁹¹ and R⁹² are in ortho-, or para-position with respect to the oxygen atom of the dibenzofurane moiety.

Compounds of formula 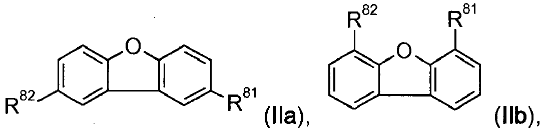 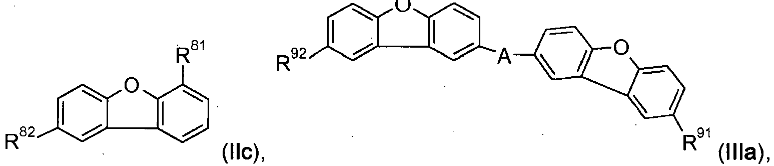 or 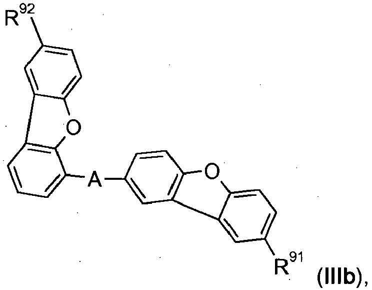 wherein A, R⁸¹, R⁸², R⁹¹ and R⁹² are as defined above, are preferred.

Preferably, R⁸¹ is phenanthryl, dibenzothiophenyl, or pyrenyl, each of which may optionally be substituted by one, or more C₁-C₁₈alkyl groups and R⁸² is phenanthryl, dibenzothiophenyl, or pyrenyl, each of which may optionally be substituted by one, or more C₁-C₁₈alkyl groups. More preferably, R⁸¹ is phenanthryl, or pyrenyl, each of which may optionally be substituted by one, or more C₁-C₁₈alkyl groups and R⁸² is phenanthryl, or pyrenyl, each of which may optionally be substituted by one, or more C₁-C₁₈alkyl groups.

Preferably, R⁹¹ is dibenzofuranyl, dibenzothiophenyl, phenanthryl, or pyrenyl, each of which may optionally be substituted by one, or more C₁-C₁₈alkyl groups and R⁹² is H, phenanthryl, dibenzothiophenyl, or pyrenyl, each of which may optionally be substituted by one, or more C₁-C₁₈alkyl groups. More preferably, R⁹¹ is dibenzofuranyl, phenanthryl, or pyrenyl, each of which may optionally be substituted by one, or more C₁-C₁₈alkyl groups and R⁹² is phenanthryl, or pyrenyl, each of which may optionally be substituted by one, or more C₁-C₁₈alkyl groups. For R⁹¹ phenanthryl and pyrenyl groups are more preferred than dibenzofuranyl groups.

In a preferred embodiment of the present invention R⁸¹ and R⁸² are independently of each other 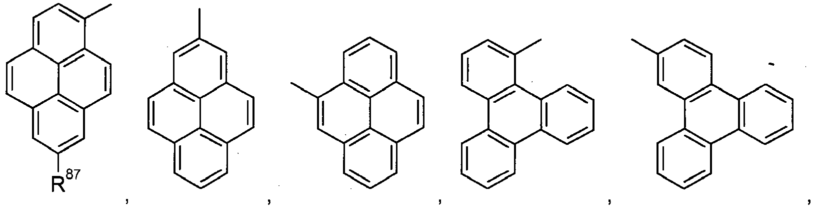 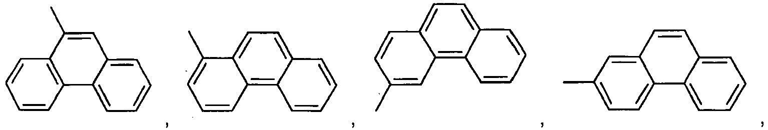 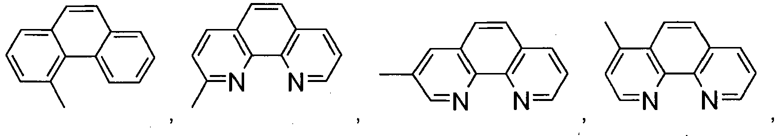 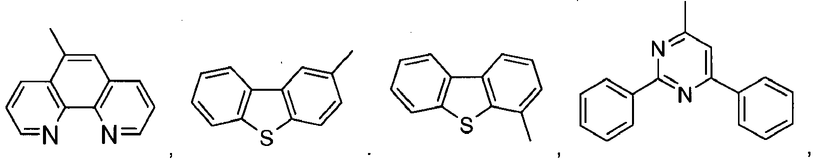 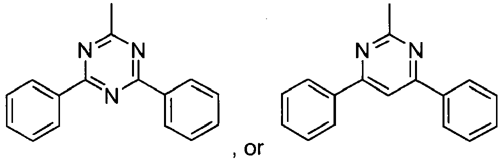 wherein R⁸⁷ is H, or C₁-C₈alkyl.

More preferably, R⁸¹ is 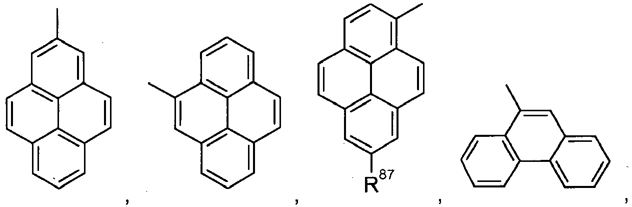 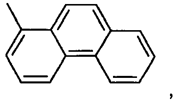 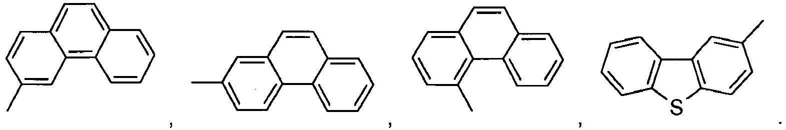 or 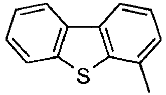 and R⁸² has the same preferences as R⁸¹.

In another preferred embodiment of the present invention R⁹¹ has the meaning of R⁸¹, or is 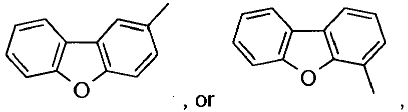 and R⁹² has the meaning of R⁸¹, or is H.

More preferably, R⁹¹ has the same preferences as R⁸¹, or is 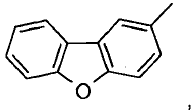 or 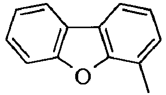 and R⁹² has the same preferences as R⁸¹, or is H.

A is a single bond, a substituted, or unsubstituted arylene group having 6 to 30 ring carbon atoms, or a substituted or unsubstituted heteroarylene group having 5 to 30 ring carbon atoms, or -SiR⁸³R⁸⁴-.

Preferred arylene radicals are phenylene, naphthylene, phenantrylene and phenalylene, which may optionally be substituted by one, or more C₁-C₁₈alkyl groups.

Preferred heteroarylene radicals are pyridinylene, pyrazinylene, pyrimidinylene, and triazolylene, which may optionally be substituted by one, or more C₁-C₁₈alkyl groups.

More preferred arylene radicals are 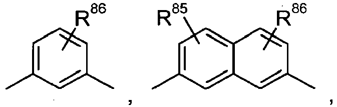 and 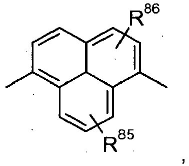 , wherein R⁸⁵ and R⁸⁶ are independently of each other H, or a C₁-C₁₈alkyl group.

In another embodiment of the present invention preferred arylene radicals are a pyrenylene, or anthrylene group. In said embodiment a groups of formula 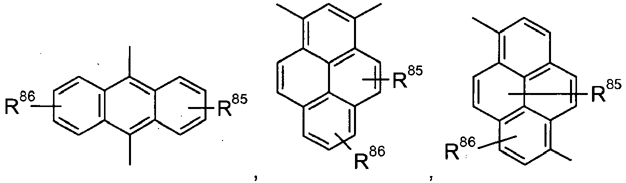 are more preferred and a group of formula 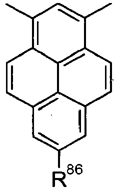 is most preferred, wherein R⁸⁵ and R⁸⁶ are independently of each other H, or a C₁-C₁₈alkyl group.

More preferred heteroarylene radicals are 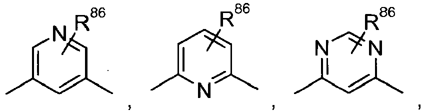 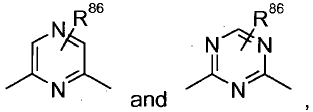 wherein R⁸⁶ is H, or a C₁-C₁₈alkyl group.

Preferred groups -SiR⁸³R⁸⁴- are -Si(CH₃)₂ , -Si(CH₂CH₃)₂-, and Si(C(CH₃)₃)₂-.

Particularly preferred compounds of formula II are compounds A-1 to A-36. Particularly preferred compounds of formula III are compounds B-1 to B-27. Reference is made to claim 5. Compound A-10 is at present most preferred.

In a particularly preferred embodiment the organic layer, especially the electron transport layer comprises a mixture of a compound of formula 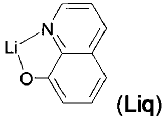 and a compound of formula

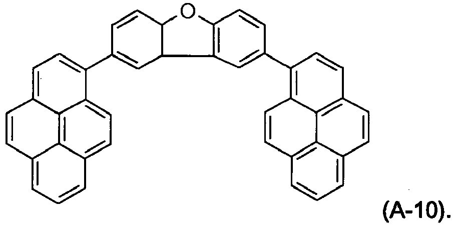

The organic electronic device according to the present invention is preferably an organic light emitting device (OLED), comprising an anode, a hole injection layer, a hole transport layer, a light emitting layer, a hole and exciton blocking layer, an electron transport layer, an electron injection layer and a cathode, wherein the organic layer comprising the compounds of formula I and II, or III constitutes the electron transport layer. An exciton blocking layer, may be arranged between the hole transport layer and the light emitting layer.

Accordingly, the present invention is also directed to an electron transport layer, comprising an organic metal complex of formula I and a compound of formula II, or III.

The organic metal complex of formula I is contained in the organic layer, especially the electron transport layer of an OLED in an amount of 99 to 1 % weight, preferably 75 to 25 % by weight, more preferably about 50 % by weight, based on the amount of compound of formula I and II, or III.

The synthesis of the compounds of formula II and III is described in WO2006128800, or can be done in analogy to the methods described therein.

The synthesis of the compounds of formula I is described, for example, in Christoph Schmitz et al. Chem. Mater. 12 (2000) 3012-3019 and WO00/32717, or can be done in analogy to the methods described therein.

C₁-C₁₈alkyl is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₆-C₁₄aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted.

C₂-C₂₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated *π*-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phehothiazinyl, isoxazolyl, furazanyl or phenoxazinyl, which can be unsubstituted or substituted.

The C₆-C₁₄aryl and C₂-C₂₀heteroaryl groups are preferably substituted by one, or more C₁-C₈alkyl groups.

Examples of arylene radicals are phenylene, naphthylene, phenalenylene, antracylene and phenantrylene, which may optionally be substituted by one or more C₁-C₁₈alkyl groups. Preferred arylene radicals are 1,3-phenylene, 3,6-naphthylene, and 4,9-phenalenylene, which may optionally be substituted by one or more C₁-C₁₈alkyl groups.

Examples of heteroarylene radicals are 1,3,4-thiadiazol-2,5-ylene, 1,3-thiazol-2,4-ylene, 1,3-thiazol-2,5-ylene, 2,4-thiophenylene, 2,5-thiophenylene, 1,3-oxazol-2,4-ylene, 1,3-oxazol-2,5-ylene and 1,3,4-oxadiazol-2,5-ylene, 2,5-indenylene, 2,6-indenylene, especially pyrazinylene, pyridinylene, pyrimidinylene, and triazolylene, which may optionally be substituted by one or more C₁-C₁₈alkyl groups. Preferred heteroarylene radicals are 2,6-pyrazinylene, 2,6-pyridinylene, 4,6-pyrimidinylene, and 2,6-triazolylene, which may optionally be substituted by one or more C₁-C₁₈alkyl groups.

The organic electronic device of the present application is, for example, an organic solar cell (organic photovoltaics), a switching element such as an organic transistors, for example organic FET and organic TFT, organic light emitting field effect transistor (OLEFET), or an organic light-emitting diode (OLED), preference being given to OLEDs.

The present application relates to the use of the organic metal complex of formula I in combination with the compound of formula II, or III as electron transport layer in an organic electronic device.

Accordingly, the present application is also directed to an organic layer, an electron transport layer, comprising an organic metal complex of formula I and a compound of formula II, or III.

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell. The layer with electron transport capacity comprises the organic metal complex of formula I and the compound of formula II, or III.

The present invention further relates to an organic light-emitting diode comprising an anode An and a cathode Ka, a light-emitting layer E arranged between the anode An and the cathode Ka, an electron transport layer arranged between the cathode Ka and the light-emitting layer E, and if appropriate at least one further layer selected from the group consisting of at least one blocking layer for holes/excitons, at least one blocking layer for electrons/excitons, at least one hole injection layer, at least one hole transport layer and at least one electron injection layer, wherein the electron transport layer comprises an organic metal complex of formula I and a compound of formula II, or III.

### Structure of the inventive OLED

The inventive organic light-emitting diode (OLED) thus generally has the following structure:
an anode (An) and a cathode (Ka) and a light-emitting layer E arranged between the anode (An) and the cathode (Ka) and an electron transport layer arranged between the cathode Ka and the light-emitting layer E.

The inventive OLED may, for example - in a preferred embodiment - be formed from the following layers:
1. Anode
2. Hole transport layer
3. Light-emitting layer
4. Blocking layer for holes/excitons
5. Electron transport layer
6. Cathode

Layer sequences different than the aforementioned structure are also possible, and are known to those skilled in the art. For example, it is possible that the OLED does not have all of the layers mentioned; for example, OLEDs which have layers (1), (3), (4), (5) and (6), are likewise suitable. In addition, the OLEDs may have a blocking layer for electrons/excitons between the hole transport layer (2) and the light-emitting layer (3).

It is additionally possible that a plurality of the aforementioned functions (electron/exciton blocker, hole/exciton blocker, hole injection, hole transport, electron injection, electron transport) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used as emitter substances in accordance with the invention.

In order to obtain particularly efficient OLEDs, for example, the HOMO (highest occupied molecular orbital) of the hole transport layer should be matched to the work function of the anode, and the LUMO (lowest unoccupied molecular orbital) of the electron transport layer should be matched to the work function of the cathode, provided that the aforementioned layers are present in the inventive OLEDs.

The anode (1) is an electrode which provides positive charge carriers. It may be formed, for example, from materials which comprise a metal, a mixture of various metals, a metal alloy, a metal oxide or a mixture of various metal oxides. Alternatively, the anode may be a conductive polymer. Suitable metals comprise metals and alloys of the metals of the main groups, transition metals and of the lanthanoids, especially the metals of groups Ib, IVa, Va and VIa of the periodic table of the elements, and the transition metals of group VIIIa. When the anode is to be transparent, generally mixed metal oxides of groups IIb, IIIb and IVb of the periodic table of the elements (IUPAC version) are used, for example indium tin oxide (ITO). It is likewise possible that the anode (1) comprises an organic material, for example polyaniline, as described, for example, in Nature, Vol. 357, pages 477 to 479 (June 11, 1992). At least either the anode or the cathode should be at least partly transparent in order to be able to emit the light formed. The material used for the anode (1) is preferably ITO.

Suitable hole transport materials for layer (2) of the inventive OLEDs are disclosed, for example, in Kirk-Othmer Encyclopedia of Chemical Technology, 4th edition, Vol. 18, pages 837 to 860, 1996. Both hole-transport molecules and polymers can be used as the hole transport material. Hole-transport molecules typically used are selected from the group consisting of tris[N-(1-naphthyl)-N-(phenylamino)]triphenylamine (1-NaphDATA), 4,4'-bis[N(1-naphthyl)-N-phenylamino]biphenyl (α-NPD), N,N'-diphenyl-N,N'-bis(3-methylphenyl)[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethy)phenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl)(4-methylphenyl)methane (MPMP), 1-phenyl-3-[p-(diethylamino)styryl]-5-[p-(diethylamino)phenyl]pyrazoline (PPR or DEASP), 1,2-trans-bis(9H-carbazol-9-yl)cyclobutane (DCZB), N,N,N',N'-tetrakis(4-methylphenyl)-(1,1'-biphenyl)-4,4'-diamine (TTB), 4,4',4"-tris(N,N-diphenylamino)triphenylamine (TDTA), 4,4',4"-tris(N-carbazolyl)triphenylamine (TCTA), N,N'-bis(naphthalen-2-yl)-N,N'-bis(phenyl)benzidine (β-NPB), N,N'-bis(3-methylphenyl)-N,N'-bis(phenyl)-9,9-spirobifluorene (Spiro-TPD), N,N'-bis(naphthalen-1-yl)-N,N'-bis(phenyl)-9,9-spirobifluorene (Spiro-NPB), N,N'-bis(3-methylphenyl)-N,N'-bis(phenyl)-9,9-dimethylfluorene (DMFL-TPD), di[4-(N,N-ditolylamino)phenyl]cyclohexane, N,N'-bis(naphthalen-1-yl)-N,N'-bis(phenyl)-9,9-dimethylfluorene, N,N'-bis(naphthalen-1-yl)-N,N'-bis(phenyl)-2,2-dimethylbenzidine, N,N'-bis(naphthalen-1-yl)-N,N'-bis(phenyl)benzidine, N,N'-bis(3-methylphenyl)-N,N'-bis(phenyl)benzidine, 2,3,5,6-tetrafluoro-7,7,8,8-tetracyanoquinodimethane (F4-TCNQ), 4,4',4"-tris(N-3-methylphenyl-N-phenylamino)triphenylamine, 4,4',4"-tris(N-(2-naphthyl)-N-phenyl-amino)triphenylamine, pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di[4-(N,N-ditolylamino)phenyl]benzidine (NTNPB), N,N'-diphenyl-N,N'-di[4-(N,N-diphenylamino)phenyl]benzidine (NPNPB), N,N'-di(naphthalen-2-yl)-N,N'-diphenylbenzene-1,4-diamine (β-NPP), N,N'-bis(3-methylphenyl)-N,N'-bis(phenyl)-9,9-diphenylfluorene (DPFL-TPD), N,N'-bis(naphthalen-1-yl)-N,N'-bis(phenyl)-9,9-diphenylfluorene (DPFL-NPB), 2,2',7,7'-tetrakis(N,N-diphenylamino)-9,9'-spirobifluorene (Spiro-TAD), 9,9-bis[4-(N,N-bis(biphenyl-4-yl)amino)phenyl]-9H-fluorene (BPAPF), 9,9-bis[4-(N,N-bis(naphthalen-2-yl)amino)phenyl]-9H-fluorene (NPAPF), 9,9-bis[4-(N,N-bis(naphthalen-2-yl)-N,N'-bisphenylamino)phenyl]-9H-fluorene (NPBAPF), 2,2',7,7'-tetrakis[N-naphthalenyl(phenyl)amino]-9,9'-spirobifluorene (Spiro-2NPB), N,N'-bis(phenanthren-9-yl)-N,N'-bis(phenyl)benzidine (PAPB), 2,7-bis[N,N-bis(9,9-spirobifluoren-2-yl)amino]-9,9-spirobifluorene (Spiro-5), 2,2'-bis[N,N-bis(biphenyl-4-yl)amino]-9,9-spirobifluorene (2,2'-Spiro-DBP), 2,2'-bis(N,N-diphenylamino)-9.9-spirobifluorene (Spiro-BPA), 2,2',7,7'-tetra(N,N-ditolyl)aminospirobifluorene (Spiro-TTB), N,N,N',N'-tetranaphthalen-2-ylbenzidine (TNB), porphyrin compounds and phthalocyanines such as copper phthalocyanines and titanium oxide phthalocyanines. Hole-transporting polymers typically used are selected from the group consisting of polyvinylcarbazoles, (phenylmethyl)polysilanes and polyanilines. It is likewise possible to obtain hole-transporting polymers by doping hole-transporting molecules into polymers such as polystyrene and polycarbonate. Suitable hole-transporting molecules are the molecules already mentioned above.

In addition - in one embodiment - it is possible to use carbene complexes as hole transport materials, the band gap of the at least one hole transport material generally being greater than the band gap of the emitter material used. In the context of the present application, "band gap" is understood to mean the triplet energy. Suitable carbene complexes are, for example, carbene complexes as described in WO 2005/019373 A2, WO 2006/056418 A2, WO 2005/113704, WO 2007/115970, WO 2007/115981 and WO 2008/000727. One example of a suitable carbene complex is fac-Iridium-tris(1,3-diphenylbenzimidazolin-2-yliden-C,C^{2'}) (Ir(dpbic)₃) with the formula: 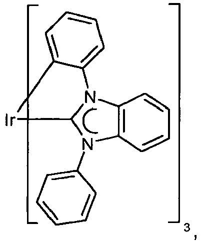 which is disclosed, for example, in WO2005/019373. Preferably, the hole transport layer comprises a compound of formula 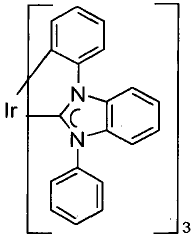 doped with molybdenum oxide (MoOₓ), especially MoO₃, or rhenium oxide (ReOₓ), especially ReO₃. The dopant is contained in an amount of from 0.1 % by weight, preferably 1 to 8 % by weight, more preferably 3 to 5 % by weight, based on the amount of dopant and carbene complex.

The light-emitting layer (3) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance.

Suitable metal complexes for use in the inventive OLEDs are described, for example, in documents WO 02/60910A1, US 2001/0015432 A1, US 2001/0019782 A1, US 2002/0055014 A 1, U S 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2 , WO 2007/115970, WO 2007/115981 and WO 2008/000727, WO2010129323, WO2010056669 and WO10086089.

The light emitting layer comprises preferably a compound of the formula 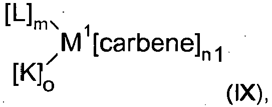 which are decribed in WO 2005/019373 A2, wherein the symbols have the following meanings:
M¹ is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a *π* complex with M¹; n1 is the number of carbene ligands, where n1 is at least 1 and when n1 > 1 the carbene ligands in the complex of the formula I can be identical or different;
m is the number of ligands L, where m can be 0 or ≥ 1 and when m > 1 the ligands L can be identical or different;
o is the number of ligands K, where o can be 0 or ≥ 1 and when o > 1 the ligands K can be identical or different;
where the sum n1 + m + o is dependent on the oxidation state and coordination number of the metal atom and on the denticity of the ligands carbene, L and K and also on the charge on the ligands, carbene and L, with the proviso that n1 is at least 1.

The compound of formula IX is preferably a compound of the formula:

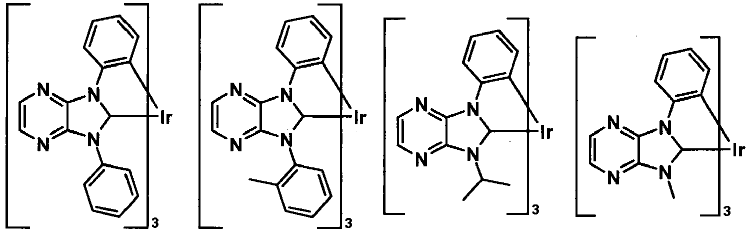

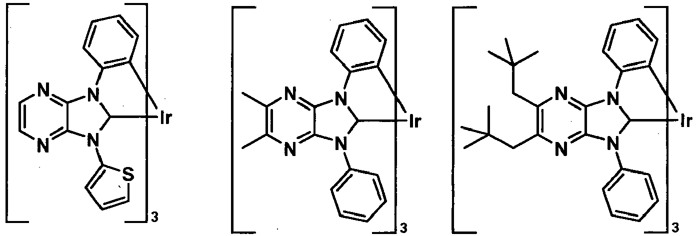

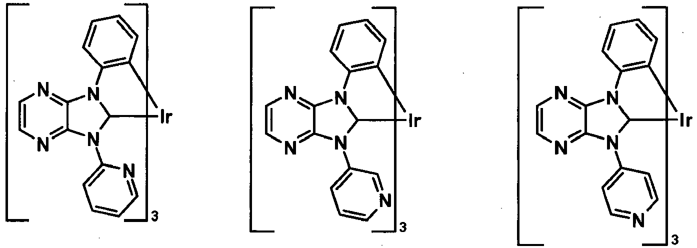

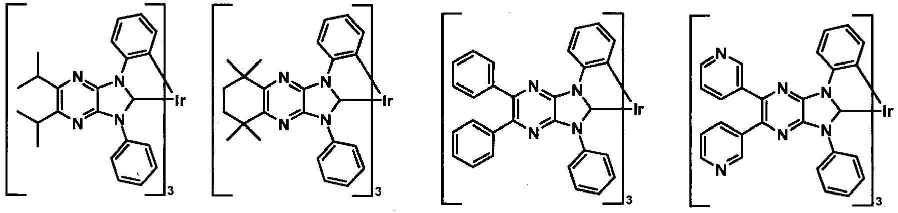

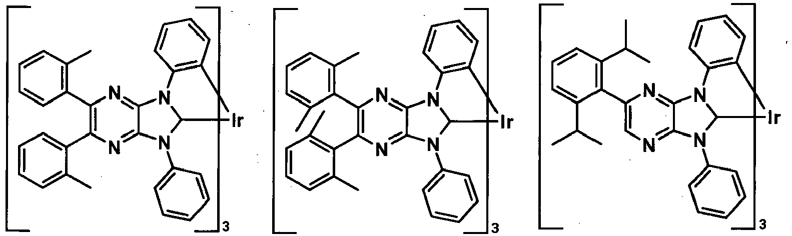

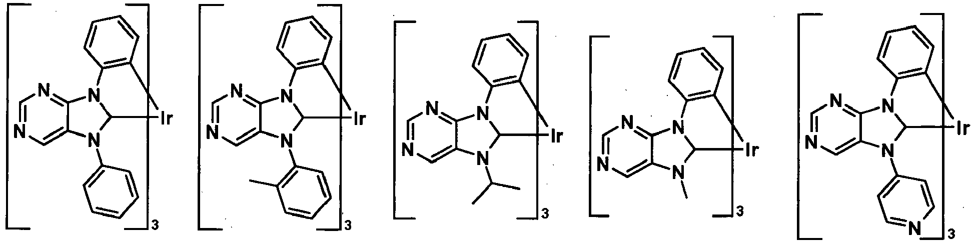

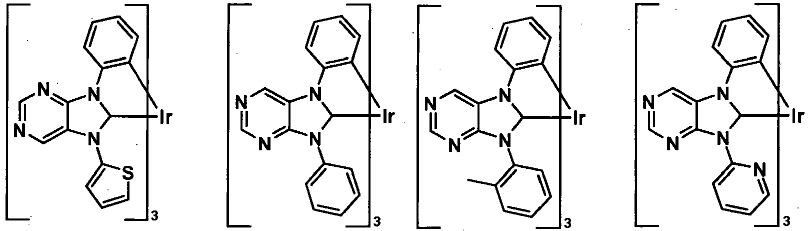

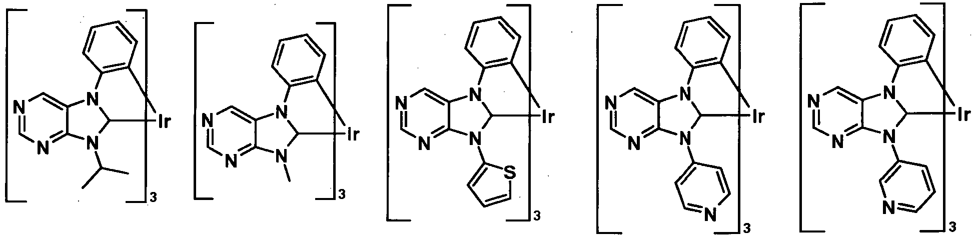

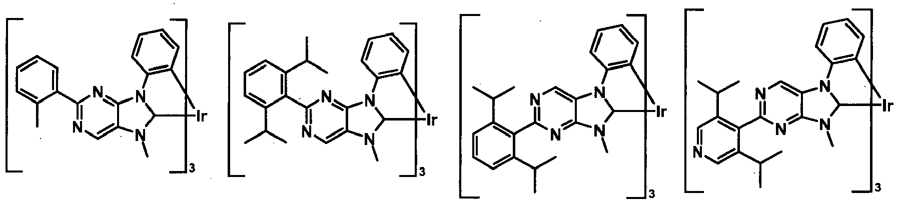

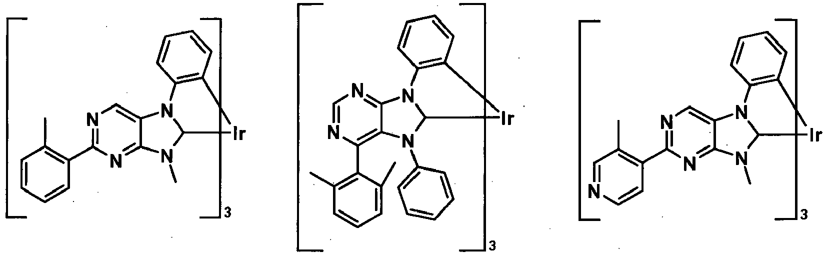

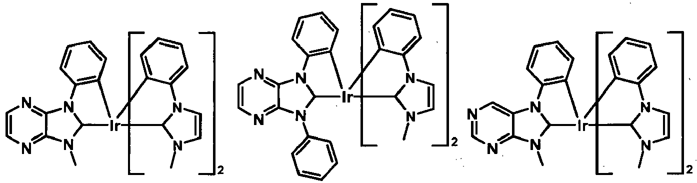

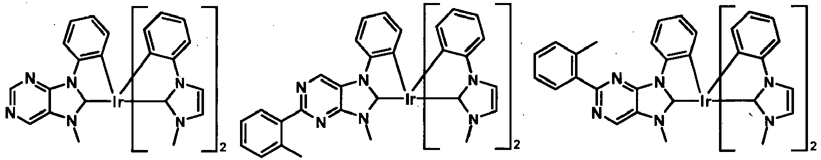

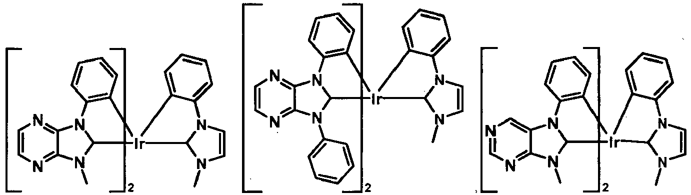

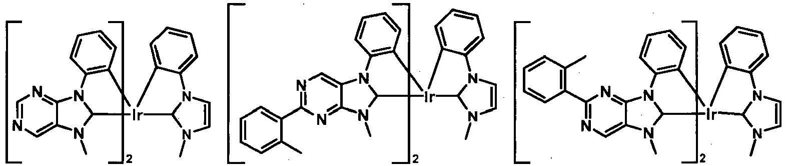

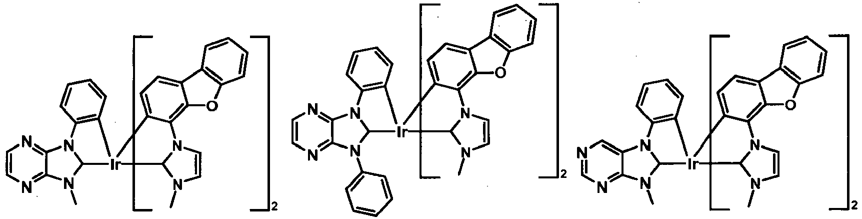

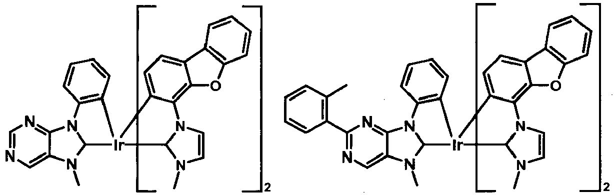

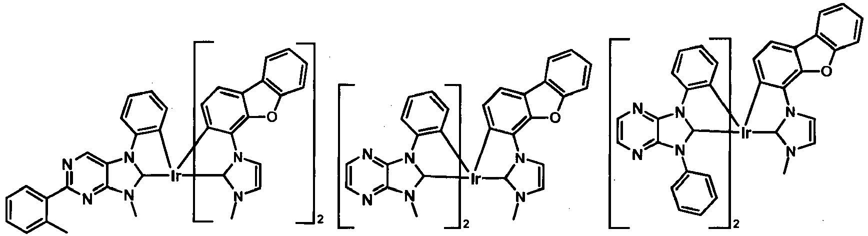

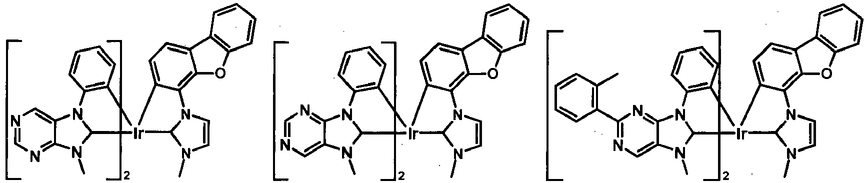

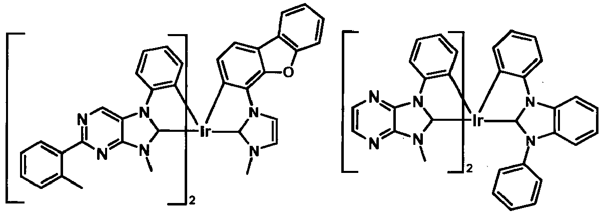

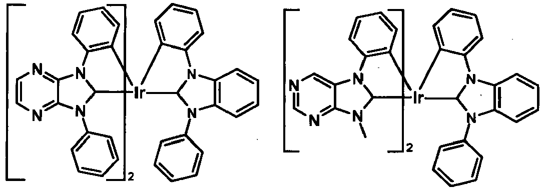

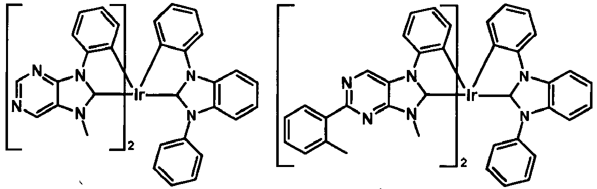

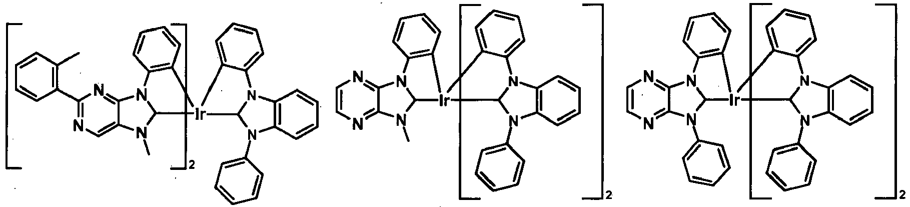

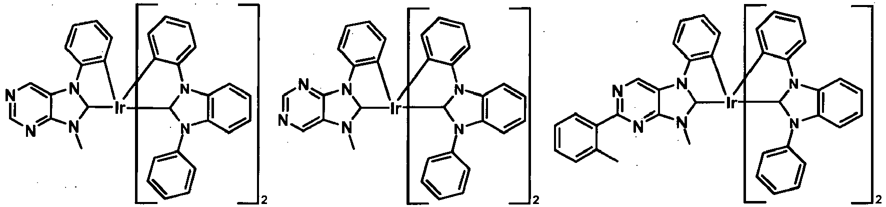

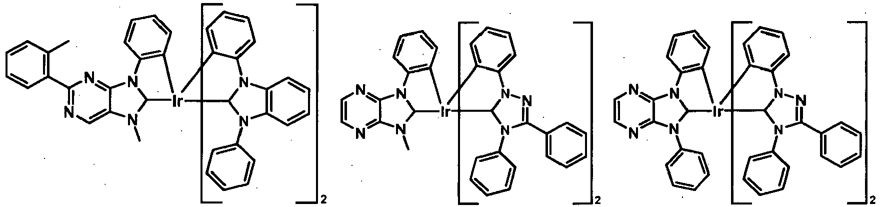

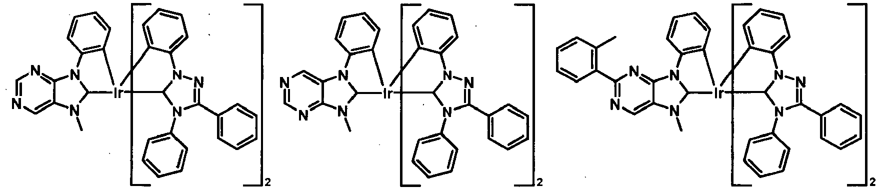

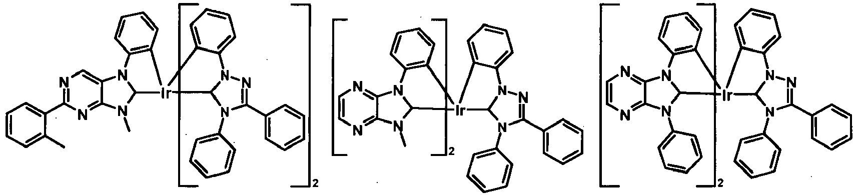

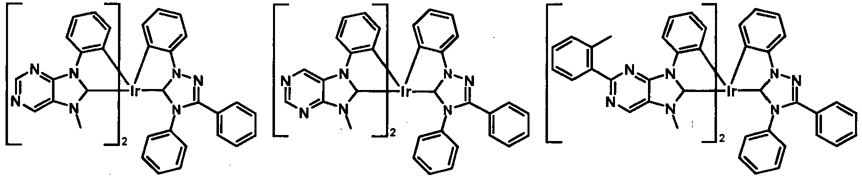

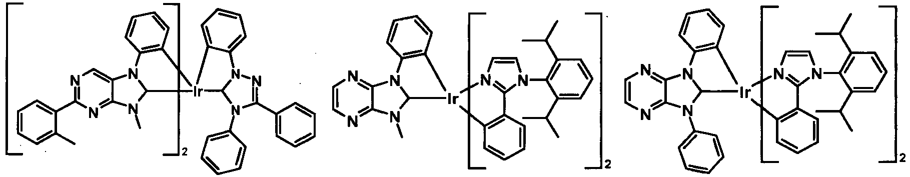

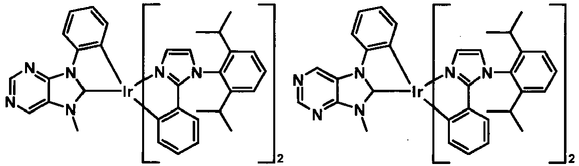

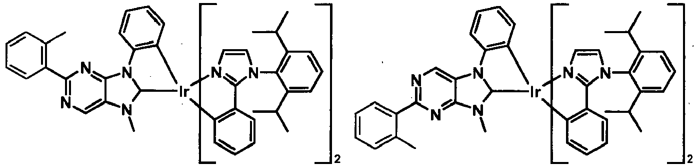

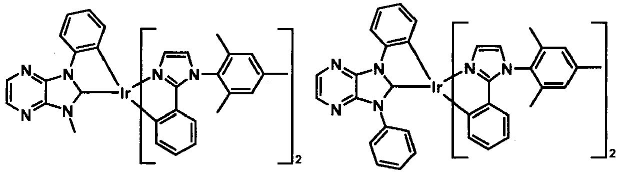

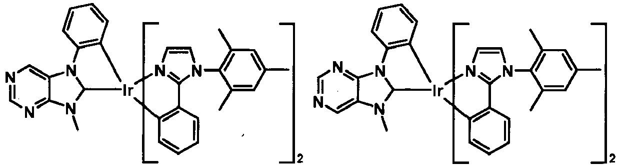

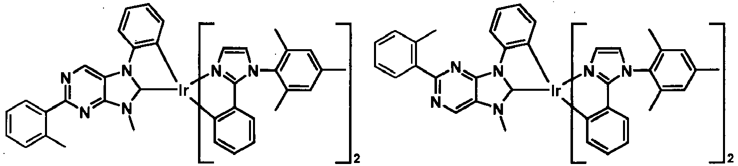

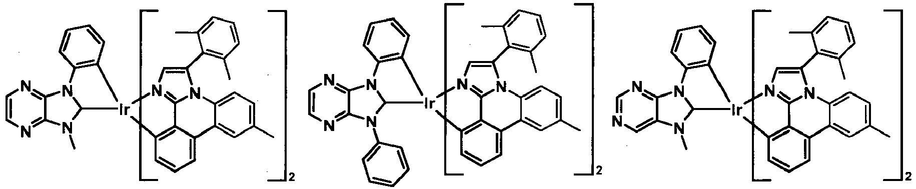

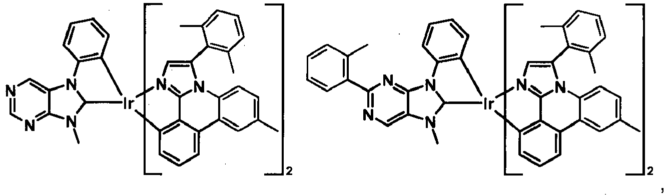

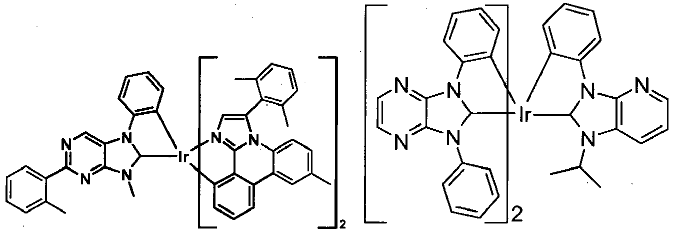

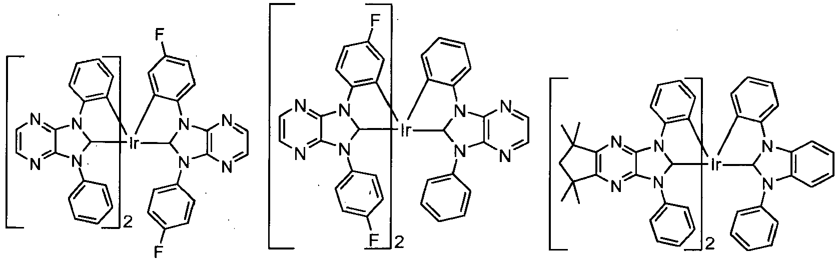

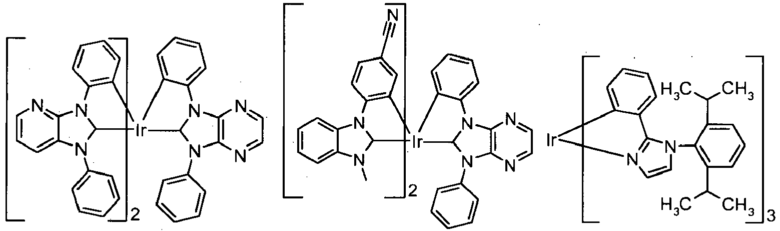

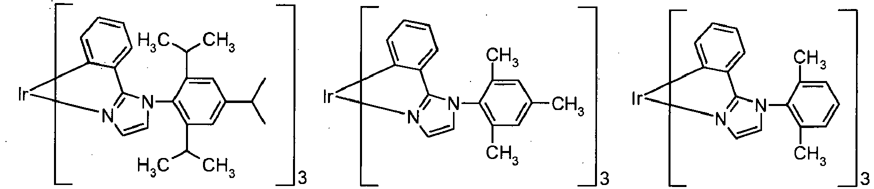

The homoleptic metal-carbene complexes may be present in the form of facial or meridional isomers, preference being given to the facial isomers.

In the case of the heteroleptic metal-carbene complexes, four different isomers may be present, preference being given to the pseudo-facial isomers.

The compound of the formula IX is more preferably a compound of the formula

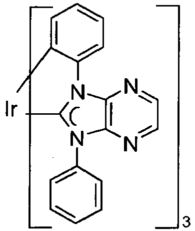

Further suitable metal complexes are the commercially available metal complexes tris(2-phenylpyridine)iridium(III), iridium(III) tris(2-(4-tolyl)pyridinato-N,C^{2'}), b i s (2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trrfluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Suitable triplet emitters are, for example, carbene complexes. In one embodiment of the present invention, the compounds of the formula X are used in the light-emitting layer as matrix material together with carbene complexes as triplet emitters. 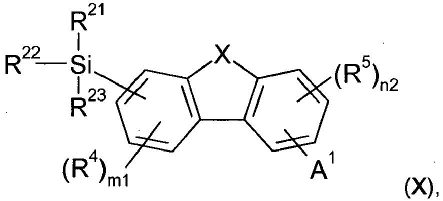 wherein
X is NR, S, O or PR;
R is aryl, heteroaryl, alkyl, cycloalkyl, or heterocycloalkyl;
A¹ is -NR⁶R⁷, -P(O)R⁸R⁹, -PR¹⁰R¹¹, -S(O)²R¹², -S(O)R¹³, -SR¹⁴, or -OR¹⁵;
R²¹, R²² and R²³ are independently of each other aryl, heteroaryl, alkyl, cycloalkyl, or heterocycloalkyl, wherein at least on of the groups R¹, R², or R³ is aryl, or heteroaryl;
R⁴ and R⁵ are independently of each other alkyl, cycloalkyl, heterocycloalkyl, aryl, heteroaryl, a group A¹, or a group having donor, or acceptor characteristics;
n2 and m1 are independently of each other 0, 1, 2, or 3;
R⁶, R⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and
R⁸, R⁹, R¹⁰, R¹¹, R¹², R¹³, R¹⁴ und R¹⁵ are independently of each other aryl, heteroaryl, alkyl, cycloalkyl, or heterocycloalkyl. Compounds of formula X, such as, for example, 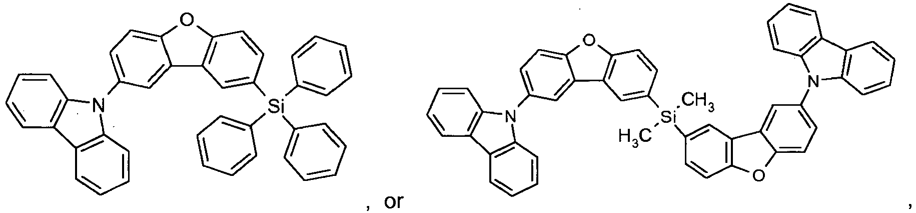 are described in WO2010079051 (PCT/EP2009/067120; in particular pages on 19 to 26 and in tables on pages 27 to 34, pages 35 to 37 and pages 42 to 43).

Additional matrix materials on basis of dibenzofurane are, for example, described in US2009066226, EP1885818B1, EP1970976, EP1998388 and EP2034538. Examples of particularly preferred matrix materials are shown below: 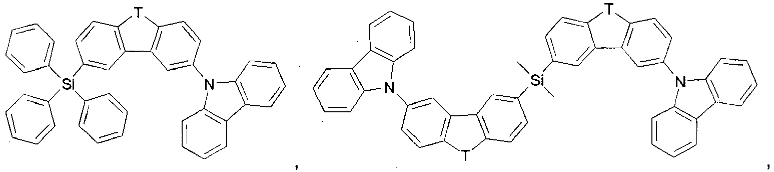 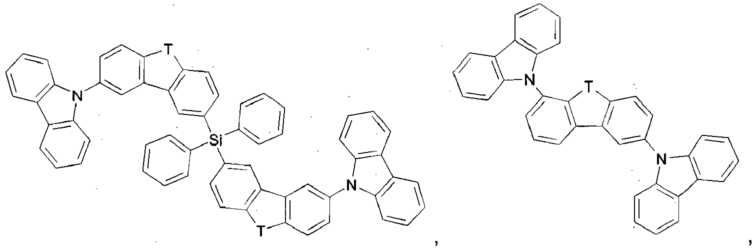 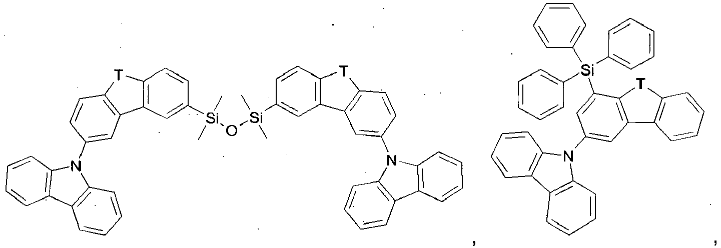 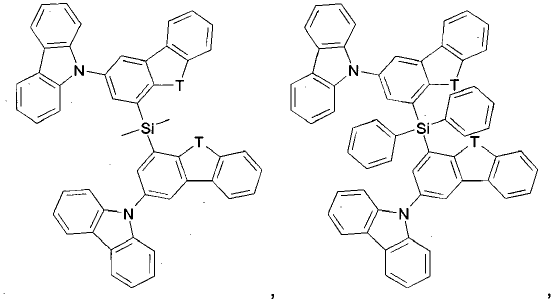 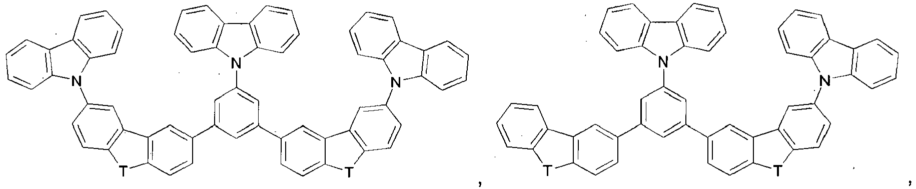 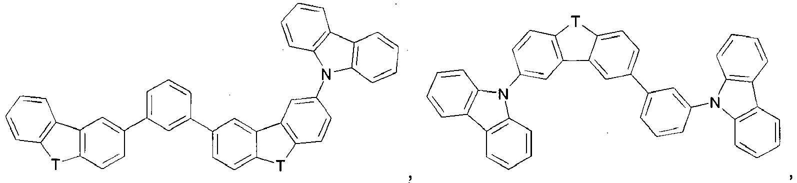 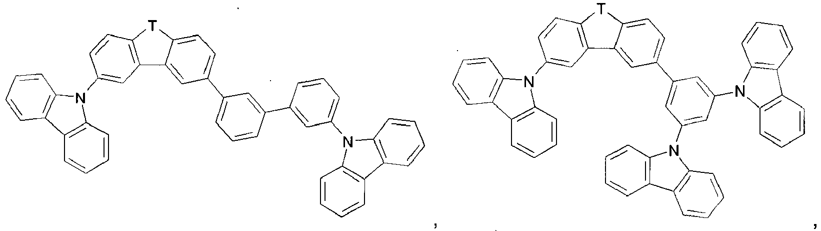 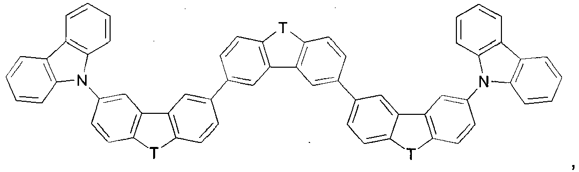 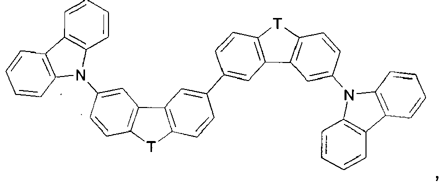 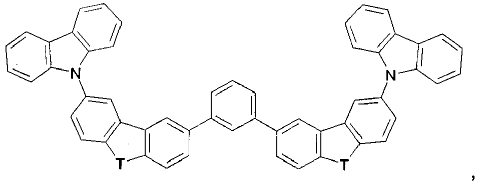 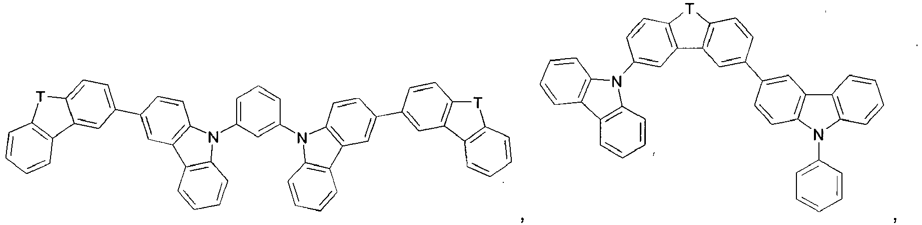 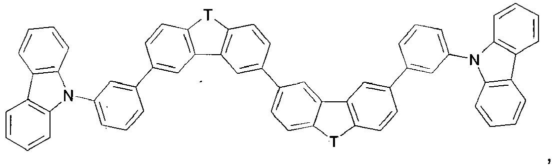 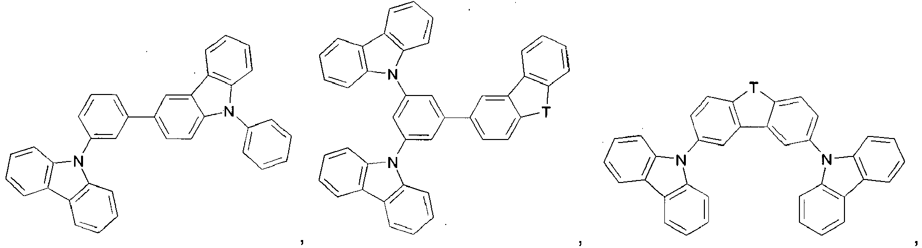 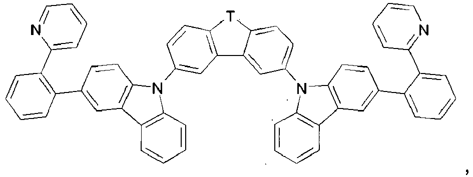 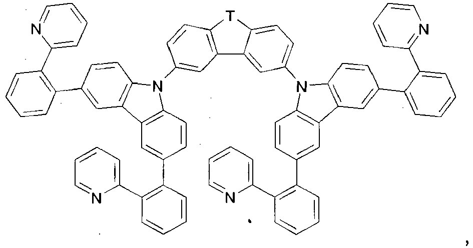 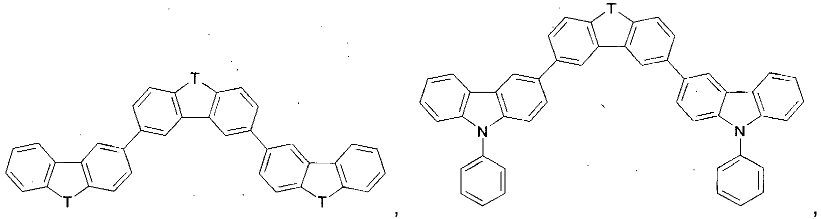 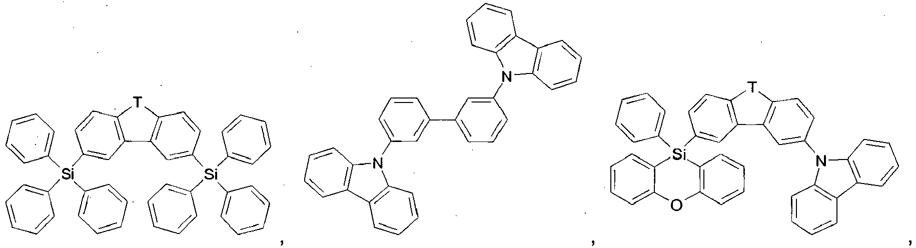 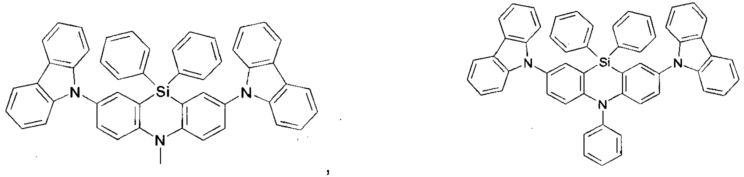 and

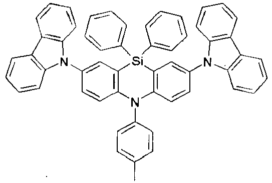

In the above-mentioned compounds T is O, or S, preferably O. If T occurs more than one time in a molecule, all groups T have the same meaning.

Suitable carbene complexes are known to those skilled in the art and are described, for example, in W O 2005/019373 A2, WO 2006/056418 A2, WO 2005/113704, WO 2007/115970, WO 2007/115981 and WO 2008/000727.

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA. In a preferred embodiment of the present invention, at least one of the above-mentioned matrix materials on basis of dibenzofurane, especially at least one of the compounds of the formula X is used as matrix material.

In a preferred embodiment, the light-emitting layer is formed from 2 to 20% by weight, preferably 5 to 17% by weight, of at least one of the aforementioned emitter materials and 80 to 98% by weight, preferably 83 to 95% by weight, of at least one of the aforementioned matrix materials - in one embodiment at least one compound of the formula X - where the sum total of the emitter material and of the matrix material adds up to 100% by weight.

In a preferred embodiment, the light-emitting layer comprises a compound of formula X, such as, for example, 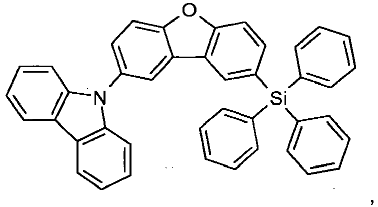 or 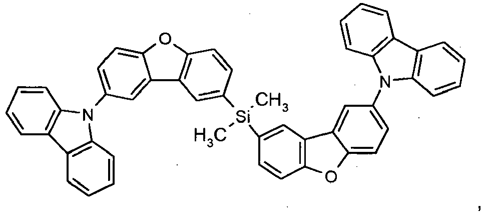 and two carbene complexes, preferably of formula 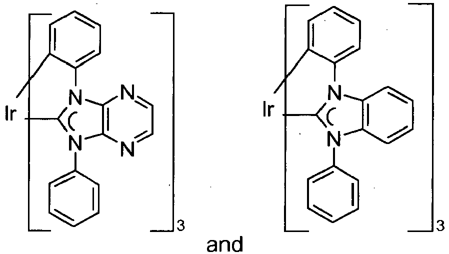 In said embodiment, the light-emitting layer is formed from 2 to 40% by weight, preferably 5 to 35% by weight, of 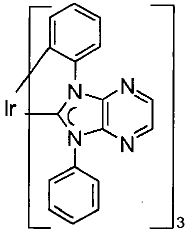 and 60 to 98% by weight, preferably 65 to 95% by weight, of a compound of the formula X and 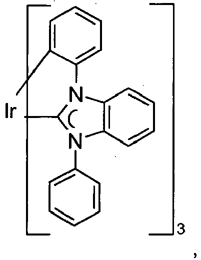 where the sum total of the carben complexes and of the compound of formula X adds up to 100% by weight.

In a further embodiment, the above-mentioned matrix materials on basis of dibenzofurane, especially the compounds of the formula X are used as hole/exciton blocker material, preferably together with carbene complexes as triplet emitters. The above-mentioned matrix materials on basis of dibenzofurane, especially compounds of the formula X may be used as matrix materials or both as matrix materials and as hole/exciton blocker materials together with carbene complexes as triplet emitters.

Suitable metal complexes for use together with the above-mentioned matrix materials on basis of dibenzofurane, especially compounds of the formula X as matrix material and/or hole/exciton blocker material, in OLEDs are thus, for example, also carbene complexes as described in WO 2005/019373 A 2 , WO 2006/056418 A2, WO 2005/113704, WO 2007/115970, WO 2007/115981 and WO 2008/000727.

Hole blocker materials typically used in OLEDs are the above-mentioned matrix materials on basis of dibenzofurane, especially compounds of formula X, 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine S,S-dioxide derivates and 1,3,5-tris(N-phenyl-2-benzylimidazolyl)benzene) (TPBI), TPBI also being suitable as electron-conducting material. Further suitable hole blockers and/or electron transport materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris-(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in WO2009003919 (PCT/EP2008/058207) and WO2009003898 (PCT/EP2008/058106) and disilyl compounds as disclosed in WO2008/034758, as a blocking layer for holes/excitons (4) or as matrix materials in the light-emitting layer (3).

In a preferred embodiment, the present invention relates to an inventive OLED comprising the layers (1) anode, (2) hole transport layer, (3) light-emitting layer, (4) blocking layer for holes/excitons, (5) electron transport layer and (6) cathode, and if appropriate further layers, wherein the electron transport layer comprises the organic metal complex of formula I and the compound of formula II, or III.

The electron transport layer (5) of the inventive OLEDs comprises the organic metal complex of formula I and the compound of formula II, or III. The layer (5) preferably improves the mobility of the electrons.

Among the materials mentioned above as hole transport materials and electron transport materials, some may fulfil several functions. For example, some of the electron-transporting materials are simultaneously hole-blocking materials when they have a low-lying HOMO. These can be used, for example, in the blocking layer for holes/excitons (4).

The charge transport layers can also be electronically doped in order to improve the transport properties of the materials used, in order firstly to make the layer thicknesses more generous (avoidance of pinholes/short circuits) and in order secondly to minimize the operating voltage of the device. For example, the hole transport materials can be doped with electron acceptors; for example, phthalocyanines or arylamines such as TPD or TDTA can be doped with tetrafluorotetracyanquinodimethane (F4-TCNQ) or with molybdenum oxide (MoOₓ), especially MoO₃, or with rhenium oxide (ReOx), especially ReO₃, or WO₃. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo. Appl. Phys. Lett., Vol. 82, No. 25, 23 June 2003 and Pfeiffer et al., Organic Electronics 2003, 4, 89 - 103. For example, the hole transport layer may, in addition to a carbene complex, e.g. Ir(dpbic)₃, be doped with molybdenum oxide (MoOₓ), especially MoO₃, or with rhenium oxide (ReOₓ), especially ReO₃, or WO₃. The cathode (6) is an electrode which serves to introduce electrons or negative charge carriers. Suitable materials for the cathode are selected from the group consisting of alkali metals of group la, for example Li, Cs, alkaline earth metals of group IIa, for example calcium, barium or magnesium, metals of group IIb of the periodic table of the elements (old IUPAC version), comprising the lanthanides and actinides, for example samarium. In addition, it is also possible to use metals such as aluminum or indium, and combinations of all metals mentioned. In addition, lithium-comprising organometallic compounds or potassium fluoride (KF) can be applied between the organic layer and the cathode in order to reduce the operating voltage.

The OLED according to the present invention may additionally comprise further layers which are known to those skilled in the art. For example, a layer which facilitates the transport of the positive charge and/or matches the band gaps of the layers to one another may be applied between the layer (2) and the light-emitting layer (3). Alternatively, this further layer may serve as a protective layer. In an analogous manner, additional layers may be present between the light-emitting layer (3) and the layer (4) in order to facilitate the transport of negative charge and/or to match the band gaps between the layers to one another. Alternatively, this layer may serve as a protective layer.

In a preferred embodiment, the inventive OLED, in addition to layers (1) to (6), comprises at least one of the following layers mentioned below:
- a hole injection layer between the anode (1) and the hole-transport layer (2);
- a blocking layer for electrons between the hole-transport layer (2) and the light-emitting layer (3);
- an electron injection layer between the electron-transport layer (5) and the cathode (6).

Materials for a hole injection layer may be selected from copper phthalocyanine, 4,4',4"-tris(N-3-methylphenyl-N-phenylamino)triphenylamine (m-MTDATA), 4,4',4"-tris(N-(2-naphthyl)-N-phenylamino)triphenylamine (2T-NATA), 4,4',4"-tris(N-(1-naphthyl)-N-phenylamino)triphenylamine (1T-NATA), 4,4',4"-tris(N,N-diphenylamino)triphenylamine (NATA), titanium oxide phthalocyanine, 2,3,5,6-tetrafluoro-7,7,8,8-tetracyanoquinodimethane (F4-TCNQ), pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxy-phenyl)amino]-9,9-spirobifluorene (MeO-Spiro-TPD), 2,2'-bis[N,N-bis(4-methoxy-phenyl)amino]-9,9-spirobifluorene (2,2'-MeO-Spiro-TPD), N,N'-diphenyl-N,N'-di-[4-(N,N-ditolylamino)phenyl]benzidine (NTNPB), N,N'-diphenyl-N,N'-di-[4-(N,N-diphenyl-amino)phenyl]benzidine (NPNPB), N,N'-di(naphthalen-2-yl)-N,N'-diphenylbenzene-1,4-diamine (α-NPP). In principle, it is possible that the hole injection layer comprises at least one of the above-mentioned matrix materials on basis of dibenzofurane, especially at least one compound of the formula X as hole injection material.

As a material for the electron injection layer, KF, or Liq, for example, can be selected. KF is more preferred than Liq.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semi-transports, typically ITO, or IZO, or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

In general, the different layers have the following thicknesses: anode (1) 50 to 500 nm, preferably 100 to 200 nm; hole-conducting layer (2) 5 to 100 nm, preferably 20 to 80 nm, light-emitting layer (3) 1 to 100 nm, preferably 10 to 80 nm, blocking layer for holes/excitons (4) 2 to 100 nm, preferably 5 to 50 nm, electron-conducting layer (5) 5 to 100 nm, preferably 20 to 80 nm, cathode (6) 20 to 1000 nm, preferably 30 to 500 nm. The relative position of the recombination zone of holes and electrons in the inventive OLED in relation to the cathode and hence the emission spectrum of the OLED can be influenced, among other factors, by the relative thickness of each layer. This means that the thickness of the electron transport layer should preferably be selected such that the position of the recombination zone is matched to the optical resonator property of the diode and hence to the emission wavelength of the emitter. The ratio of the layer thicknesses of the individual layers in the OLED depends on the materials used. The layer thicknesses of any additional layers used are known to those skilled in the art. It is possible that the electron-conducting layer and/or the hole-conducting layer have greater thicknesses than the layer thicknesses specified when they are electrically doped.

Use of the electron transport layer of the present application makes it possible to obtain OLEDs with high efficiency and with low operating voltage. Frequently, the OLEDs obtained by the use of the electron transport layer of the present application additionally have high lifetimes. The efficiency of the OLEDs can additionally be improved by optimizing the other layers of the OLEDs. Shaped substrates and novel hole-transport materials which bring about a reduction in the operating voltage or an increase in the quantum efficiency are likewise usable in the inventive OLEDs. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, laptops, digital cameras, MP3 players, vehicles and destination displays on buses and trains. Further devices in which the inventive OLEDs can be used are, for example, keyboards; items of clothing; furniture; wallpaper.

In addition, the electron transport layer of the present application can be used in OLEDs with inverse structure. The structure of inverse OLEDs and the materials typically used therein are known to those skilled in the art.

In addition, the present invention relates to an apparatus selected from the group consisting of stationary visual display units such as visual display units of computers, televisions, visual display units in printers, kitchen appliances and advertising panels, illuminations, information panels, and mobile visual display units such as visual display units in cellphones, laptops, digital cameras, MP3 players, vehicles and destination displays on buses and trains; illumination units; keyboards; items of clothing; furniture; wallpaper, comprising the inventive organic electronic device, with the electron transport layer.

The following examples are included for illustrative purposes only and do not limit the scope of the claims. Unless otherwise stated, all parts and percentages are by weight.

### Examples

### Comparative Application Example 1

The ITO substrate used as the anode is first cleaned with commercial detergents for LCD production (Deconex^{®} 20NS, and 250RGAN-ACID^{®} neutralizing agent) and then in an acetone/isopropanol mixture in an ultrasound bath. To eliminate any possible organic residues, the substrate is exposed to a continuous ozone flow in an ozone oven for a further 25 minutes. This treatment also improves the hole injection properties of the ITO. Then AJ20-1000 (commercially available from Plexcore) is spin-coated and dried to form an hole injection layer (∼40 nm).

Thereafter, the organic materials specified below are applied by vapor deposition to the clean substrate at a rate of approx. 0.5-5 nm/min at about 10⁻⁸ mbar. As a hole transport and exciton blocker, Ir(dpbic)₃ (V1) is applied to the substrate with a thickness of 45 nm, wherein the first 35 nm are doped with MoOₓ (∼50%) to improve the conductivity. 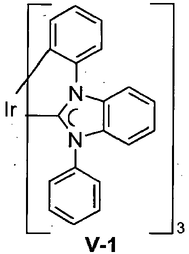 Ir(dpbic)₃ (for preparation, see Ir complex (7) in the application WO 2005/019373).

Subsequently, a mixture of 30% by weight of compound 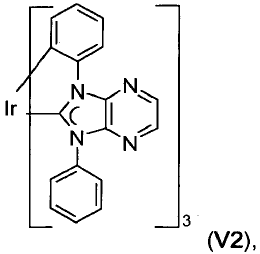 35% by weight of compound (V-1) and 35% by weight of compound 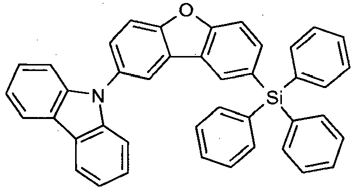 (V3, described in PCT/EP2009/067120) is applied by vapor deposition in a thickness of 20 nm.

Subsequently, the material 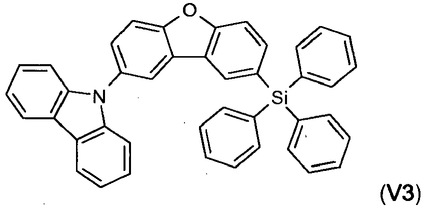 is applied by vapor deposition with a thickness of 5 nm as exciton and hole blocker.

Next, a mixture of 50 % by weight of 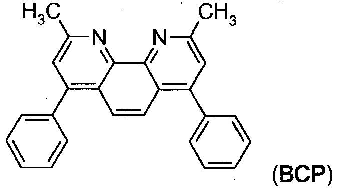 and 50 % by weight of 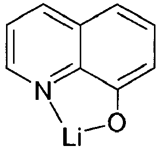 (8-hydroxyquinolinolato-lithium **(Liq))** is applied as electron transport layer by vapor deposition in a thickness of 40 nm, as are a 2 nm-thick potassium fluoride layer (electron injection layer) and finally a 100 nm-thick Al electrode.

### Comparative Application Example 2

Production and construction of an OLED as in the comparative application example 1, except compound **A-10** is used alone instead of the mixture of BCP and **Liq.**

### Comparative Application Example 3

Production and construction of an OLED as in the comparative application example 1, except **Liq** is used alone instead of the mixture of BCP and **Liq.**

### Comparative Application Example 4

Production and construction of an OLED as in the comparative application example 1, except compound **A-10** is used instead of the mixture of BCP and **Liq** and **Liq** is used instead of potassium fluoride.

### Application Example 1

Production and construction of an OLED as in the comparative application example 1, except a mixture of 50 % by weight of compound **A-10** and 50 % by weight of **Liq** is used instead of the mixture of BCP and **Liq.**

### Application Example 2

Production and construction of an OLED as in the comparative application example 1, except a mixture of 50 % by weight of compound **A-10** and 50 % by weight of **Liq** is used instead of the mixture of BCP and **Liq** and **Liq** is used instead of potassium fluoride.

### Application Example 3

The ITO substrate used as the anode is first cleaned with commercial detergents for LCD production (Deconex^{®} 20NS, and 250RGAN-ACID^{®} neutralizing agent) and then in an acetone/isopropanol mixture in an ultrasound bath. To eliminate any possible organic residues, the substrate is exposed to a continuous ozone flow in an ozone oven for a further 25 minutes. This treatment also improves the hole injection properties of the ITO. Then AJ20-1000 (commercially available from Plexcore) is spin-coated and dried to form an hole injection layer (∼40 nm).

Thereafter, the organic materials specified below are applied by vapor deposition to the clean substrate at a rate of approx. 0.5-5 nm/min at about 10⁻⁸ mbar. As a hole transport and exciton blocker, Ir(dpbic)₃ **(V1)** is applied to the substrate with a thickness of 40 nm, wherein the first 35 nm are doped with MoOₓ (∼50%) to improve the conductivity. Subsequently, a mixture of 30% by weight of compound (*V2*), 35% by weight of compound **(V1)** and 35% by weight of compound **(V3)** is applied by vapor deposition in a thickness of 20 nm. Subsequently, the material **(V3)** is applied by vapor deposition with a thickness of 5 nm as exciton and hole blocker.

Next, a mixture of 50 % by weight of compound **(A-10)** and 50 % by weight of Liq is applied as electron transport layer by vapor deposition in a thickness of 40 nm, as are a 1 nm-thick **Liq** layer (electron injection layer) and finally a 100 nm-thick Al electrode.

### Comparative Application Example 5

Production and construction of an OLED as in the application example 3, except that compound 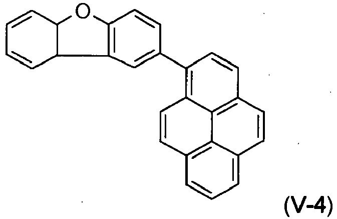 is used instead of compound **A-10.**

To characterize the OLED, electroluminescence spectra are recorded at various currents and voltages. In addition, the current-voltage characteristic is measured in combination with the light output emitted. The light output can be converted to photometric parameters by calibration with a photometer. To determine the lifetime, the OLED is operated at a constant current density and the decrease in the light output is recorded. The lifetime is defined as that time which lapses until the luminance decreases to half of the initial luminance.

V at 300 cd/m², Im/W at 300 cd/m², EQE (%) at 300 cd/m² and lifetime (h) at 300 cd/m² measured for the devices of the Application Examples and Comparative Application Examples are shown in the Tables 1-1 to 1-5 below, wherein the measured data of the Comparative Application Examples are set to 100 and the data of the Application Examples are specified in relation to those of the Comparative Application Examples.

**Table 1-1**

| **Device** | **ET Layer** | **EI Layer** | **V at 300 cd/m²** | **Im/W at 300 cd/m²** | **EQE²) (%) at 300 cd/m²** | **Lifetime (h) at 300 cd/m²** | **Color** |
|---|---|---|---|---|---|---|---|
| **Appl. Ex. 1** | **Liq¹⁾** | KF | 85 | 170 | 140 | 750 | X=0.171 |
| | Cpd. **A-10¹⁾** | | | | | | Y=0.294 |
| **Comp. Appl. Ex. 1** | **Liq¹⁾** | KF | 100 | 100 | 100 | 100 | X=0.167 |
| | **BCP¹⁾** | | | | | | Y=0.282 |

**Table 1-2**

| **Device** | **ET Layer** | **EI Layer** | **V at 300 cd/m²** | **Im/W at 300 cd/m²** | **EQE²) (%) at 300 cd/m2** | **Lifetime (h) at 300 cd/m²** | **Color** |
|---|---|---|---|---|---|---|---|
| **Appl. Ex. 1** | **Liq**¹⁾ | KF | 60 | 160 | 100 | 140 | X=0.171 |
| | Cpd. **A-10¹⁾** | | | | | | Y=0.294 |
| **Comp. Appl. Ex. 2** | Cpd. **A-10** | KF | 100 | 100 | 100 | 100 | X=0.171 |
| | | | | | | | Y=0.288 |

**Table 1-3**

| **Device** | **ET Layer** | **EI Layer** | **V at 300 cd/m²** | **Im/W at 300 cd/m²** | **EQE²⁾ (%) at 300 cd/m²** | **Lifetime (h) at 300** | **Color** |
|---|---|---|---|---|---|---|---|
| **Appl. Ex. 1** | **Liq**¹⁾ | KF | 40 | 260 | 112 | 360 | X=0.171 |
| | Cpd. **A-10¹⁾** | | | | | | Y=0.294 |
| **Comp. Appl. Ex. 3** | **Liq** | KF | 100 | 100 | 100 | 100 | X=0.172 |
| | | | | | | | Y=0.300 |

**Table 1-4**

| **Device** | **ET Layer** | **EI Layer** | **V at 300 cd/m²** | **Im/W at 300 cd/m²** | **EQE²) (%) at 300 cd/m²** | **Lifetime (h) at 300 cd/m²** | **Color** |
|---|---|---|---|---|---|---|---|
| **Appl. Ex. 2** | **Liq¹⁾** | **Liq** | 35 | 2400 | 800 | 5600 | X=0.174 |
| | Cpd. **A-10¹⁾** | | | | | | Y=0.296 |
| **Comp. Appl. Ex.4** | Cpd. **A-10** | **Liq** | 100 | 100 | 100 | 100 | X=0.169 |
| | | | | | | | Y=0.281 |

**Table 1-5**

| **Device** | **ET Layer** | **EI Layer** | **V at 300 cd/m²** | **Im/W at 300 cd/m²** | **EQE²) (%) at 300 cd/m²** | **Lifetime (h) at 300 cd/m²** | **Color** |
|---|---|---|---|---|---|---|---|
| **Appl. Ex. 3** | **Liq¹⁾** | **Liq** | 90 | 110 | 102 | 1650 | X=0.170 |
| | Cpd. **A-10¹⁾** | | | | | | Y=0.290 |
| **Comp. Appl. Ex. 4** | **Liq¹⁾** | **Liq** | 100 | 100 | 100 | 100 | X=0.170 |
| | Cpd. **V-4** | | | | | | Y=0.300 |

| | | | | | | | |
|---|---|---|---|---|---|---|---|
| ¹⁾ 50% by weight. ²⁾ External quantum efficiency (EQE) is # a of generated photons escaped from a substance or a device / # of electrons flowing through it. ET Layer = Electron Transport Layer. EI Layer = Electron Injection Layer. | | | | | | | |

The life time, power efficiency, quantum efficiency and/or voltage at 300 cd/m² of the devices of the Application Examples are superior as compared with the devices of the Comparative Application Examples.

## Claims

1. An organic electronic device including a first electrode, a second electrode, and an organic layer interposed between the first electrode and the second electrode, wherein the organic layer comprises
an organic metal complex of formula 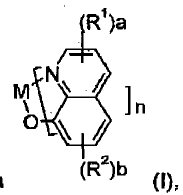 and
a compound of formula 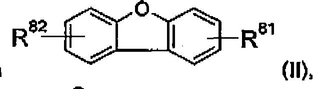 or 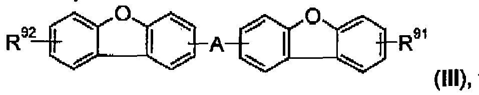 wherein
R¹ and R² are independenly of each other F, C₁-C₈alkyl, or C₆-C₁₄aryl, which may optionally be substituted by one, or more C₁-C₈alkyl groups, or
two substituents R¹ and/or R² combine to form a fused benzene ring group, which may optionally be substituted by one, or more C₁-C₈alkyl groups,
a and b are independently of each other 0, or an integer 1 to 3,
R⁸¹ is phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
R⁸² is phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
R⁹¹ is phenanthryl, pyrenyl, triphenylenyl, 1,10-phenanthrolinyl, triazinyl, dibenzothiophenyl, pyrimidinyl, or dibenzofuranyl, each of which may optionally be substituted by one, or more C₁-C₁₈alkyl, C₆-C₁₄aryl, or C₂-C₂₀heteroaryl groups,
R⁹² is H, phenanthryl, pyrenyl, triphenylenyl, 1,10-phenanthrolinyl, triazinyl, dibenzothiophenyl, or pyrimidinyl, each of which may optionally be substituted by one, or more C₁-C₁₈alkyl, C₆-C₁₄aryl, or C₂-C₂₀heteroaryl groups,
A is a single bond, an arylene, or heteroarylene group, each of which may optionally be substituted by one, or more C₁-C₁₈alkyl groups; or -SiR⁸³R⁸⁴-, wherein R⁸³ and R⁸⁴ are independently of each other a C₁-C₁₈alkyl, or a C₆-C₁₄aryl group, which may optionally be substituted by one, or more C₁-C₁₈alkyl groups;
M is an alkali metal atom, or an earth alkaline metal atom,
n is 1, if M is an alkali metal atom, n is 2, if M is an earth alkaline metal atom, wherein the organic layer comprising the organic metal complex of formula I and the compound of formula II, or III constitutes the electron transport layer.

2. The organic electronic device according to claim 1, wherein the compound of formula II is a compound of formula 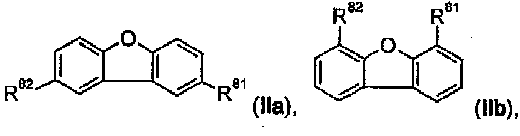 or 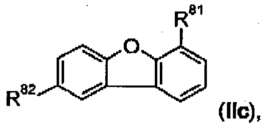 and the compound of formula III is a compound or formula 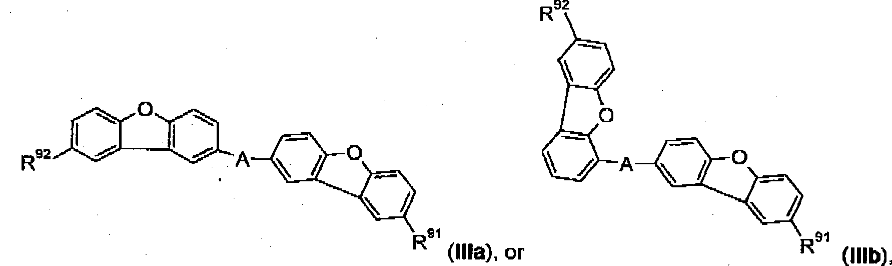 wherein A, R⁸¹, R⁸², R⁹¹ and R⁹² are as defined in claim 1.

3. The organic electronic device according to claim 2, wherein R⁸¹ is 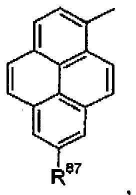 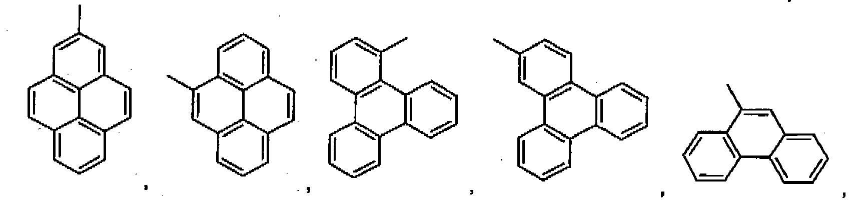 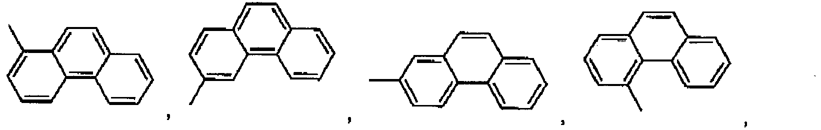 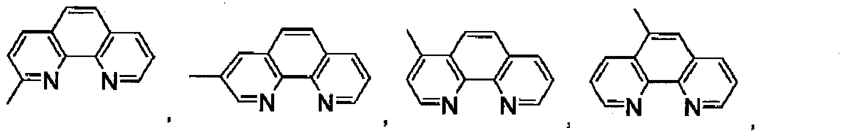 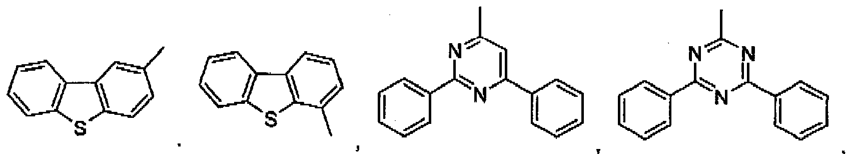 or 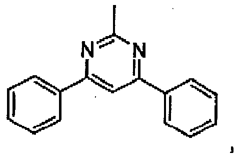 wherein R⁸⁷ is H, or C₁-C₈alkyl;
R⁸² has the meaning of R⁸¹,
R⁹¹ has the meaning of R⁸¹, or is 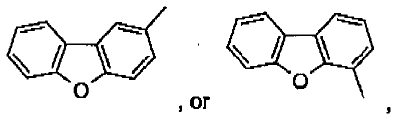 R⁹² has the meaning of R⁸¹, or is H.

4. The organic electronic device according to any of claims 1 to 3, wherein A is a single bond, a group -SIR⁸³R⁸⁴-, a group of formula 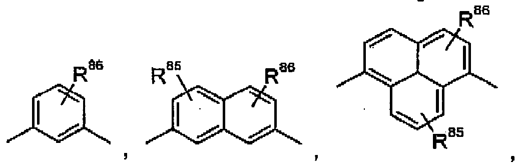 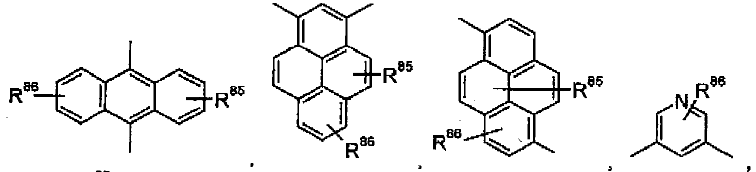 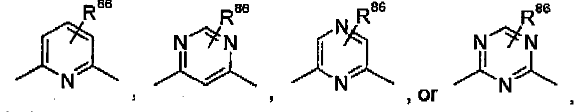 wherein R⁸⁵ and R⁸⁶ are independently of each other H, or a C₁-C₁₈alkyl group and R⁸³ and R⁸⁴ are independently of each other a C₁-C₈alkyl group.

5. The organic electronic device according to any of claims 1 to 4, wherein the compound of formula II, or III is a compound of formula 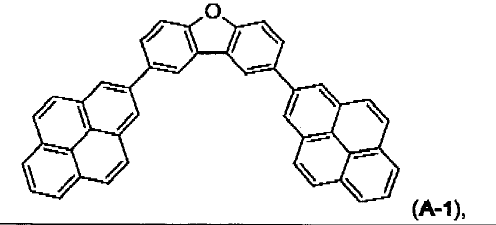 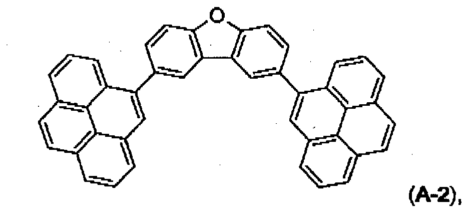 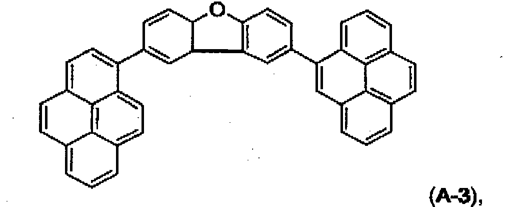 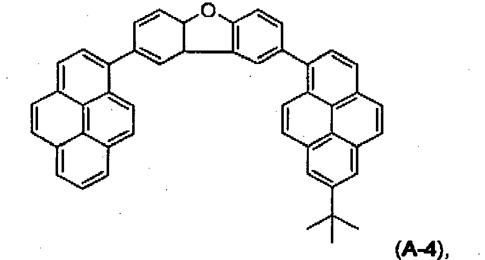 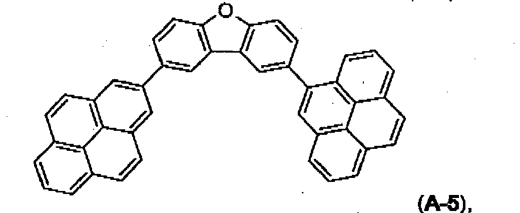 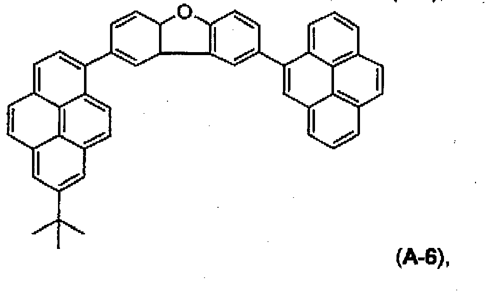 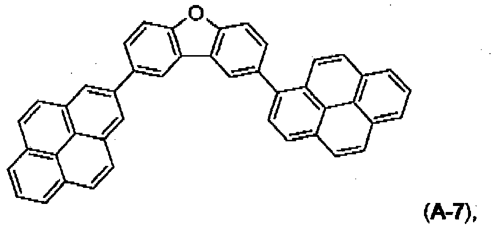 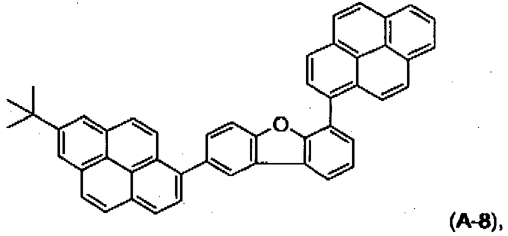 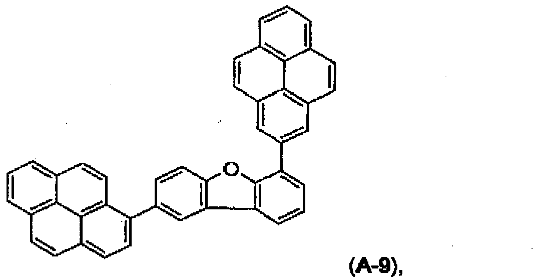 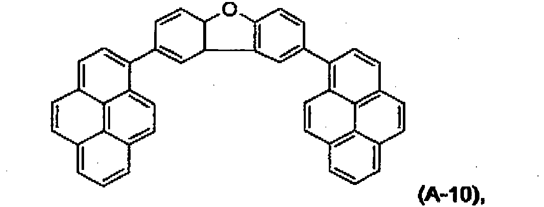 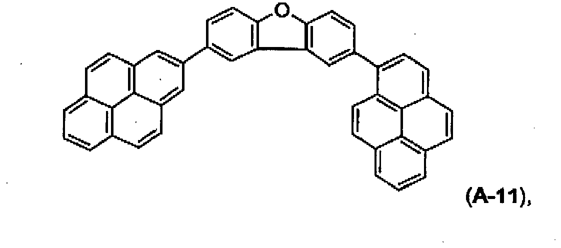 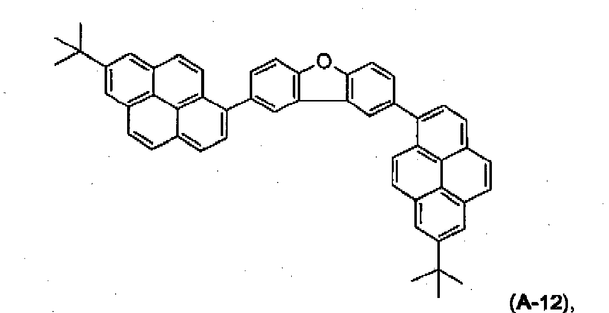 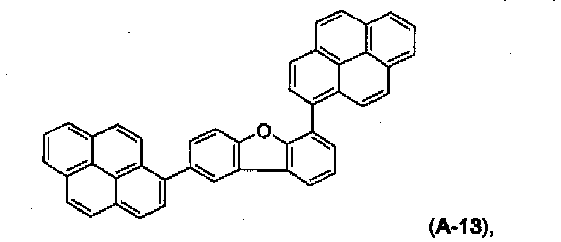 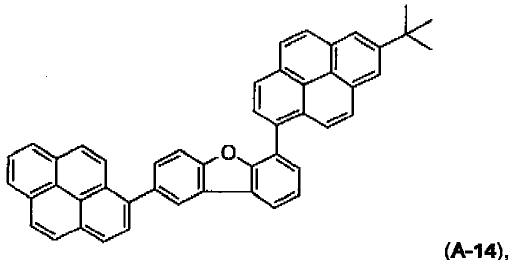 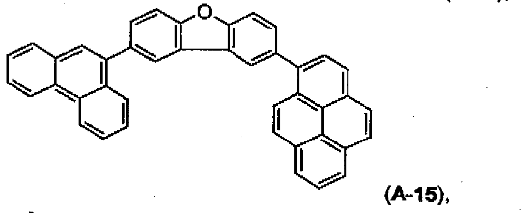 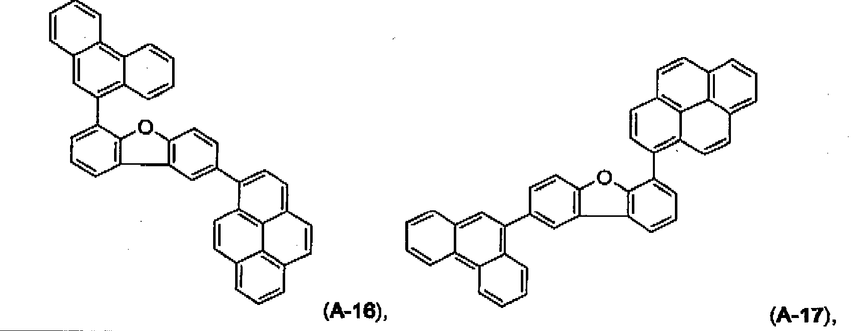 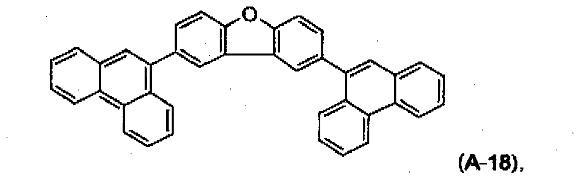 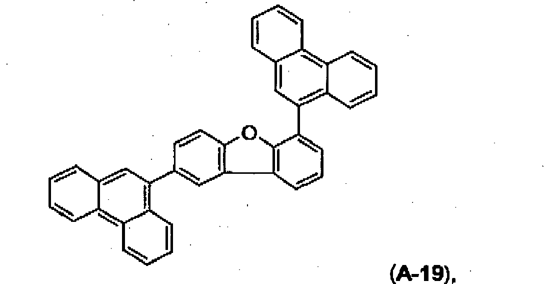 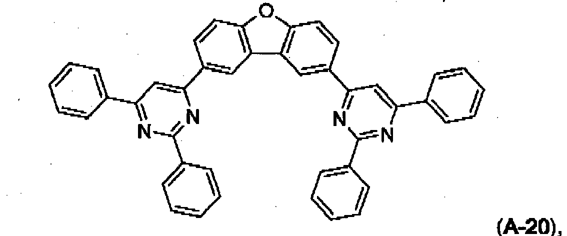 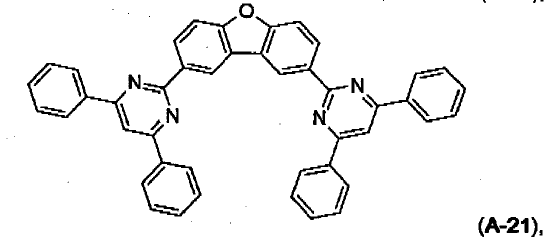 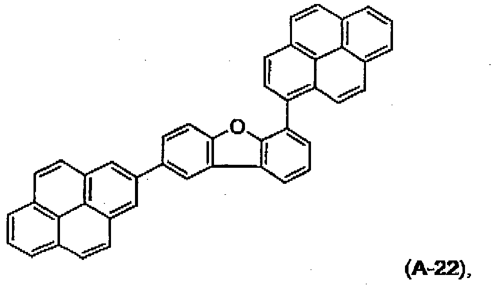 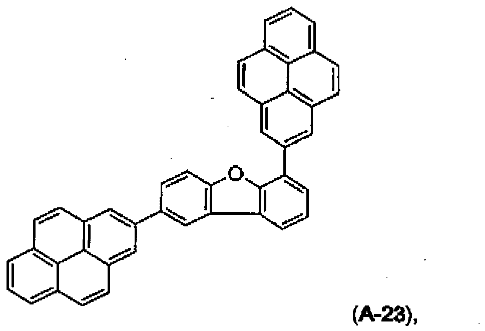 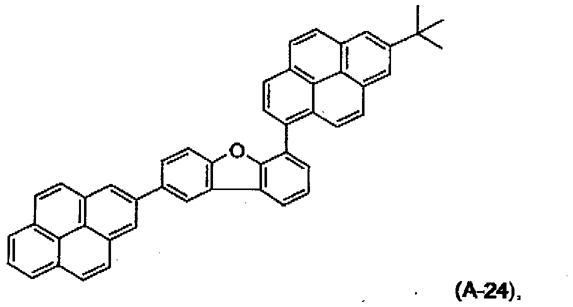 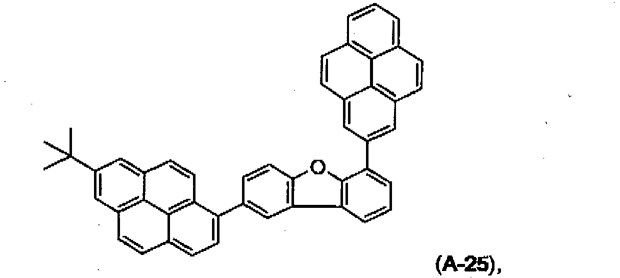 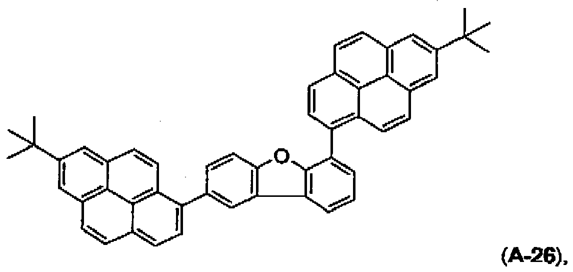 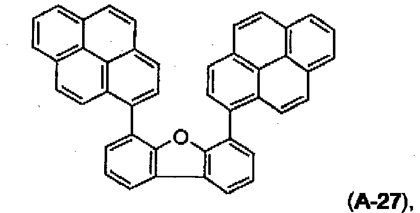 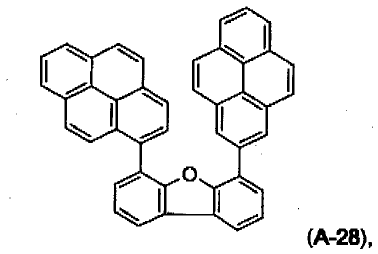 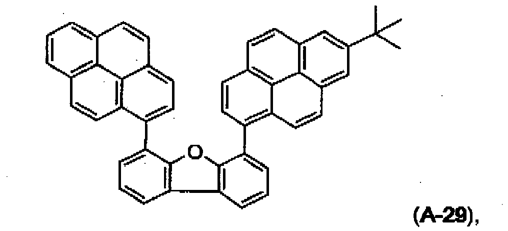 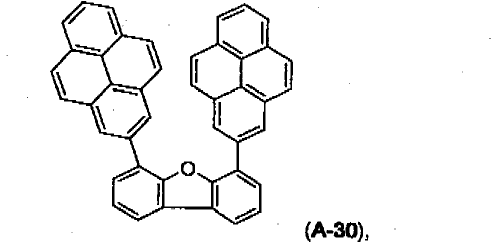 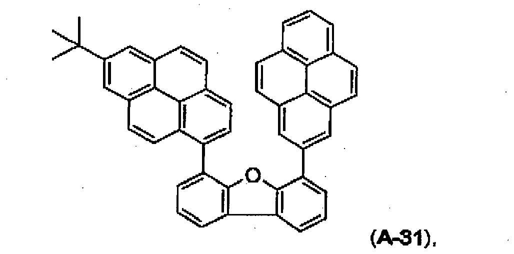 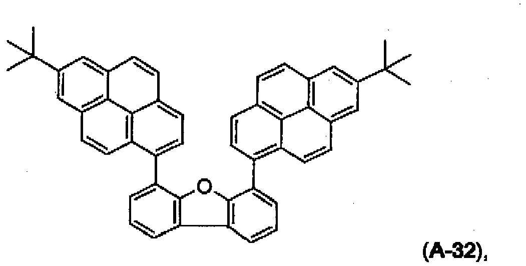 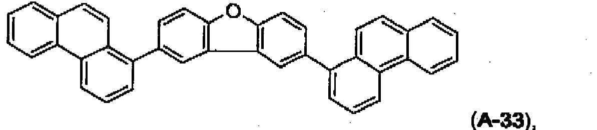 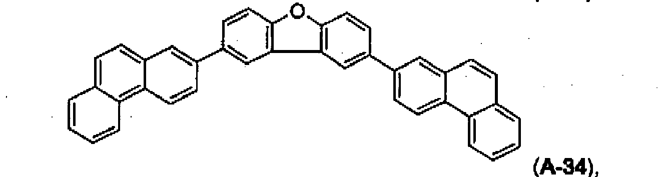 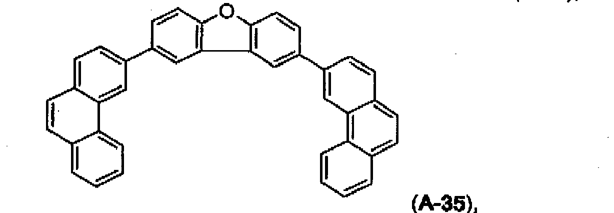 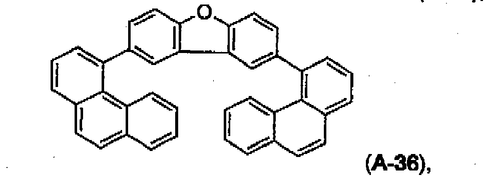 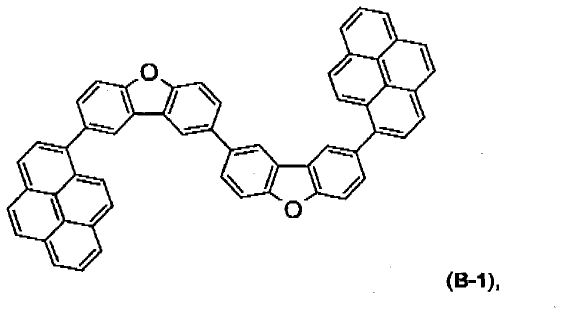 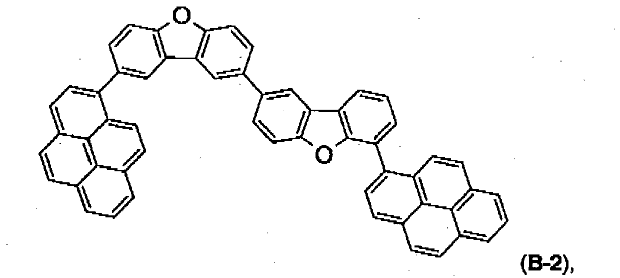 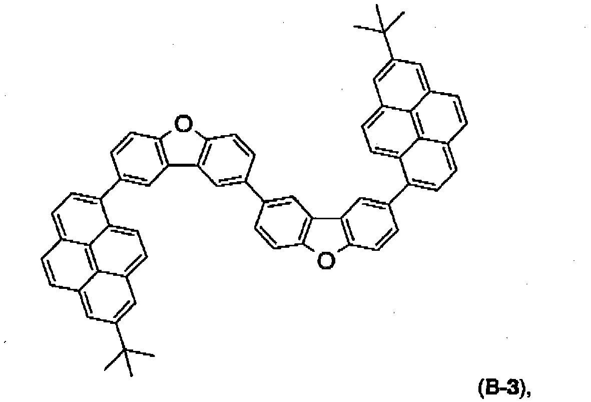 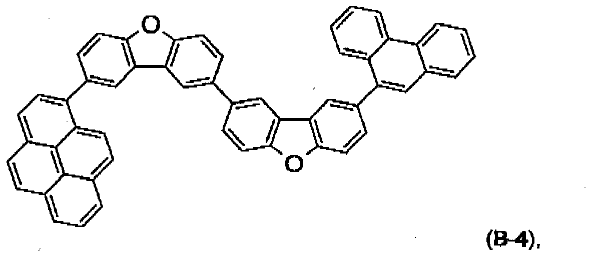 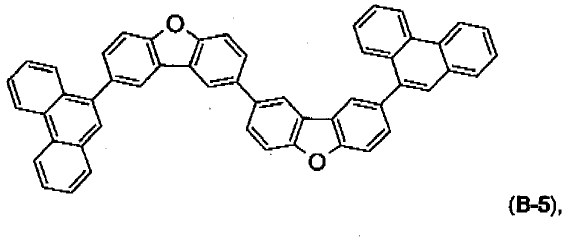 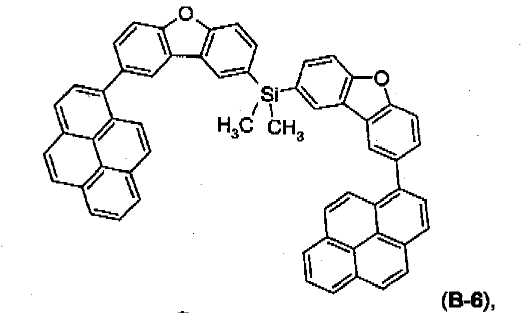 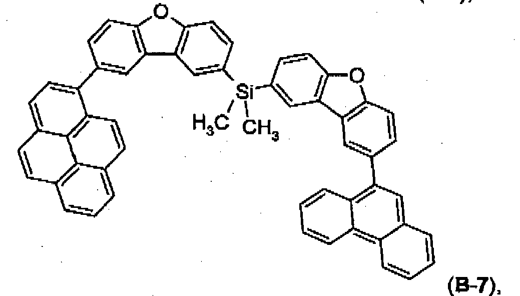 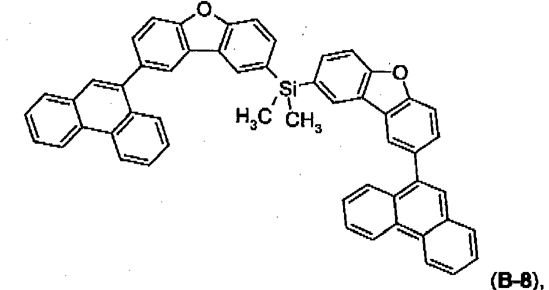 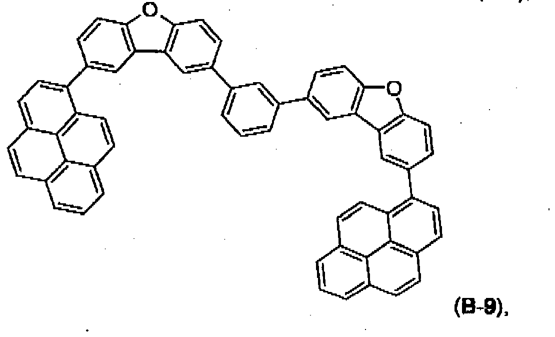 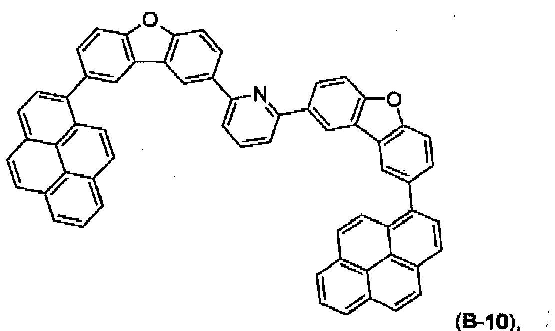 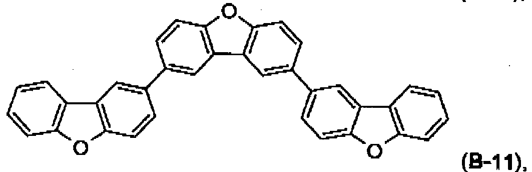 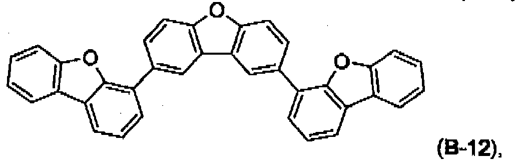 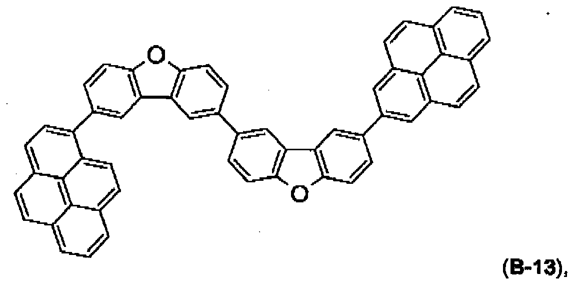 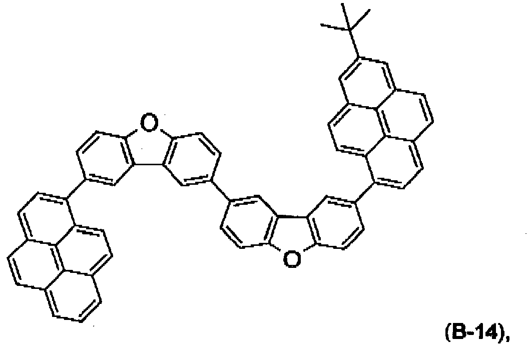 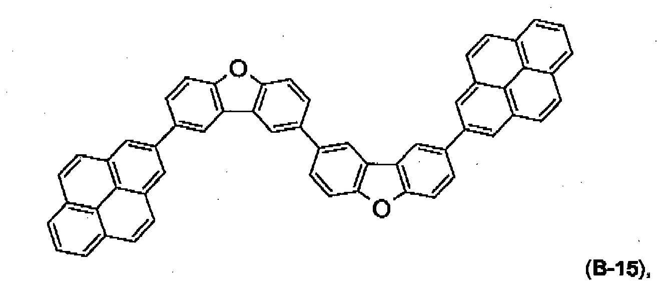 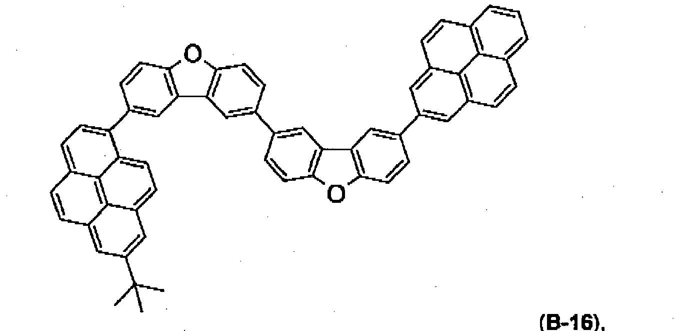 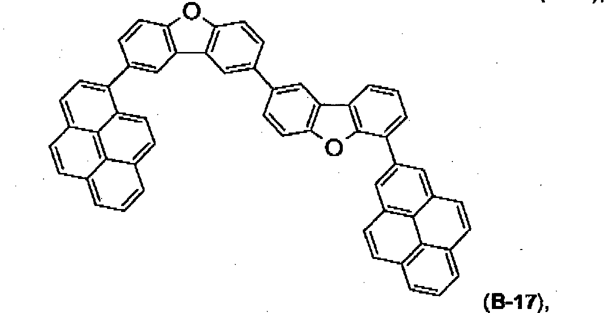 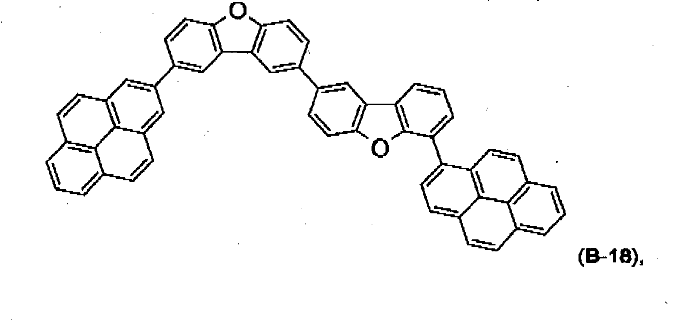 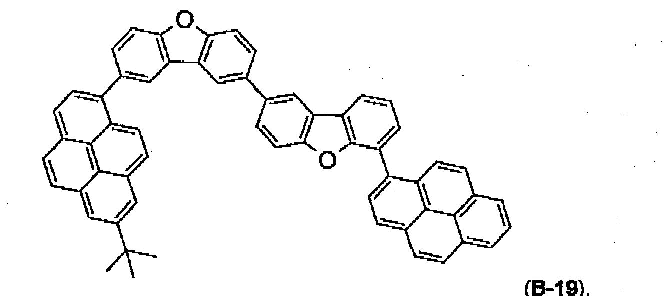 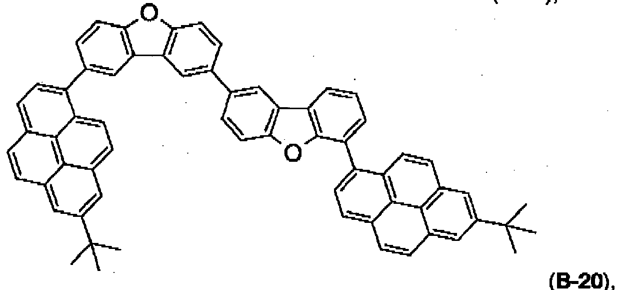 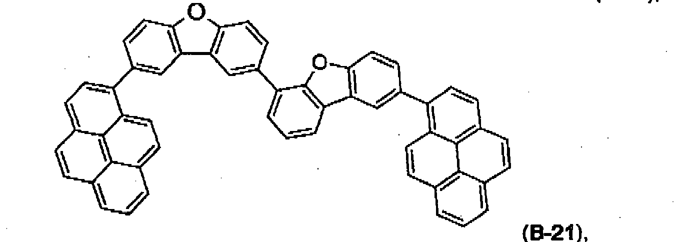 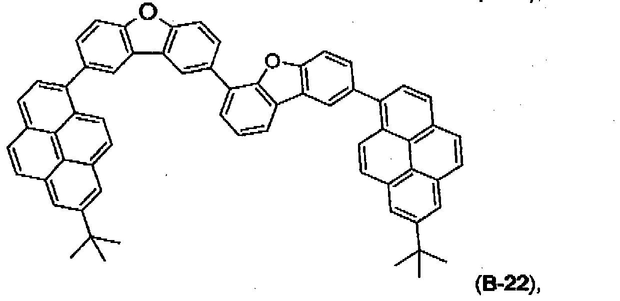 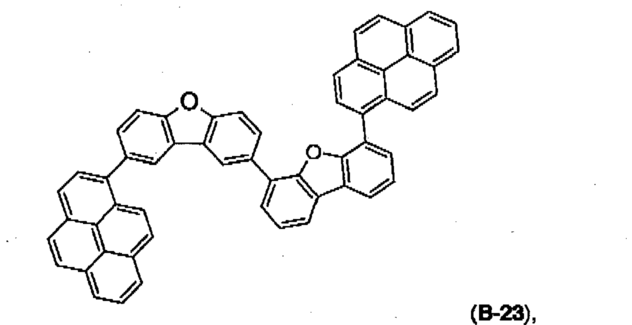 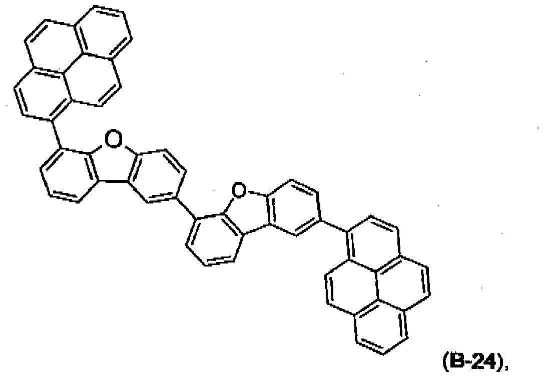 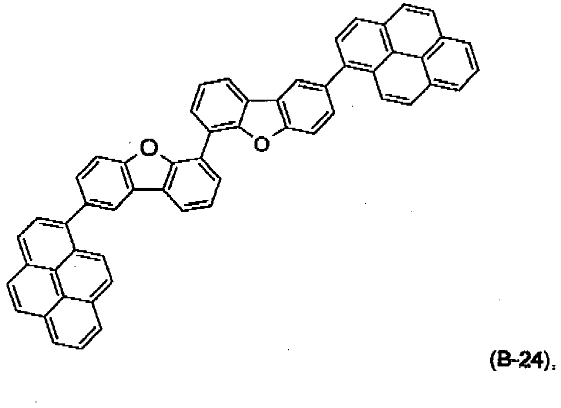 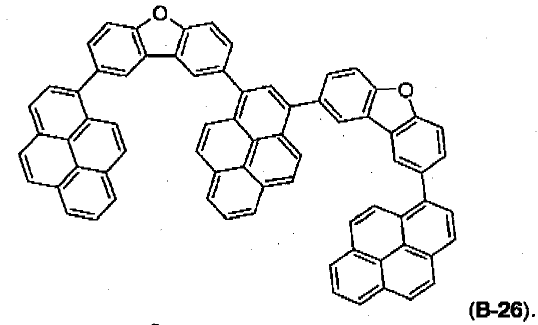 or

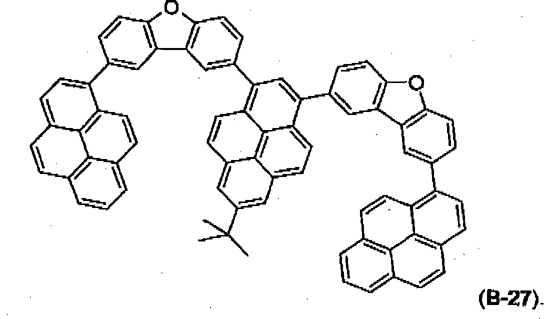

6. The organic electronic device according to any of claims 1 to 5, wherein the compound of
formule I is a compound of formula

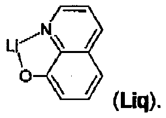

7. The organic electronic device according to any of claims 1 to 6, which is an organic light emitting device, comprising an anode, a hole injection layer, a hole transport layer, a light emitting layer, a hole and exciton blocking layer, an electron transport layer, an electron injection layer and a cathode, wherein the organic layer comprising the compounds of formula I and II, or III constitutes the electron transport layer.

8. The organic electronic device according to claim 7, wherein the electron transport layer
comprises a mixture of a compound of formula 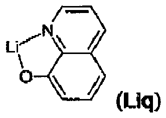 and a compound of formula

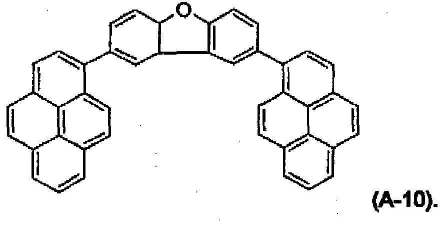

9. The organic electronic device according to claim 7, or 8, wherein the electron injection layer comprises, or consists of potassium fluoride, or Liq.

10. The organic electronic device according to any of claims 7 to 9, wherein the light emitting
layer comprises a compound of the formula 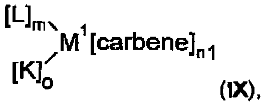 wherein the symbols have the following meanings:
M¹ is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a *π* complex with M¹;
N1 is the number of carbene ligands, where n1 is at least 1 and when n1 > 1 the carbene ligands in the complex of the formula I can be identical or different;
m is the number of ligands L, where m can be 0 or ≥ 1 and when m > 1 the ligands L can be identical or different;
o is the number of ligands K, where o can be 0 or ≥ 1 and when o > 1 the ligands K can be identical or different;
where the sum n1 + m + o is dependent on the oxidation state and coordination number of the metal atom and on the denticity of the ligands carbene, L and K and also on the charge on the ligands, carbene and L, with the proviso that n1 is at least 1.

11. The organic electronic device according to claim 10, wherein the compound of the formula IX is a compound of the formula

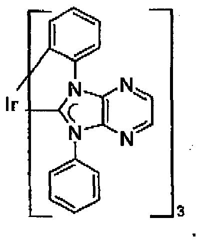

12. The organic electronic device according to any of claims 7 to 11, wherein the hole transport
layer comprises a compound of formula 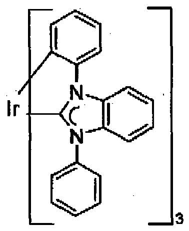 doped with molybdenum oxide (MoOₓ), especially MoO₃, or rhenium oxide (ReOₓ), especially ReO₃.

13. An electron transport layer, comprising an organic metal complex of formula I as defined in claim 1 and a compound of formula II, or III as defined in claim 1.

14. Use of the electron transport layer according to claim 13 in an organic electronic device.

15. An apparatus comprising the organic electronic device according to any of claims 1 to 12, or the electron transport layer according to claim 13.

## Patentansprüche

1. Organische elektronische Vorrichtung mit einer ersten Elektrode, einer zweiten Elektrode und einer zwischen der ersten Elektrode und der zweiten Elektrode angeordneten organischen Schicht, wobei die organische Schicht einen organischen Metallkomplex der Formel 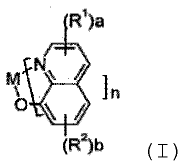 und eine Verbindung der Formel 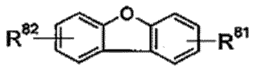 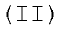 oder eine Verbindung der Formel 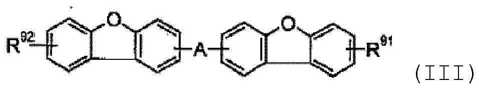 umfasst, wobei
R¹ und R² unabhängig voneinander für F, C₁-C₈-Alkyl oder C₆-C₁₈-Aryl, das gegebenenfalls durch eine oder mehrere C₁-C₈-Alkylgruppen substituiert sein kann, stehen, oder
zwei Substituenten R¹ und/oder R² zusammen eine anellierte Benzolringgruppe, die gegebenenfalls durch eine oder mehrere C₁-C₈-Alkylgruppen substituiert sein kann, bilden,
a und b unabhängig voneinander für 0 oder eine ganze Zahl von 1 bis 3 stehen,
R⁸¹ für Phenanthryl, Pyrenyl, Triphenylenyl, 1,10-Phenanthrolinyl, Triazinyl, Dibenzothiophenyl oder Pyrimidinyl steht, wobei jede dieser Gruppen gegebenenfalls durch eine oder mehrere C₁-C₁₈-Alkyl-, C₆-C₁₄-Aryl- oder C₂-C₂₀-Heteroarylgruppen substituiert sein kann,
R⁸² für Phenanthryl, Pyrenyl, Triphenylenyl, 1,10-Phenanthrolinyl, Triazinyl, Dibenzothiophenyl oder
Pyrimidinyl steht, wobei jede dieser Gruppen gegebenenfalls durch eine oder mehrere C₁-C₁₈-Alkyl-, C₆-C₁₄-Aryl- oder C₂-C₂₀-Heteroarylgruppen substituiert sein kann,
R⁹¹ für Phenanthryl, Pyrenyl, Triphenylenyl, 1,10-Phenanthrolinyl, Triazinyl, Dibenzothiophenyl, Pyrimidinyl oder Dibenzofuranyl steht, wobei jede dieser Gruppen gegebenenfalls durch eine oder mehrere C₁-C₁₈-Alkyl-, C₆-C₁₄-Aryl- oder C₂-C₂₀-Heteroarylgruppen substituiert sein kann,
R⁹² für H, Phenanthryl, Pyrenyl, Triphenylenyl, 1,10-Phenanthrolinyl, Triazinyl, Dibenzothiophenyl oder Pyrimidinyl steht, wobei jede dieser Gruppen gegebenenfalls durch eine oder mehrere C₁-C₁₈-Alkyl-, C₆-C₁₄-Aryl- oder C₂-C₂₀-Heteroarylgruppen substituiert sein kann,
A für eine Einfachbindung, eine Arylen- oder Heteroarylengruppe, die jeweils gegebenenfalls durch eine oder mehrere C₁-C₁₈-Alkylgruppen substituiert sein kann, oder -SiR⁸³R⁸⁴ steht, wobei R⁸³ und R⁸⁴ unabhängig voneinander für eine C₁-C₁₈-Alkyl- oder C₆-C₁₄-Arylgruppe stehen, die gegebenenfalls durch eine oder mehrere C₁-C₁₈-Alkylgruppen substituiert sein kann;
M für ein Alkalimetallatom oder ein Erdalkalimetallatom steht,
n für 1 steht, wenn M für ein Alkalimetallatom steht, n für 2 steht, wenn M für ein Erdalkalimetallatom steht, wobei die organische Schicht, die den organischen Metallkomplex der Formel I und die Verbindung der Formel II oder III umfasst, die Elektronentransportschicht darstellt.

2. Organische elektronische Vorrichtung nach Anspruch 1, wobei es sich bei der Verbindung der Formel II um eine Verbindung der Formel 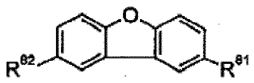 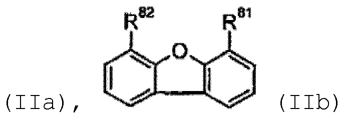 oder 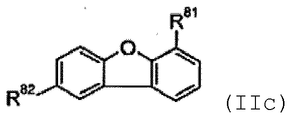 handelt und es sich bei der Verbindung der Formel III um eine Verbindung der Formel 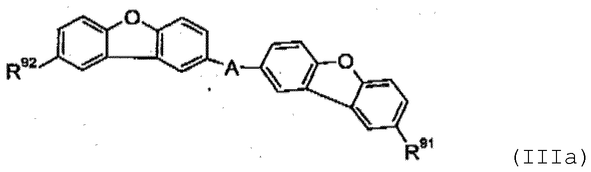 oder 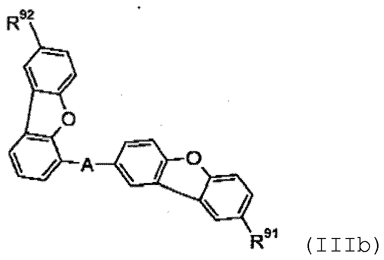 handelt, wobei A, R⁸¹, R⁸², R⁹¹ und R⁹² wie in Anspruch 1 definiert sind.

3. Organische elektronische Vorrichtung nach Anspruch 2, wobei R⁸¹ für 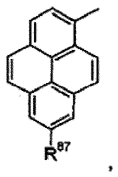 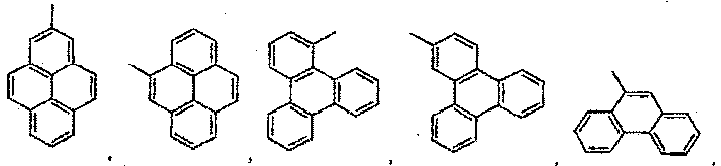 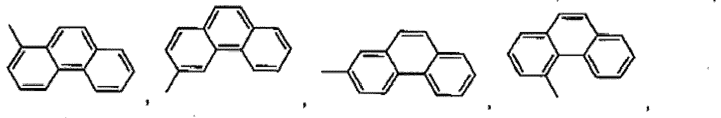 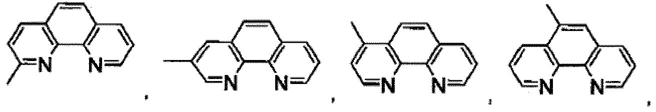 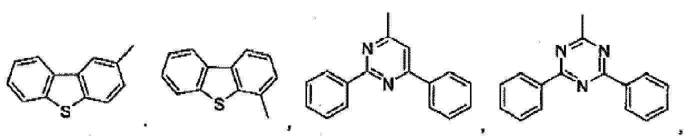 oder 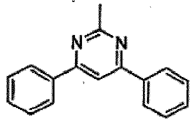 steht, wobei R⁸⁷ für H oder C₁-C₈-Alkyl steht;
R⁸² die Bedeutung von R⁸¹ hat,
R⁹¹ die Bedeutung von R⁸¹ hat oder für 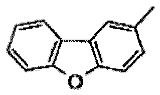 oder 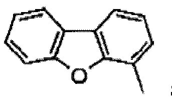 steht,
R⁹² die Bedeutung von R⁸¹ hat oder für H steht.

4. Organische elektronische Vorrichtung nach einem der Ansprüche 1 bis 3, wobei A für eine Einfachbindung, eine Gruppe -SiR⁸³R⁸⁴, eine Gruppe der Formel 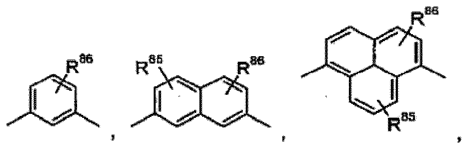 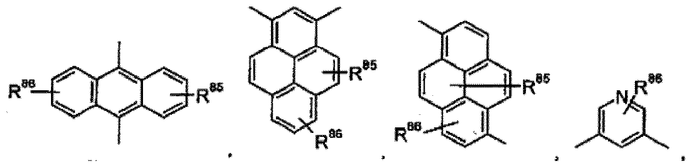 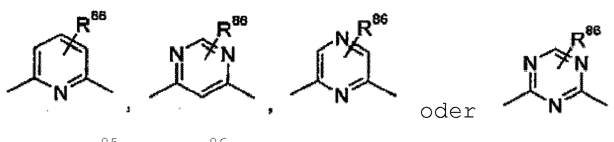 steht, wobei R⁸⁵ und R⁸⁶ unabhängig voneinander für H oder eine C₁-C₁₈-Alkylgruppe stehen und R⁸³ und R⁸⁴ unabhängig voneinander für eine C₁-C₈-Alkylgruppe stehen.

5. Organische elektronische Vorrichtung nach einem der Ansprüche 1 bis 4, wobei es sich bei der Verbindung der Formel II oder III um eine Verbindung der Formel 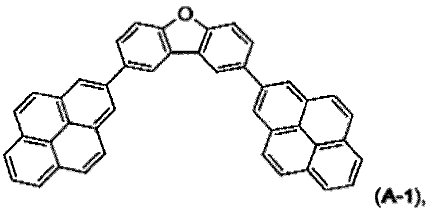 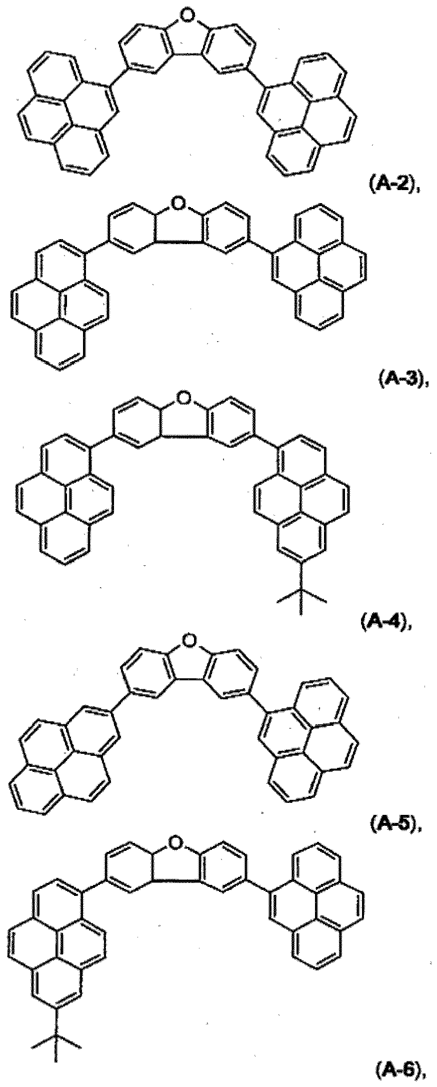 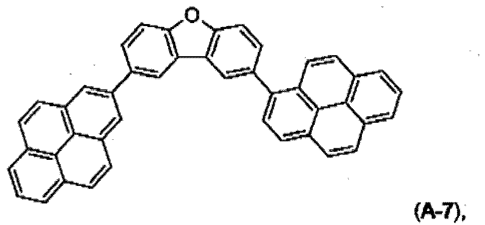 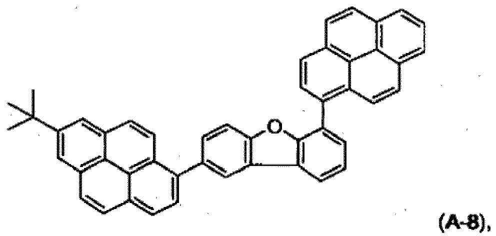 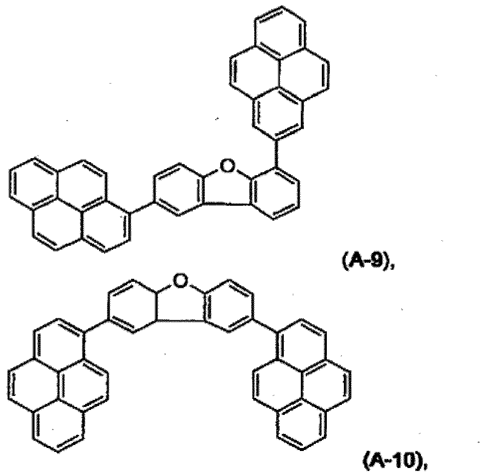 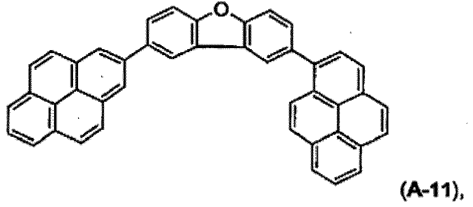 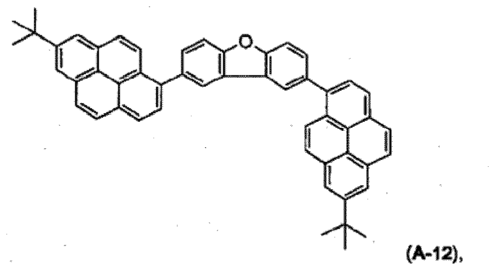 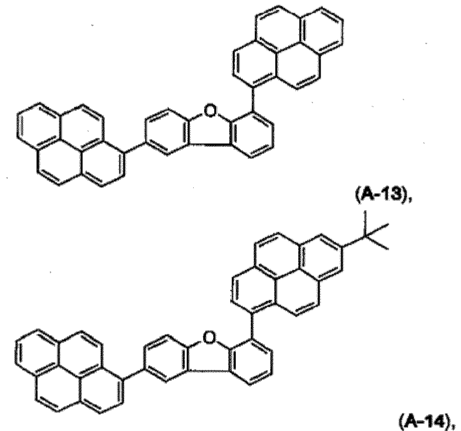 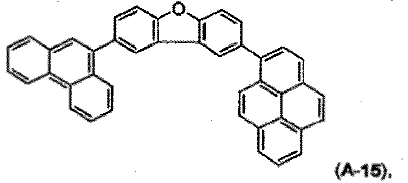 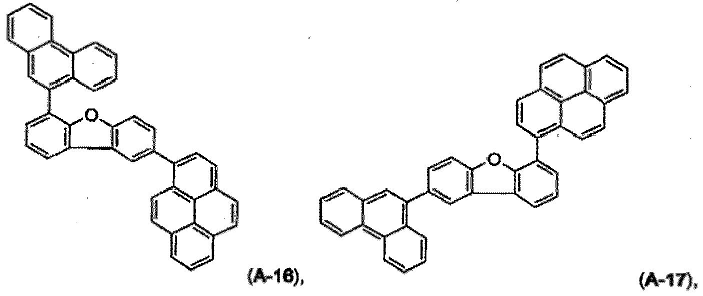 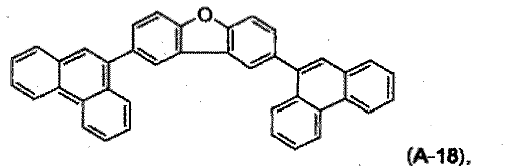 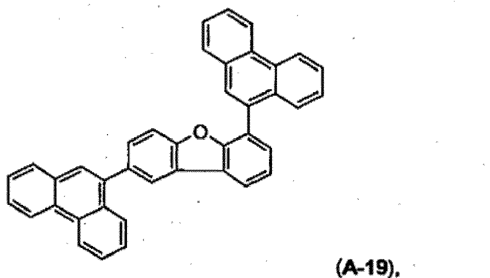 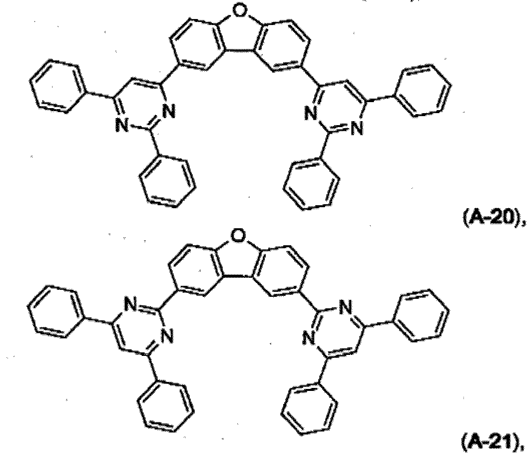 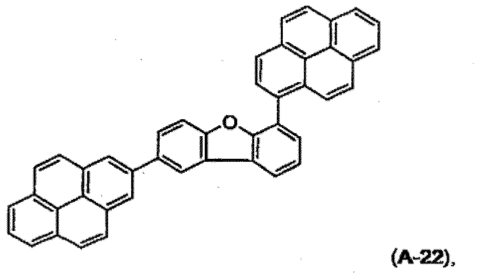 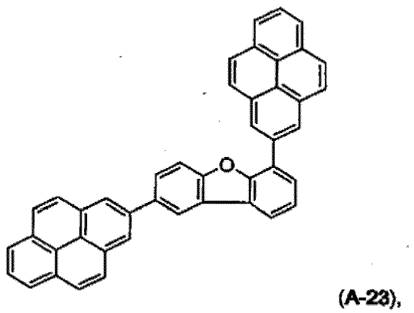 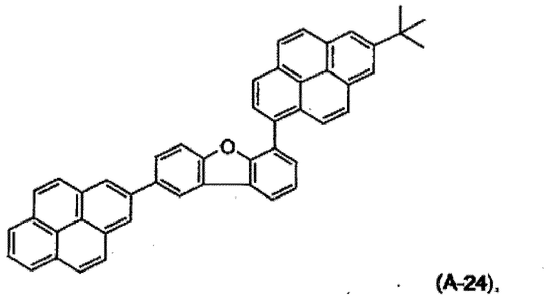 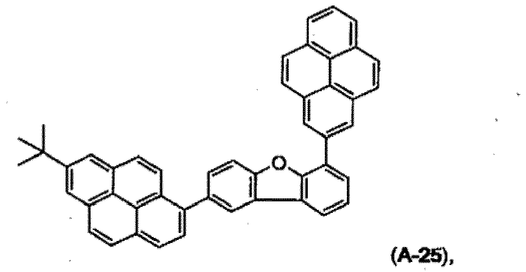 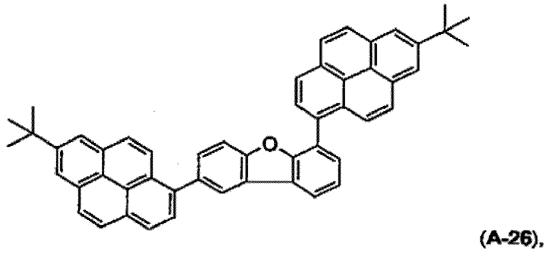 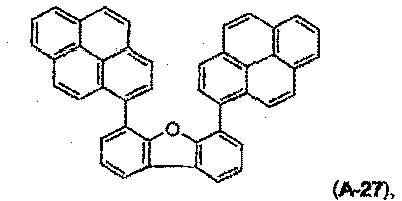 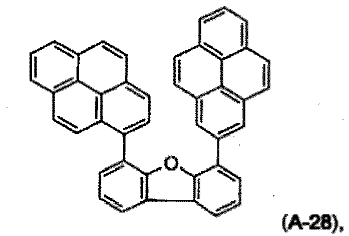 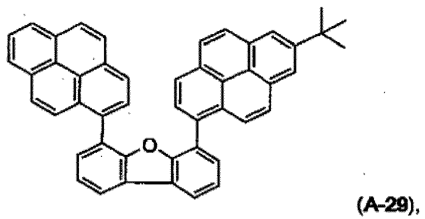 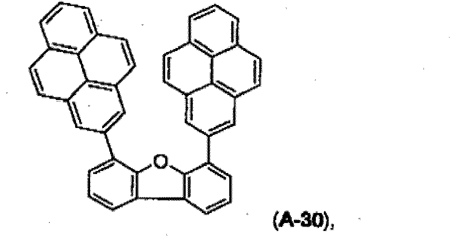 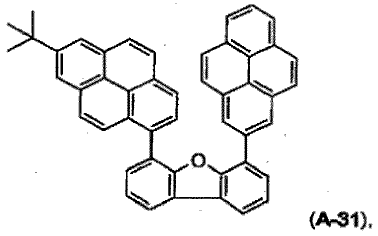 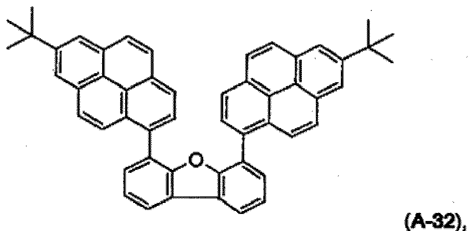 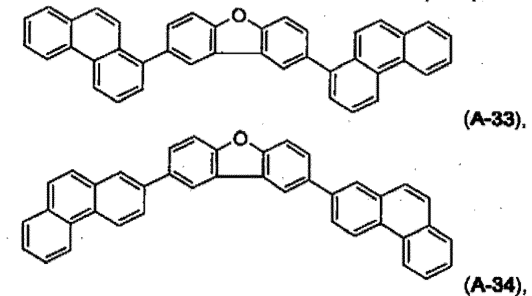 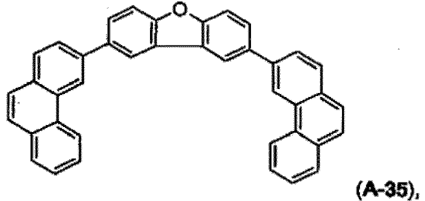 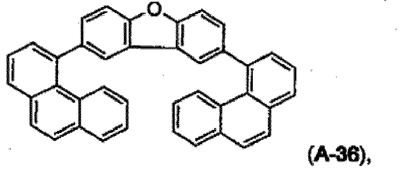 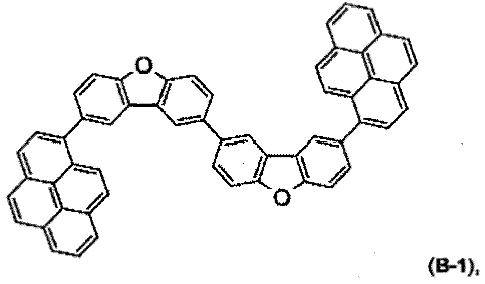 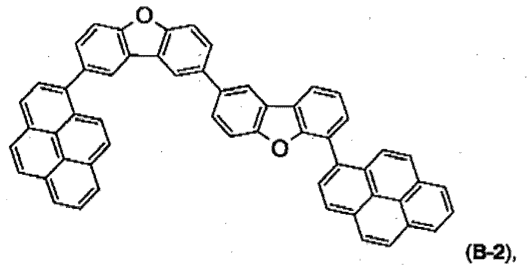 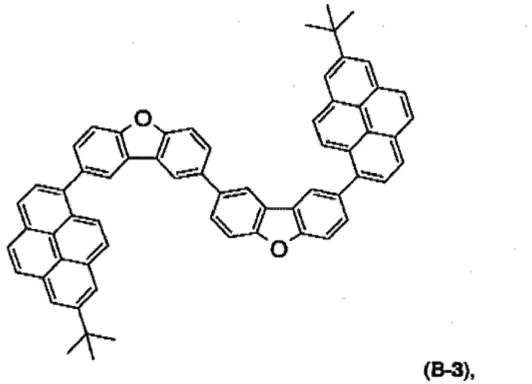 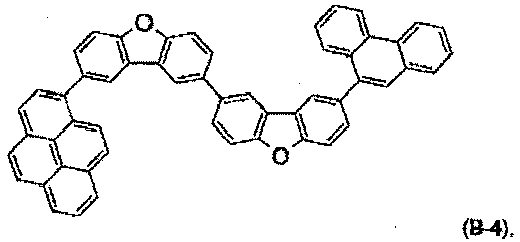 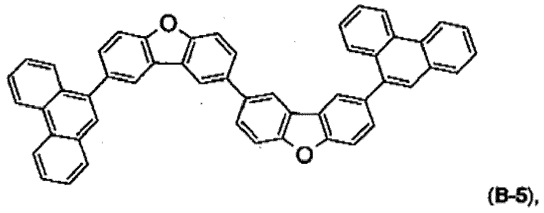 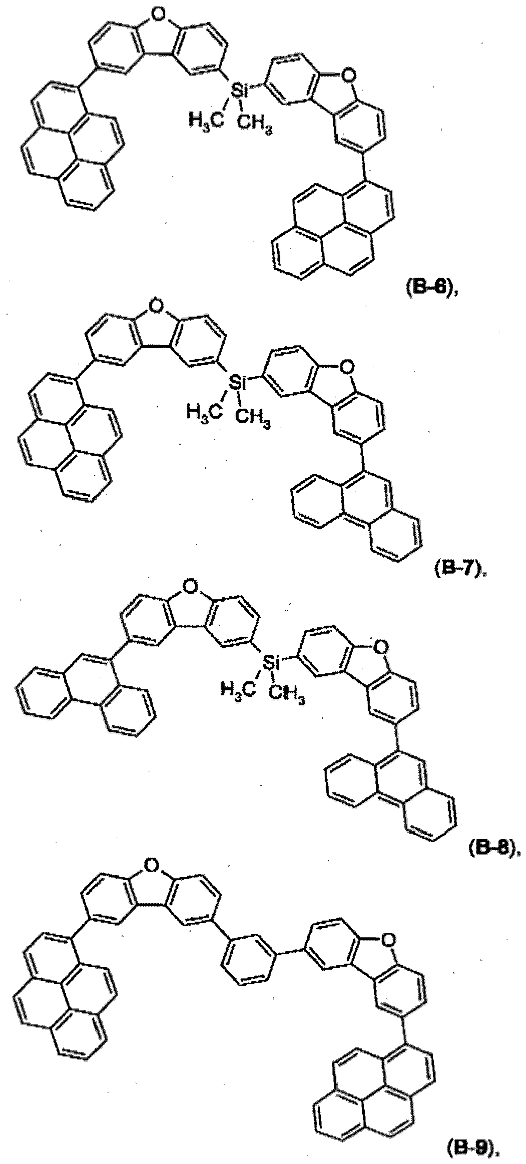 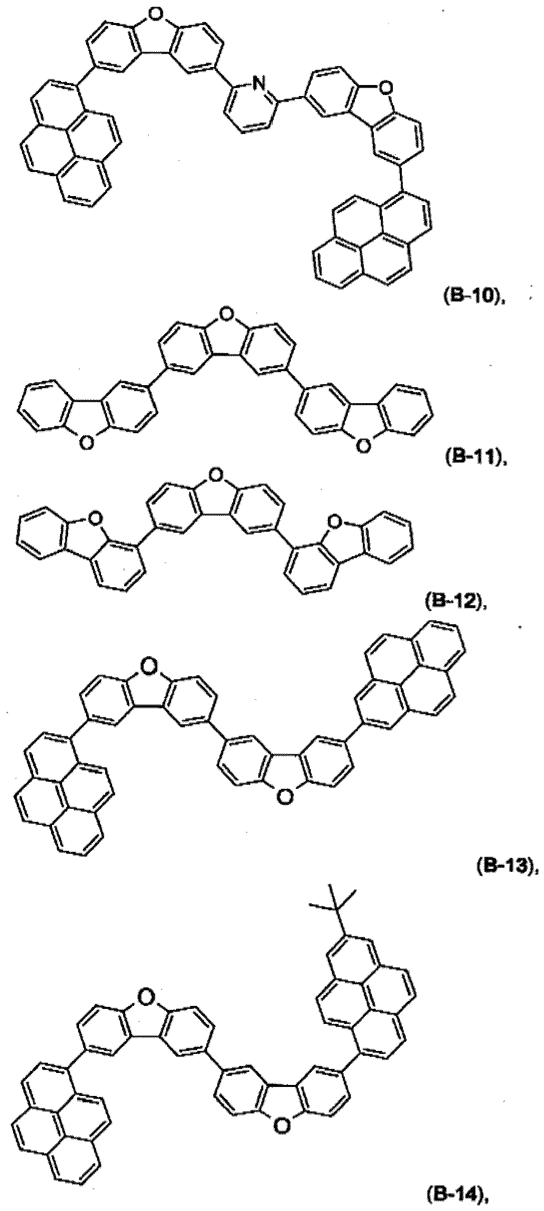 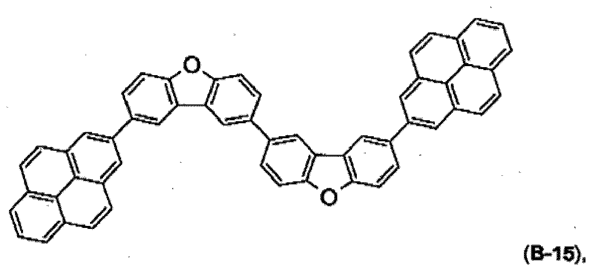 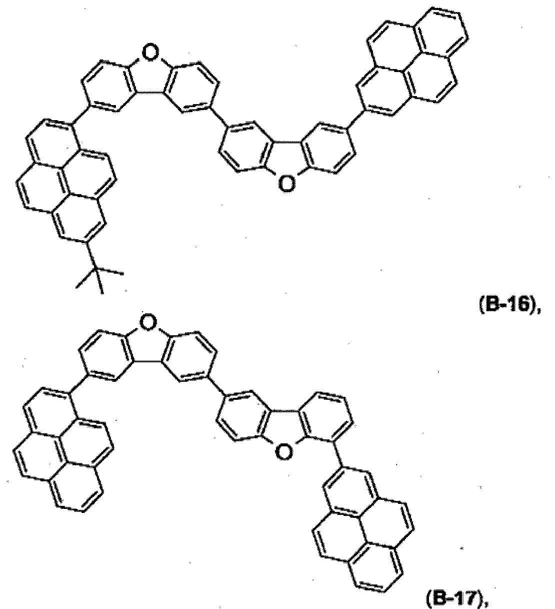 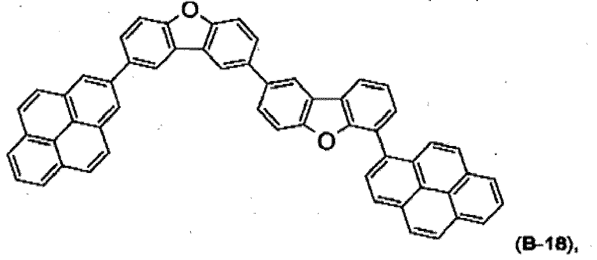 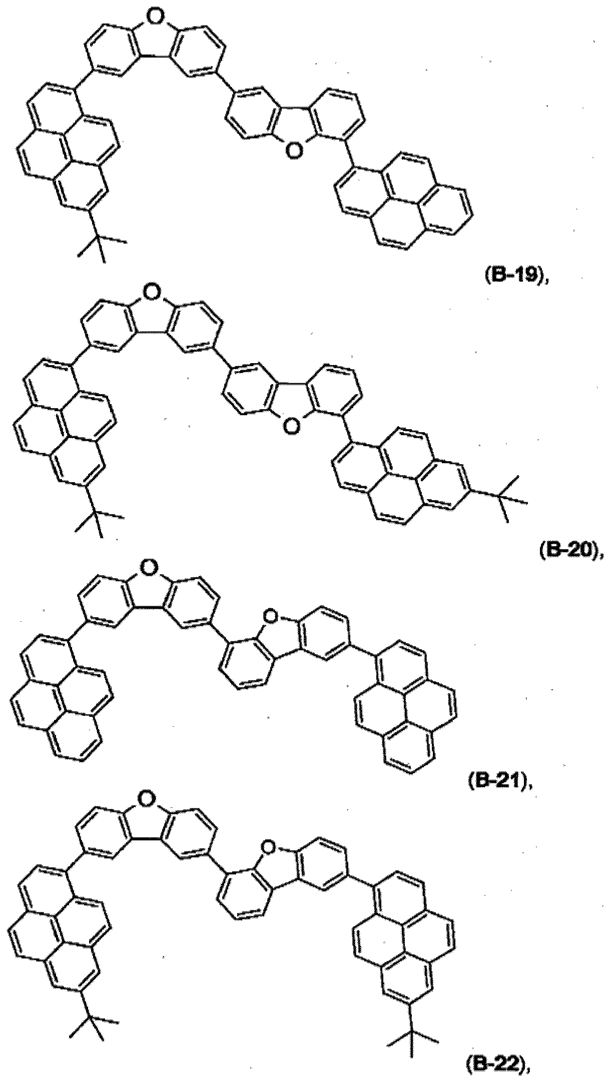 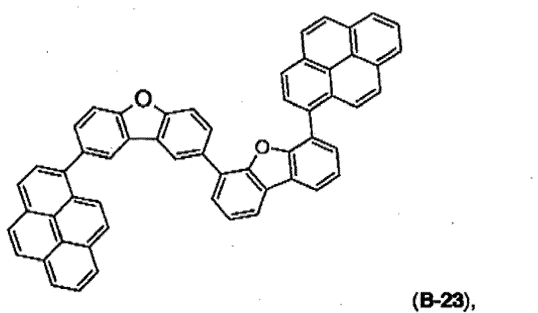 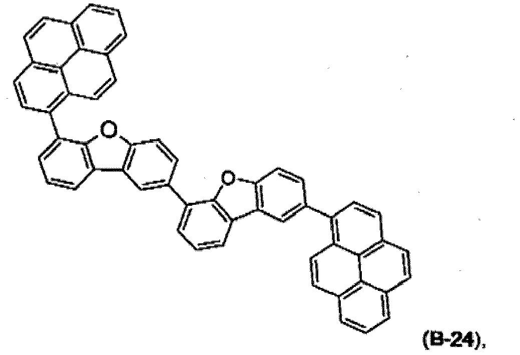 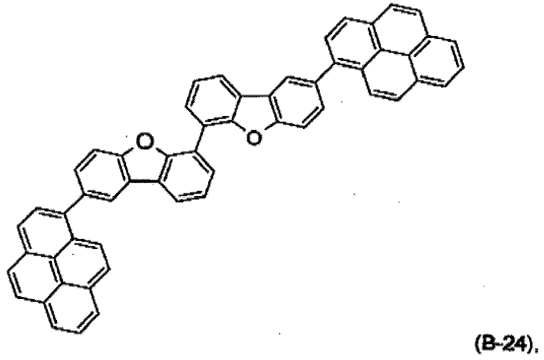 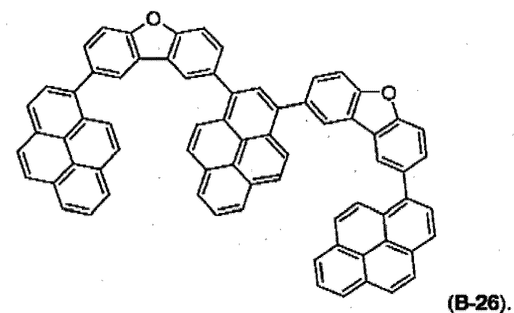 oder 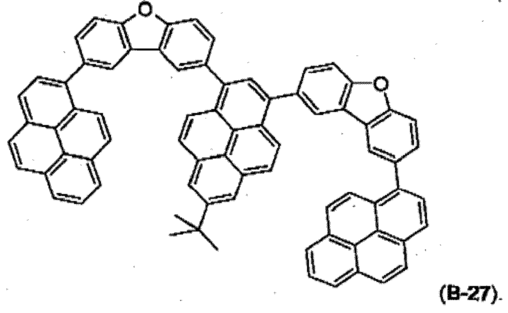 handelt.

6. Organische elektronische Vorrichtung nach einem der Ansprüche 1 bis 5, wobei es sich bei der Verbindung der Formel I um eine Verbindung der Formel 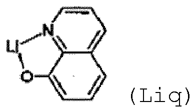 handelt.

7. Organische elektronische Vorrichtung nach einem der Ansprüche 1 bis 6, bei der es sich um eine organische lichtemittierende Vorrichtung mit einer Anode, einer Lochinjektionsschicht, einer Lochtransportschicht, einer lichtemittierenden Schicht, einer Loch- und Excitonblockierungsschicht, einer Elektronentransportschicht, einer Elektroneninjektionsschicht und einer Kathode handelt, wobei die organische Schicht, die die Verbindungen der Formel I und II oder III umfasst, die Elektronentransportschicht darstellt.

8. Organische elektronische Vorrichtung nach Anspruch 7, wobei die Elektronentransportschicht eine Mischung einer Verbindung der Formel 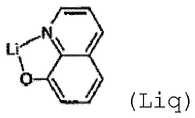 und einer Verbindung der Formel 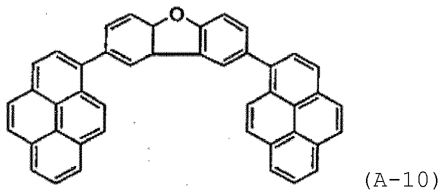 umfasst.

9. Organische elektronische Vorrichtung nach Anspruch 7 oder 8, wobei die Elektronentransportschicht Kaliumfluorid oder Liq umfasst oder aus Kaliumfluorid oder Liq besteht.

10. Organische elektronische Vorrichtung nach einem der Ansprüche 7 bis 9, wobei die lichtemittierende Schicht eine Verbindung der Formel 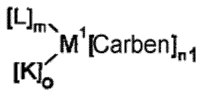 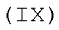 umfasst, wobei die Symbole die folgenden Bedeutungen aufweisen:
M¹ Metallatom, ausgewählt aus der Gruppe bestehend aus Co, Rh, Ir, Nb, Pd, Pt, Fe, Ru, Os, Cr, Mo, W, Mn, Tc, Re, Cu, Ag und Au in jeder für das entsprechende Metallatom möglichen Oxidationsstufe;
Carben Carbenligand, der neutral oder monoanionisch und mono-, bi- oder tridentat sein kann; wobei es sich bei dem Carbenliganden auch um einen Bis- oder Triscarbenliganden handeln kann;
L mono- oder dianionischer Ligand, der mono- oder bidentat sein kann;
K neutraler mono- oder bidentater Ligand aus der Gruppe bestehend aus Phosphinen; Phosphonaten und Derivaten davon, Arsenaten und Derivaten davon; Phosphiten; CO; Pyridinen; Nitrilen und konjugierten Dienen, die einen *π*-Komplex mit M¹ bilden;
n1 Zahl der Carbenliganden, wobei n1 mindestens 1 ist und die Carbenliganden in dem Komplex der Formel I bei n1 > 1 gleich oder verschieden sein können;
m Zahl der Liganden L, wobei m 0 oder ≥ 1 sein kann und die Liganden L bei m > 1 gleich oder verschieden sein können;
o Zahl der Liganden K, wobei o 0 oder ≥ 1 sein kann und die Liganden K bei o > 1 gleich oder verschieden sein können;
wobei die Summe n1 + m + o von der Oxidationsstufe und Koordinationszahl des Metallatoms und von der Zähnigkeit der Liganden Carben, L und K sowie von der Ladung der Liganden Carben und L abhängig ist, mit der Maßgabe, dass n1 mindestens 1 ist.

11. Organische elektronische Vorrichtung nach Anspruch 10, wobei es sich bei der Verbindung der Formel IX
um eine Verbindung der Formel 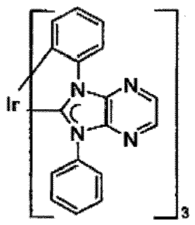 handelt.

12. Organische elektronische Vorrichtung nach einem der Ansprüche 7 bis 11, wobei die Lochtransportschicht eine Verbindung der Formel 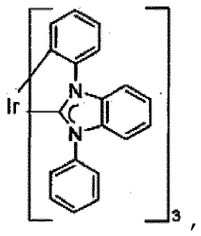 die mit Molybdänoxid (MoOₓ), insbesondere MoO₃, oder Rheniumoxid (ReOₓ), insbesondere ReO₃, dotiert ist, umfasst.

13. Elektronentransportschicht, umfassend einen organischen Metallkomplex der Formel I gemäß Anspruch 1 und eine Verbindung der Formel II oder III gemäß Anspruch 1.

14. Verwendung der Elektronentransportschicht nach Anspruch 13 in einer organischen elektronischen Vorrichtung.

15. Apparatur, umfassend die organische elektronische Vorrichtung nach einem der Ansprüche 1 bis 12 oder die Elektronentransportschicht nach Anspruch 13.

## Revendications

1. Dispositif électronique organique comportant une première électrode, une deuxième électrode, et une couche organique intercalée entre la première électrode et la deuxième électrode, la couche organique comprenant un complexe organométallique de formule 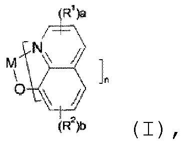 et un composé de formule 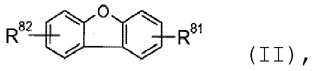 ou 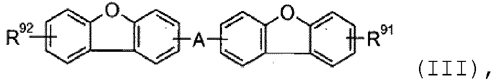 dans lesquels
R¹ et R² représentent indépendamment l'un de l'autre F, un alkyle en C₁-C₈, ou un aryle en C₆-C₁₄, qui peut éventuellement être substitué par un ou plusieurs groupes alkyle en C₁-C₈, ou
deux substituants R¹ et/ou R² se combinent pour former un groupe cyclique benzénique condensé, qui peut éventuellement être substitué par un ou plusieurs groupes alkyle en C₁-C₈,
a et b représentent indépendamment l'un de l'autre 0, ou un entier de 1 à 3,
R⁸¹ représente un groupe phénanthryle, pyrényle, triphénylényle, 1,10-phénanthrolinyle, triazinyle, dibenzothiophényle, ou pyrimidinyle, chacun d'eux pouvant éventuellement être substitué par un ou plusieurs groupes alkyle en C₁-C₁₈, aryle en C₆-C₁₄ ou hétéroaryle en C₂-C₂₀.
R⁸² représente un groupe phénanthryle, pyrényle, triphénylényle, 1,10-phénanthrolinyle, triazinyle, dibenzothiophényle, ou pyrimidinyle, chacun d'eux pouvant éventuellement être substitué par un ou plusieurs groupes alkyle en C₁-C₁₈, aryle en C₆-C₁₄ ou hétéroaryle en C₂-C₂₀,
R⁹¹ représente un groupe phénanthryle, pyrényle, triphénylényle, 1,10-phénanthrolinyle, triazinyle, dibenzothiophényle, pyrimidinyle, ou dibenzofuranyle, chacun d'eux pouvant éventuellement être substitué par un ou plusieurs groupes alkyle en C₁-C₁₈, aryle en C₆-C₁₄ ou hétéroaryle en C₂-C₂₀,
R⁹² représente H ou un groupe phénanthryle, pyrényle, triphénylényle, 1,10-phénanthrolinyle, triazinyle, dibenzothiophényle, ou pyrimidinyle, chacun d'eux pouvant éventuellement être substitué par un ou plusieurs groupes alkyle en C₁-C₁₈, aryle en C₆-C₁₄ ou hétéroaryle en C₂-C₂₀,
A représente une simple liaison ou un groupe arylène ou hétéroarylène, chacun d'eux pouvant éventuellement être substitué par un ou plusieurs groupes alkyle en C₁-C₁₈ ; ou -SiR⁸³R⁸⁴-, dans lequel R⁸³ et R⁸⁴ représentent indépendamment l'un de l'autre un alkyle en C₁-C₁₈, ou un groupe aryle en C₆-C₁₄, qui peut éventuellement être substitué par un ou plusieurs groupes alkyle en C₁-C₁₈,
M représente un atome de métal alcalin, ou un atome de métal alcalino-terreux,
n vaut 1 si M est un atome de métal alcalin, n vaut 2 si M est un atome de métal alcalino-terreux,
la couche organique comprenant le complexe organométallique de formule I et le composé de formule II ou III constituant la couche de transport d'électrons.

2. Dispositif électronique organique selon la revendication 1, dans lequel le composé de formule II est un composé de formule 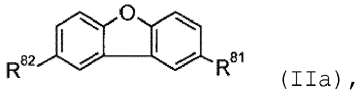 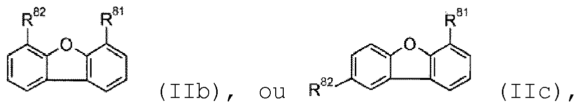 et le composé de formule III est un composé de formule 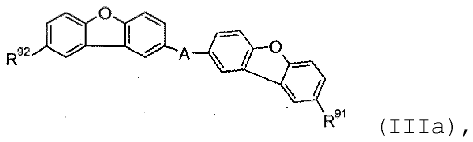 ou 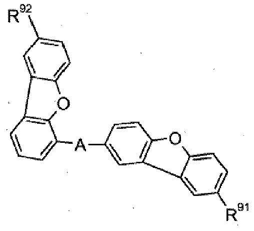 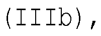 dans lesquels A, R⁸¹, R⁸², R⁹¹ et R⁹² sont tels que définis dans la revendication 1.

3. Dispositif électronique organique selon la revendication 2, dans lequel R⁸² représente 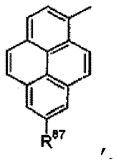 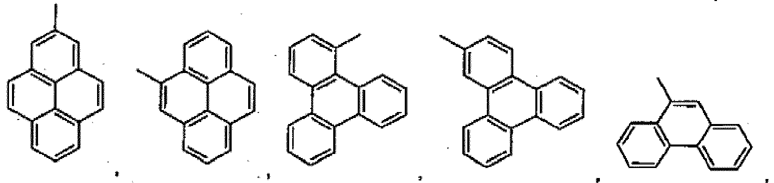 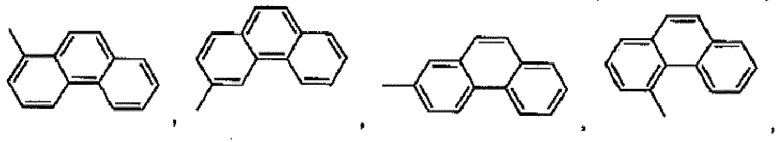 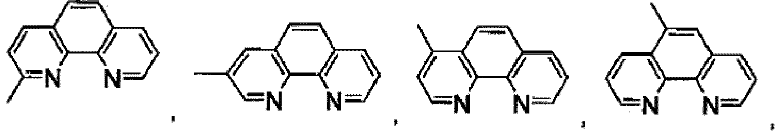 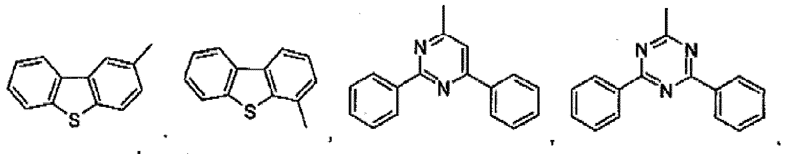 ou 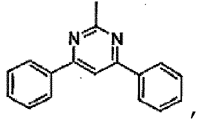 où R⁸⁷ représente H, ou un alkyle en C₁-C₈,
R⁸² a la signification de R⁸¹,
R⁹¹ a la signification de R⁸¹, ou représente 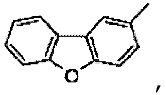 ou 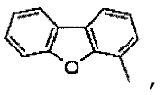 R⁹² a la signification de R⁸¹, ou représente H.

4. Dispositif électronique organique selon l'une quelconque des revendications 1 à 3, dans lequel A représente une simple liaison, un groupe -SiR⁸³R⁸⁴-, un groupe de formule 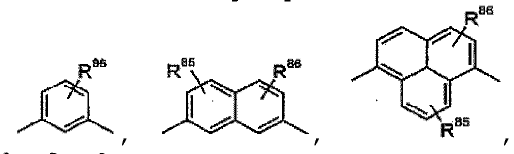 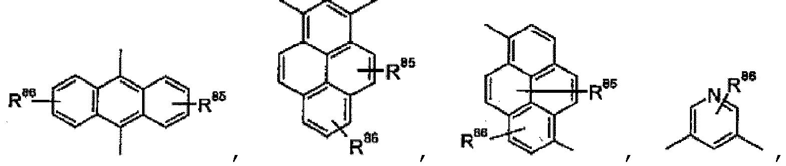 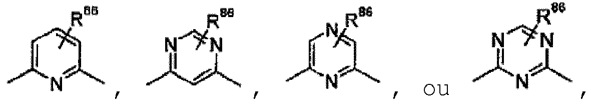 dans lesquels R⁸⁵ et R⁸⁶ représentent indépendamment l'un de l'autre H, ou un groupe alkyle en C₁-C₁₈, et R⁸³ et R⁸⁴ représentent indépendamment l'un de l'autre un groupe alkyle en C₁-C₈.

5. Dispositif électronique organique selon l'une quelconque des revendications 1 à 4, dans lequel le composé de formule II ou III est un composé de formule 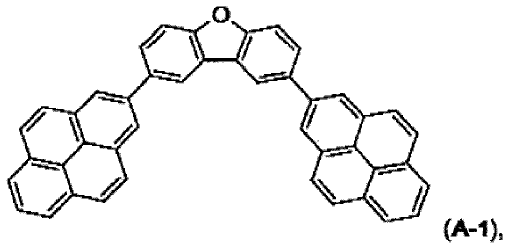 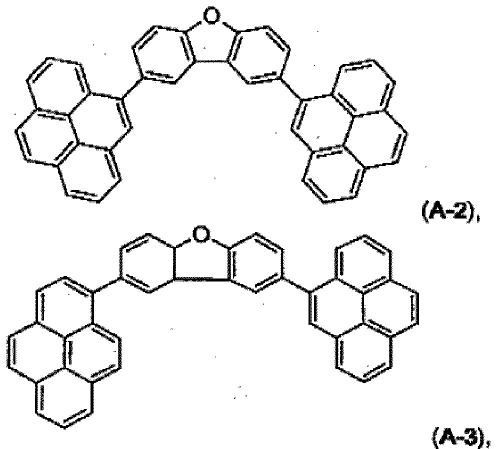 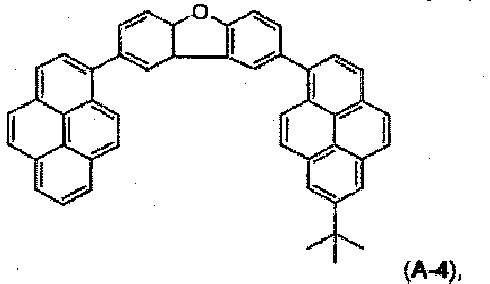 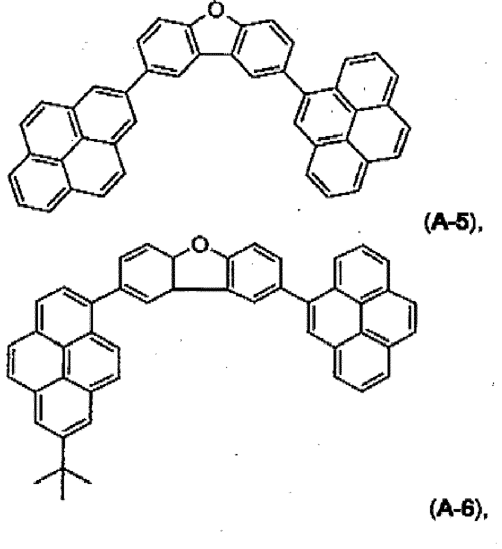 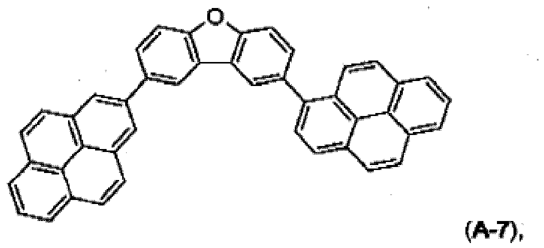 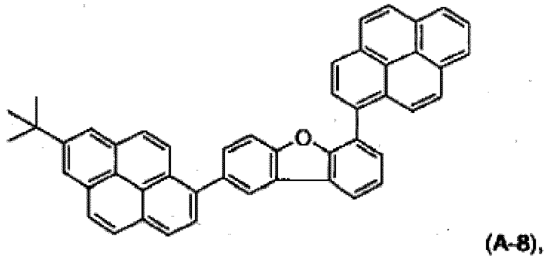 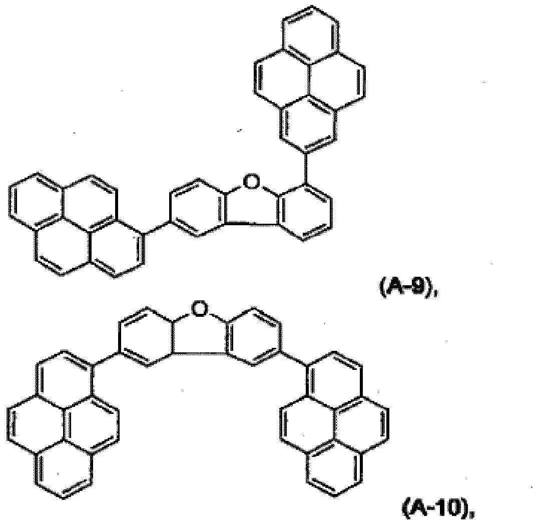 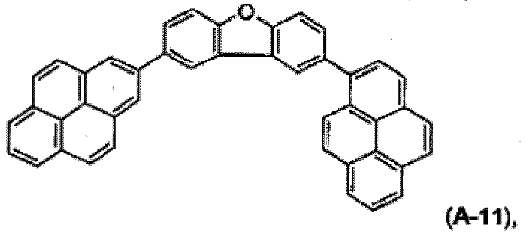 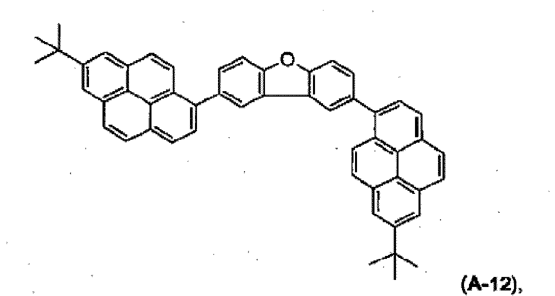 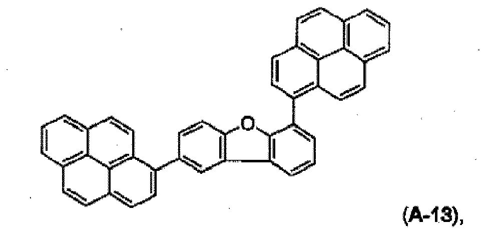 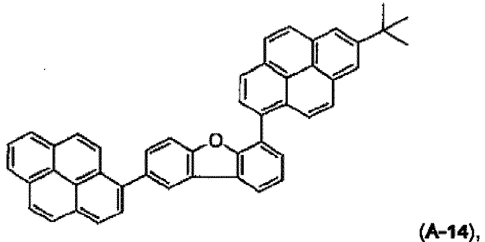 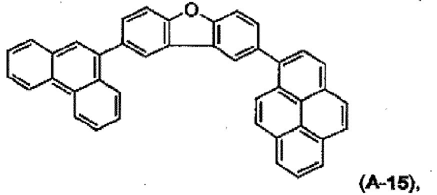 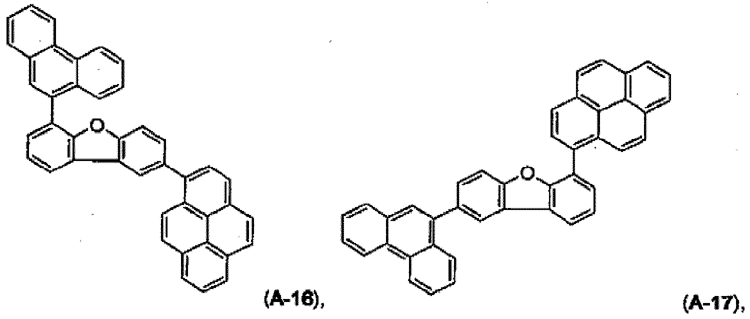 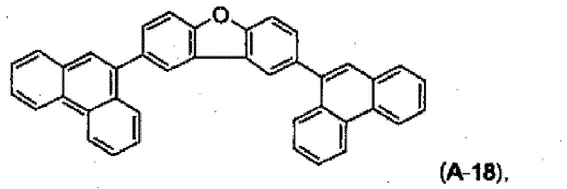 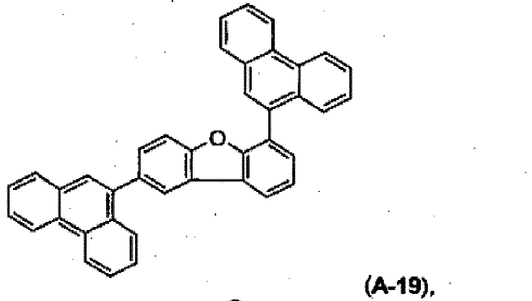 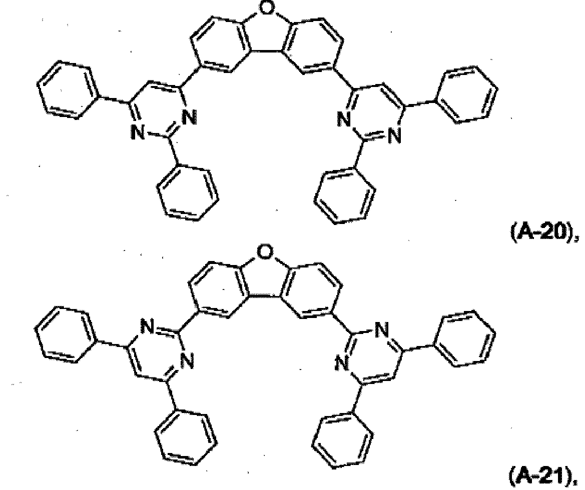 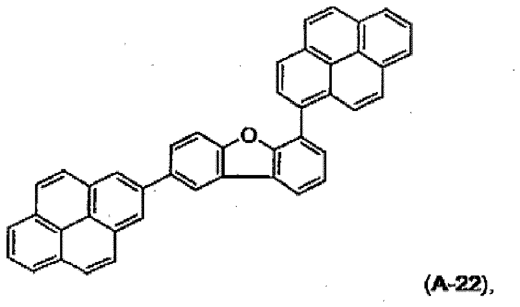 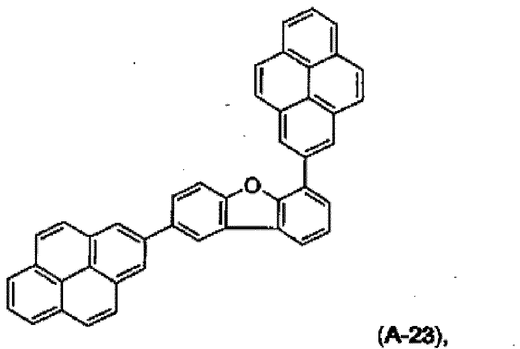 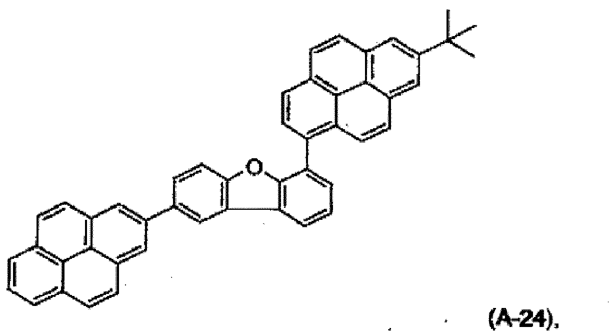 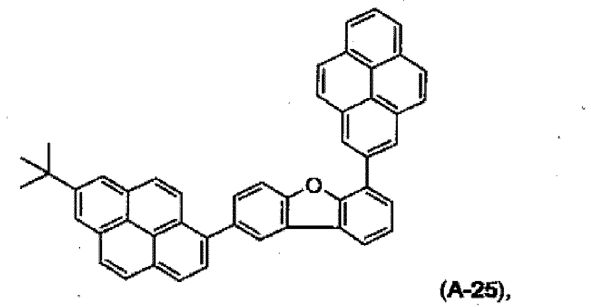 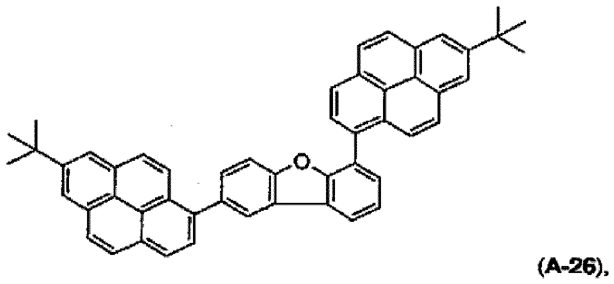 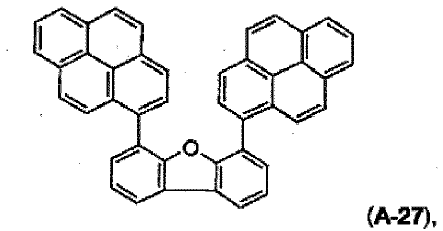 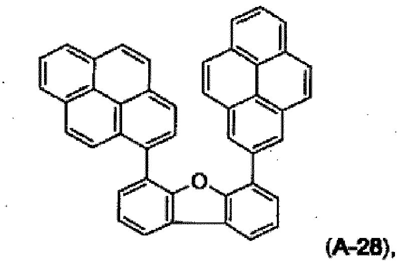 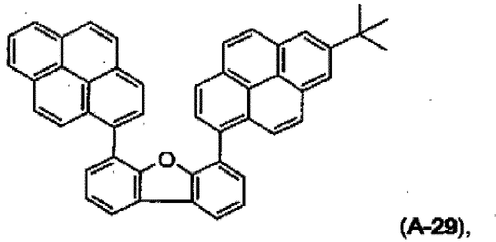 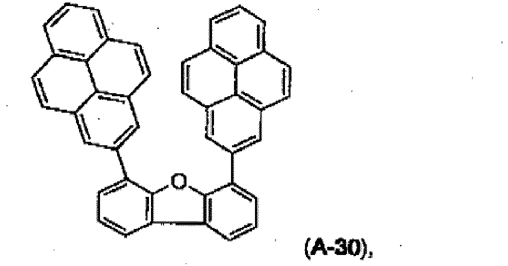 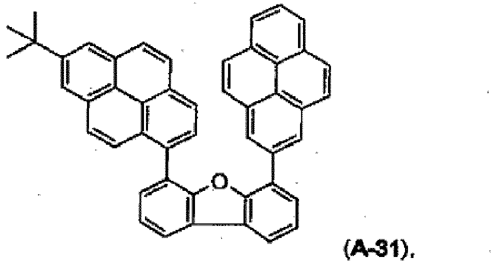 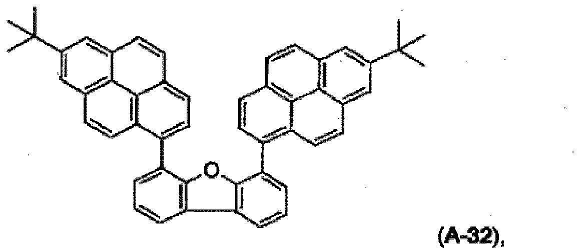 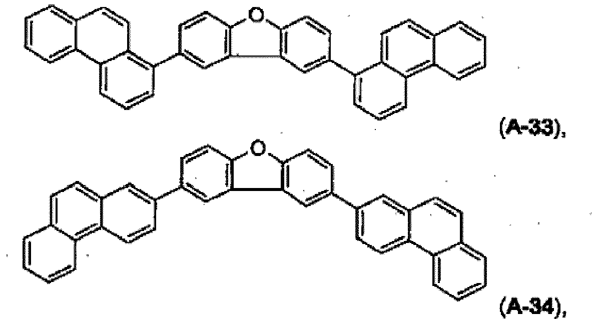 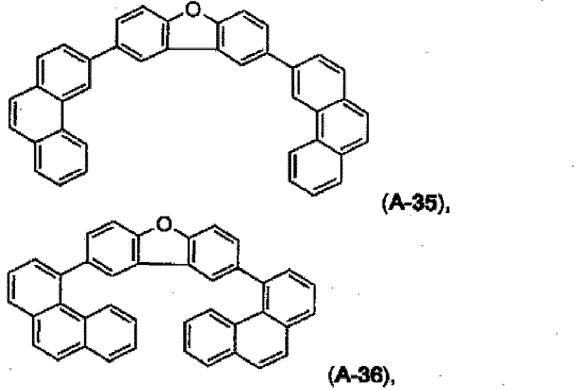 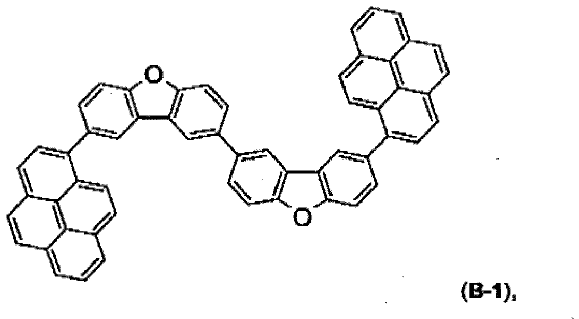 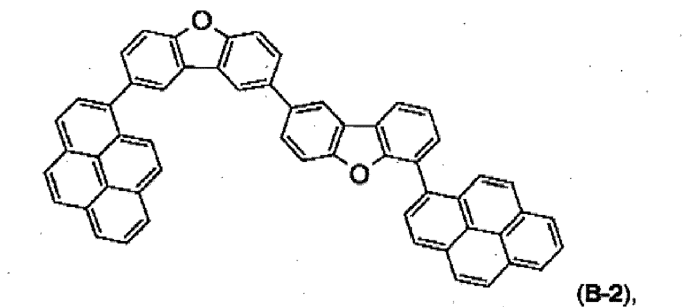 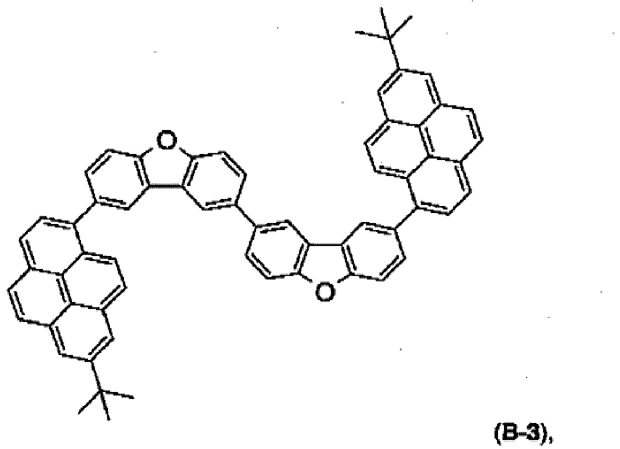 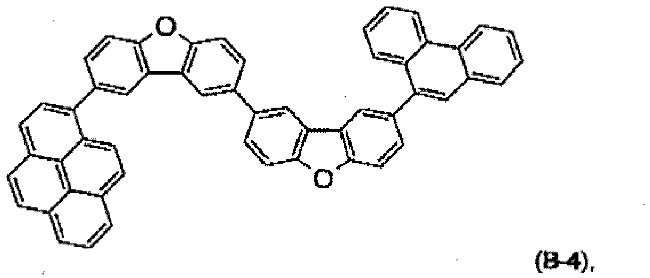 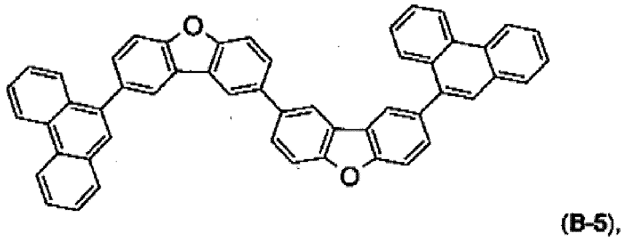 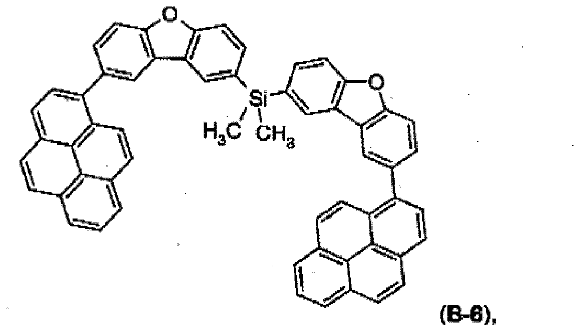 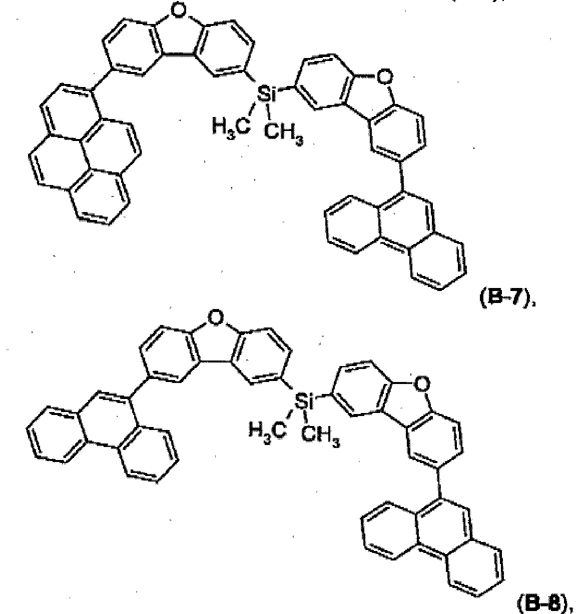 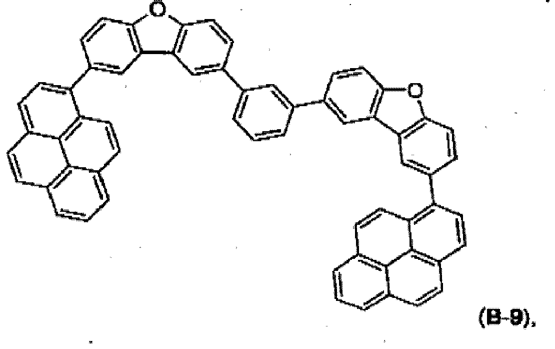 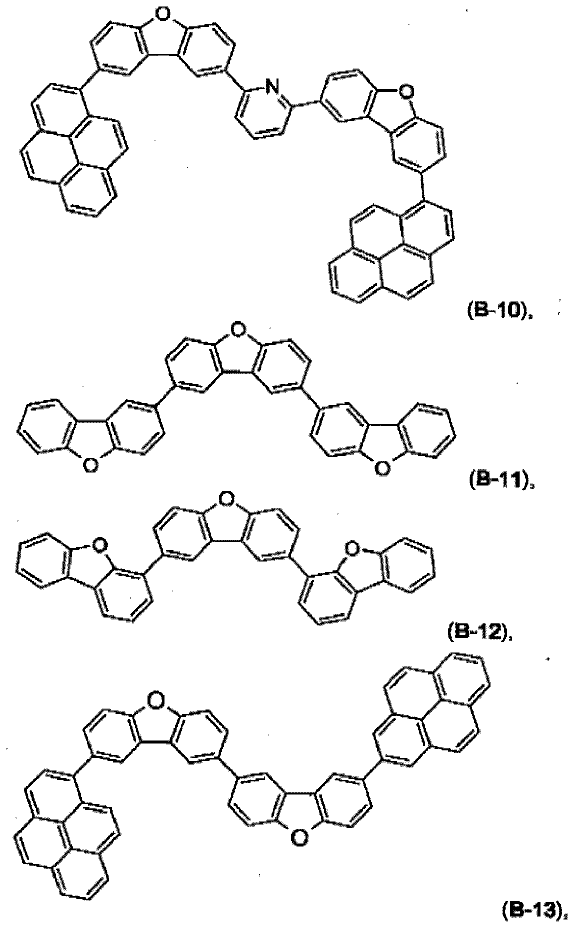 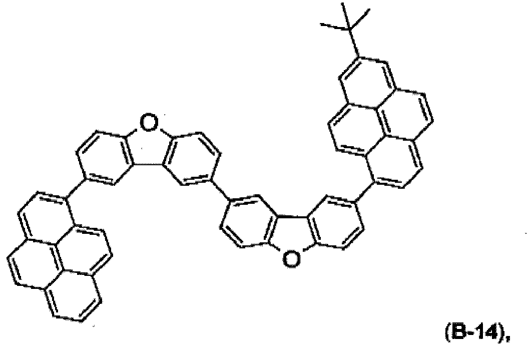 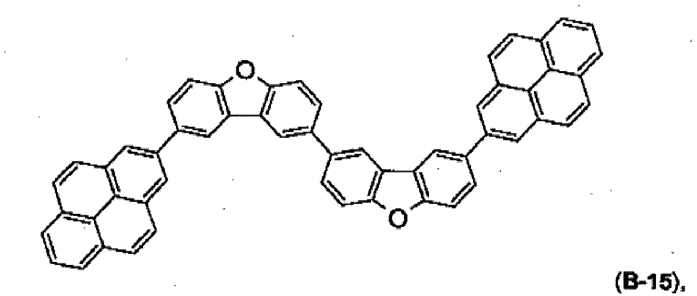 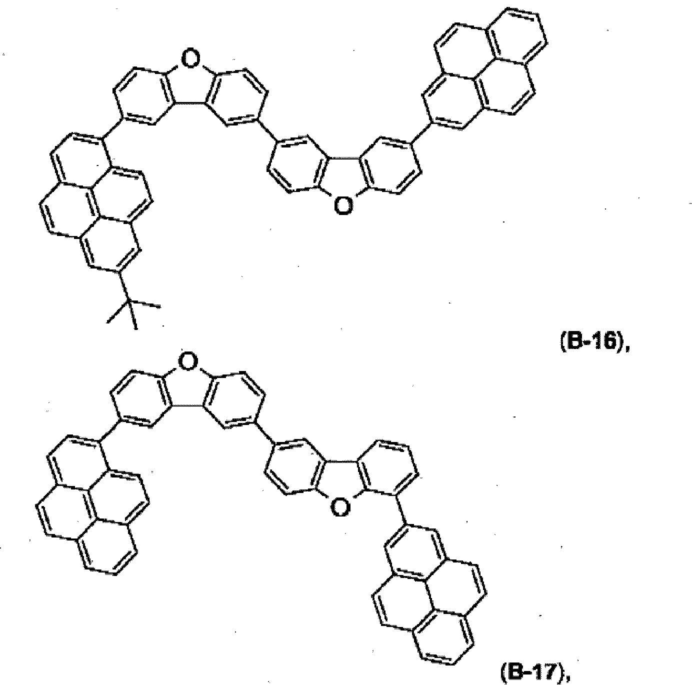 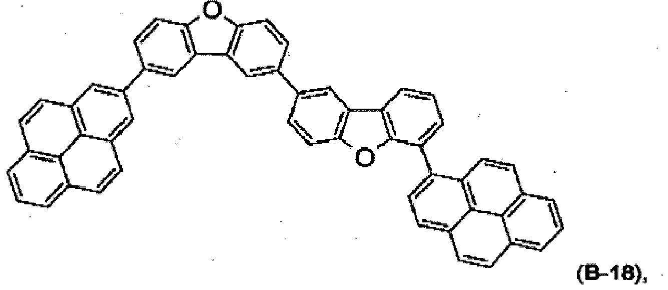 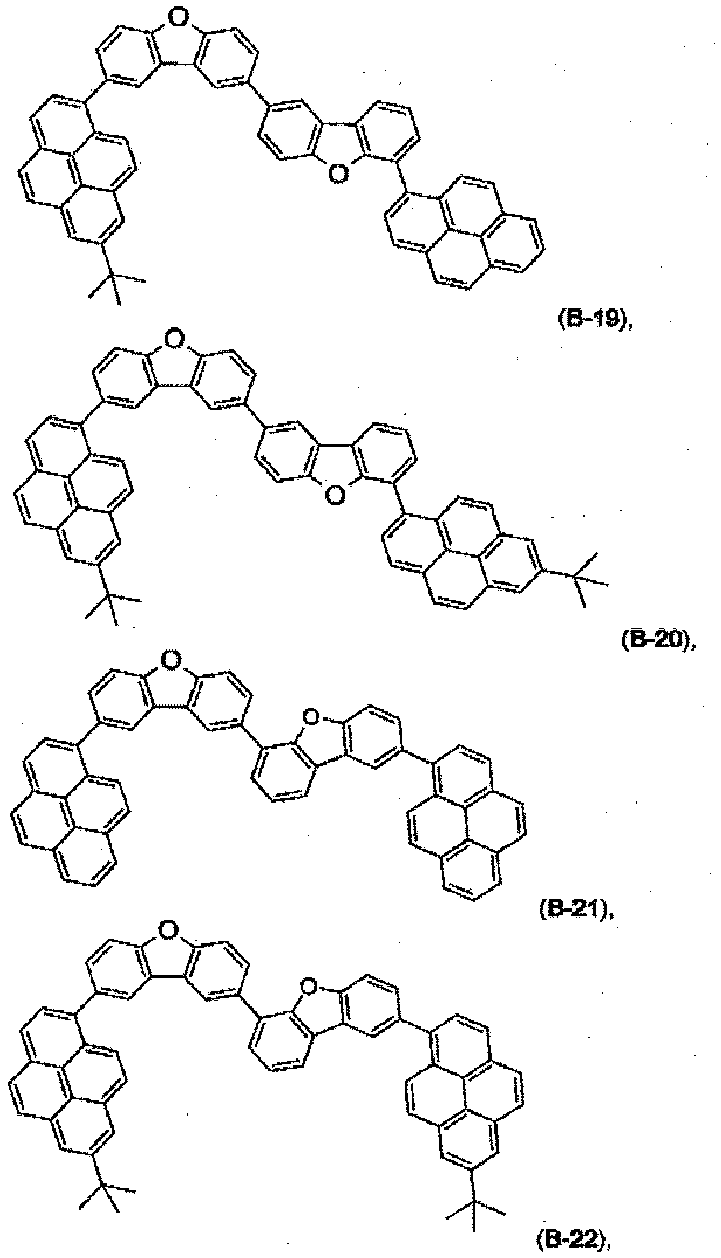 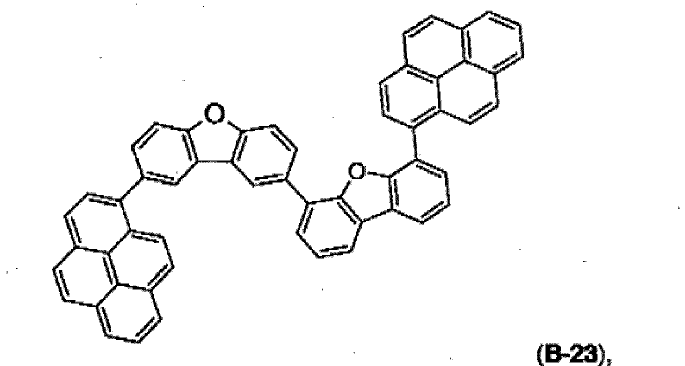 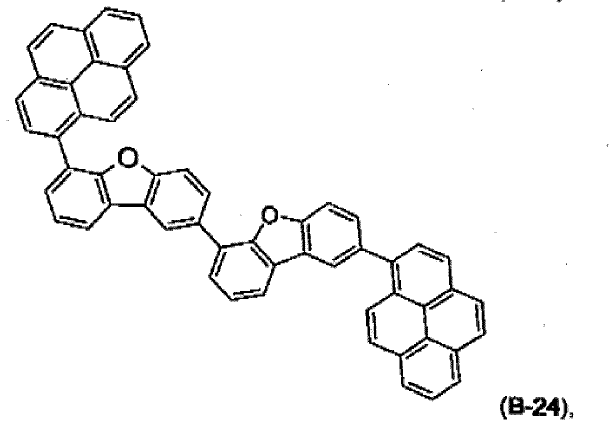 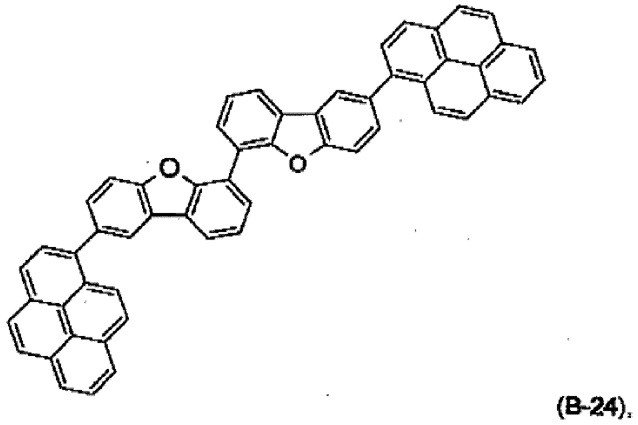 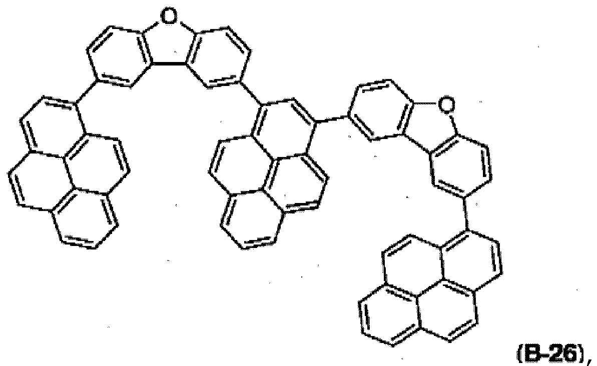 ou

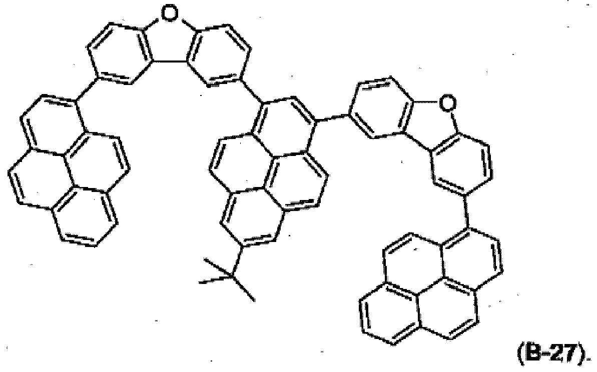

6. Dispositif électronique organique selon l'une quelconque des revendications 1 à 5, dans lequel le composé de formule I est un composé de formule

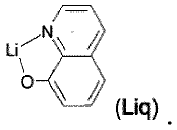

7. Dispositif électronique organique selon l'une quelconque des revendications 1 à 6, qui est un dispositif émetteur de lumière organique, comprenant une anode, une couche d'injection de trous, une couche de transport de trous, une couche émettrice de lumière, une couche de blocage de trous et d'excitons, une couche de transport d'électrons, une couche d'injection d'électrons et une cathode, la couche organique comprenant les composés de formule I et II, ou III constituant la couche de transport d'électrons.

8. Dispositif électronique organique selon la revendication 7, dans lequel la couche de transport d'électrons comprend un mélange d'un composé de formule 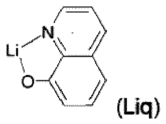 et d'un composé de formule

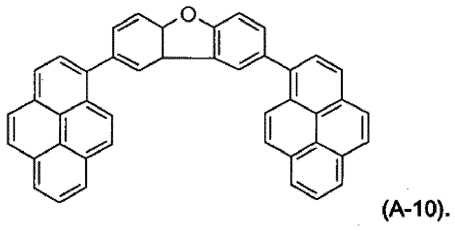

9. Dispositif électronique organique selon la revendication 7 ou 8, dans lequel la couche d'injection d'électrons comprend ou consiste en du fluorure de potassium, ou Liq.

10. Dispositif électronique organique selon l'une quelconque des revendications 7 à 9, dans lequel la couche émettrice de lumière comprend un composé de formule 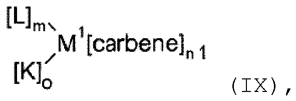 dans laquelle les symboles ont les significations suivantes :
M¹ est un atome métallique choisi dans le groupe constitué par Co, Rh, Ir, Nb, Pd, Pt, Fe, Ru, Os, Cr, Mo, W, Mn, Tc, Re, Cu, Ag et Au dans tout état d'oxydation possible pour l'atome métallique respectif ; carbene est un ligand carbénique qui peut être non chargé ou monoanionique et monodenté, bidenté ou tridenté, le ligand carbénique étant également susceptible d'être un ligand biscarbénique ou triscarbénique ;
L est un ligand monoanionique ou dianionique, qui peut être monodenté ou bidenté ;
K est un ligand monodenté ou bidenté non chargé choisi dans le groupe constitué par les phosphines, les phosphonates et leurs dérivés, les arsénates et leurs dérivés, les phosphites, CO, les pyridines, les nitriles et les diènes conjugués qui forment un complexe π avec M¹;
n1 est le nombre de ligands carbéniques, n1 valant au moins 1, et quand n1 > 1, les ligands carbéniques dans le complexe de formule I peuvent être identiques ou différents ;
m est le nombre de ligands L, m pouvant valoir 0 ou être ≥ 1, et quand m > 1, les ligands L peuvent être identiques ou différents ;
o est le nombre de ligands K, o pouvant valoir 0 ou être ≥ 1, et quand o > 1, les ligands K peuvent être identiques ou différents ;
la somme n1+m+o dépendant de l'état d'oxydation et du nombre de coordination de l'atome métallique et de la denticité des ligands carbéniques, L et K, mais aussi de la charge sur les ligands carbéniques et L, à condition que n1 soit au moins égal à 1.

11. Dispositif électronique organique selon la revendication 10, dans lequel le composé de formule IX est un composé de formule

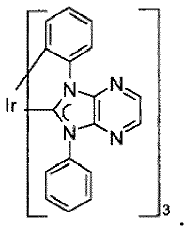

12. Dispositif électronique organique selon l'une quelconque des revendications 7 à 11, dans lequel la couche de transport de trous comprend un composé de formule 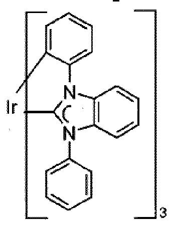 dopé avec un oxyde de molybdène (MoOₓ), en particulier MoO₃, ou un oxyde de rhénium (ReOₓ), en particulier ReO₃.

13. Couche de transport d'électrons, comprenant un complexe organométallique de formule I tel que défini dans la revendication 1 et un composé de formule II, ou III tel que défini dans la revendication 1.

14. Utilisation de la couche de transport d'électrons selon la revendication 13 dans un dispositif électronique organique.

15. Appareil comprenant le dispositif électronique organique selon l'une quelconque des revendications 1 12, ou la couche de transport d'électrons selon la revendication 13.
